# FASCICULE DE BREVET EUROPEEN

(11) **EP 0 648 763 B1**
(45) Date de publication et mention de la délivrance du brevet: **09.05.2001**
(21) Numéro de dépôt: 94400008.2
(22) Date de dépôt: 04.01.1994
(51) Int. Cl.: C07D 403/10, C07C 47/56, C07D 233/90, C07C 311/58, C07C 311/18, C07C 311/37, C07C 311/46, A61K 31/415

(54) **Procédé de préparation de dérivés soufrés de l'imidazole et les intermédiaires obtenus**
Verfahren zur Herstellung von schwefelhaltigen Imidazolderivaten und erhaltene Zwischenprodukte
Process for the preparation of sulfur derivatives of imidazole and intermediates obtained

(30) Priorité: 19.10.1993 FR 9312413
(43) Date de publication de la demande: 19.04.1995
(73) Titulaire: Aventis Pharma S.A., 92160 Antony (FR)
(72) Inventeur: Bhatnagar, Neerja, F-91600 Savigny sur Orge (FR); Buendia, Jean, F-94170 Le Perreux sur Marne (FR); Griffoul, Christine, f-93110 Rosny sous Bois (FR); Heitsch, Holger, D-65719 Hofheim (DE); Wagner, Adalbert, D-65795 Hattersheim/Main (DE)

(56) Documents cités:
- EP-A- 0 443 983
- EP-A- 0 499 415
- EP-A- 0 550 313
- WO-A-91/14679
- WO-A-93/04059
- FR-A- 2 254 320
- JOURNAL OF CHEMICAL SOCIETY, PERKIN TRANSACTIONS no. 6 , 1978 , LETCHWORTH GB pages 653 - 657 S. A. GLOVER ET AL 'N-Iodo-amides: Cyclisation of substituted biphenyl-2-carboxamides'

## Description

La présente invention concerne un nouveau procédé de préparation de dérivés soufrés de l'imidazole et les nouveaux intermédiaires obtenus.

La présente invention a pour objet un nouveau procédé de préparation de produits de formule (I) : 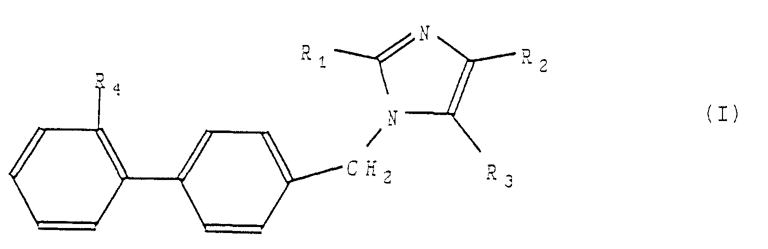 dans laquelle :
R₁ représente un radical alkyle ou alkényle linéaire ou ramifié renfermant au plus 4 atomes de carbone,
R₂ et R₃, identiques ou différents, sont choisis parmi :
   a) l'atome d'hydrogène ; le radical mercapto ; les radicaux formyle ; carboxy libre, salifié ou estérifié ; les atomes d'halogène ; le radical hydroxyle ; cyano ; nitro ; acyle ;
   b) les radicaux alkyle, alkényle, alcoxy, alkylthio dans lequel l'atome de soufre est éventuellement mono ou dioxydé, ces radicaux étant linéaires ou ramifiés et renfermant au plus 6 atomes de carbone, les radicaux phényle, benzoyle, phénylthio dans lequel l'atome de soufre est éventuellement mono ou dioxydé, tous ces radicaux étant éventuellement substitués par un ou plusieurs radicaux identiques ou différents choisis parmi :
      les atomes d'halogène,
      les radicaux hydroxyle, trifluorométhyle, cyano, nitro, formyle, alkyle et alcoxy renfermant au plus 4 atomes de carbone, phényle et carboxy libre, salifié ou estérifié,
   c) les radicaux 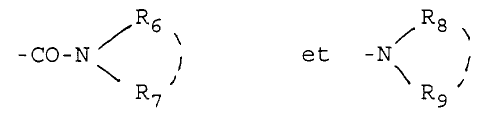
   dans lesquels :
   **ou bien** R₆, R₇, R₈ et R₉, identiques ou différents, sont choisis parmi l'atome d'hydrogène, les acides aminés, les radicaux alkyle, ces radicaux renfermant au plus 6 atomes de carbone, phényle, benzyle, phénéthyle,
   **ou bien** R₆ et R₇ et R₈ et R₉ forment respectivement avec l'atome d'azote auxquels ils sont liés un radical hétérocyclique, ces radicaux identiques ou différents étant choisis parmi les radicaux imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, nitro, alkyle et alcoxy, ces radicaux renfermant au plus 6 atomes de carbone et phényle,
R₄ représente le radical cyano, carboxy libre, salifié ou estérifié, le radical -(CH₂)ₚ-SO₂-X-R₁₀ dans lequel p représente les valeurs 0 et 1, X représente les radicaux -NH-, -NH-CO-, -NH-CO-O-, 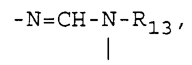 -NH-CO-NH- ou une simple liaison et R₁₀ et R₁₃ identiques ou différents, représentent un atome d'hydrogène, un radical alkyle ou alkényle linéaire ou ramifié, renfermant au plus 6 atomes de carbone et éventuellement substitués, pyridyle, phényle, benzyle, nitropyridyle, pyrimidyle, tétrazolyle, diazolyle, pipéridinyle, alkylpipéridinyle, thiazolyle, alkylthiazolyle, tétrahydrofuranyle, méthyltétrahydrofuranyle ;
les radicaux alkyl et alkényl étant éventuellement substitués par un ou plusieurs radicaux choisis parmi les atomes d'halogène, le radical hydroxyle, nitro, les radicaux alkyle, alkényle et alcoxy renfermant au plus 4 atomes de carbone, le radical trifluorométhyle, cyano, amino, mono et dialkylamino, carboxy libre, salifié ou estérifié, phényl, tétrazolyle ; lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I), caractérisé en ce que l'on fait réagir un composé de formule (A) : 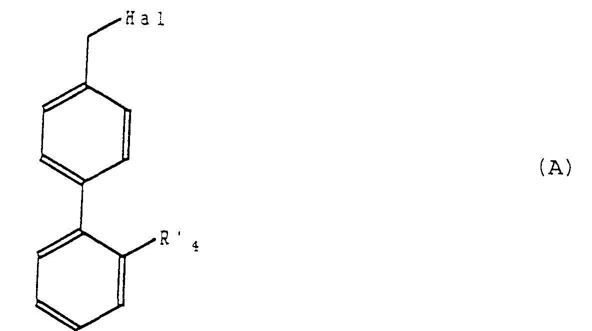 dans laquelle Hal représente un atome d'halogène et R'₄ a la signification indiquée ci-dessus pour R₄ dans laquelle les éventuelles fonctions réactives sont éventuellement protégées avec un agent d'oxydation, pour obtenir le composé de formule (B) : 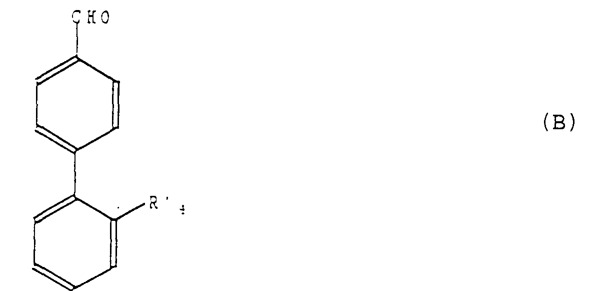 dans laquelle R'₄ a la signification indiquée ci-dessus, que l'on fait réagir avec un composé de formule (II) : 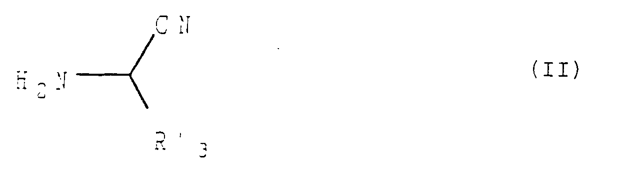 dans laquelle R'₃ a la signification indiquée ci-dessus pour R₃ dans laquelle les éventuelles fonctions réactives sont éventuellement protégées, pour obtenir un produit de formule (C) 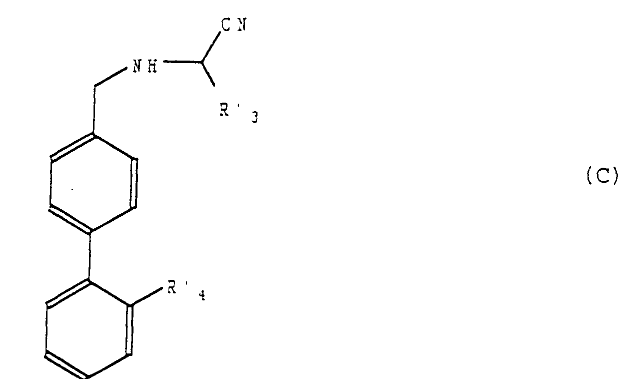 dans laquelle R'₃ et R'₄ ont les significations indiquées ci-dessus, que l'on fait réagir avec un composé de formule (III) :

R'₁-CO-Hal (III)

dans laquelle R'₁ a la signification indiquée ci-dessus pour R₁ dans laquelle les éventuelles fonctions réactives sont éventuellement protégées et Hal représente un atome d'halogène, pour obtenir un produit de formule (D) : 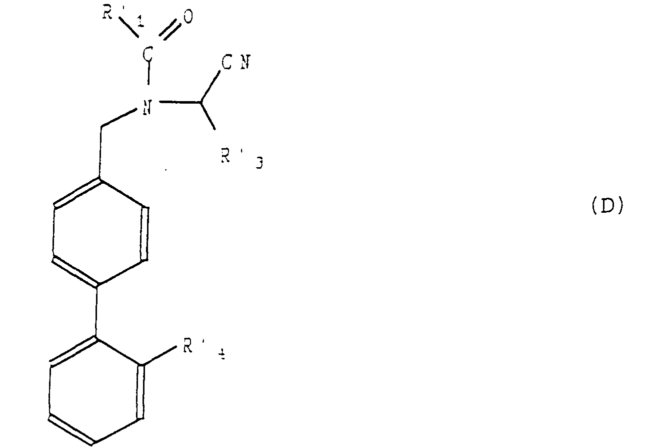 dans laquelle R'₁, R'₃ et R'₄ ont les significations indiquées ci-dessus, que l'on soumet à une réaction d'addition sur le radical CN, à l'aide d'un réactif capable d'introduire le substituant R'₂, R'₂ ayant la signification indiquée ci-dessus pour R₂ dans lequel les éventuelles fonctions réactives sont éventuellement protégées, le produit de formule (D) étant transformé en produit de formule (E) soit par amidification dans un solvant tel que par exemple un alcool tel que le méthanol ou l'éthanol, ou par réaction du composé R'₂-SH sur la fonction cyano dans un solvant tel que par exemple le toluène, le tétrahydrofuranne ou le dichloroéthane,
soit dans le cas où R'₂ représente un radical comprenant un atome de soufre, par thioamidification effectuée par action d'un composé de formule R₁₀-SH, R₁₀Sna ou R₁₀SK dans lequel R₁₀ représente le reste du radical R'₂ tel que défini ci-dessus,
une telle thioamidification pouvant être réalisée par exemple en faisant barboter le composé de formule R₁₀-SH, R₁₀Sna ou R₁₀SK tel que défini ci-dessus dans un solvant tel que par exemple un alcool tel que l'éthanol ou le méthanol ou encore le dichloroéthane, le dichlorométhane, le toluène, le tétrahydrofuranne en présence d'une base, par exemple la triéthylamine, pour obtenir un produit de formule (E) : 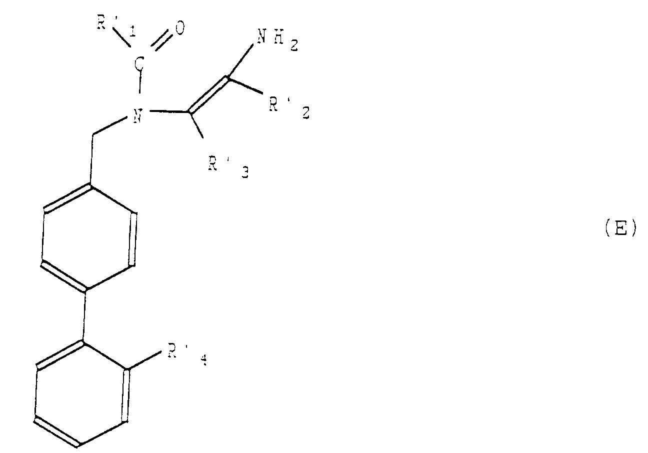
dans laquelle R'₁, R'₂, R'₃ et R'₄ ont les significations indiquées ci-dessus, que l'on soumet le cas échéant à une réaction de substitution de l'atome d'oxygène par un atome de soufre pour obtenir le produit de formule (L) : 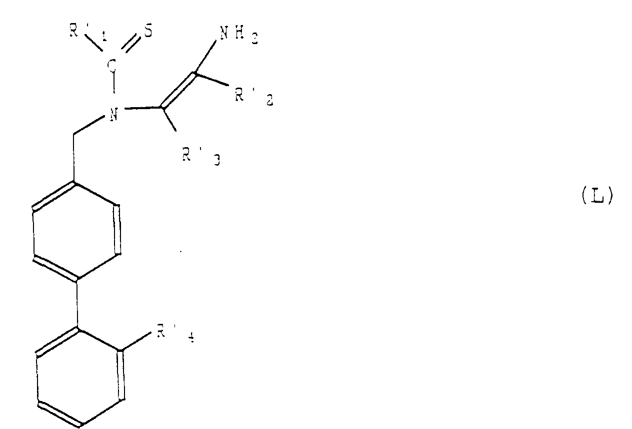 dans laquelle R'₁, R'₂, R'₃ et R'₄ ont les significations indiquées ci-dessus, produit de formule (E) ou produit de formule (L) que l'on soumet à une réaction de cyclisation réalisée soit par une catalyse acide en utilisant par exemple l'amberlyst H⁺, l'acide tosylique ou l'acide sulfurique, dans un solvant tel que par exemple le toluène, l'acétate d'éthyle, le dichlorométhane, le dichloroéthane, soit avec le pentachlorure dans la pyridine ou la diméthylaminopyridine, soit encore dans la diméthylsulfone, pour obtenir un produit de formule (I') : 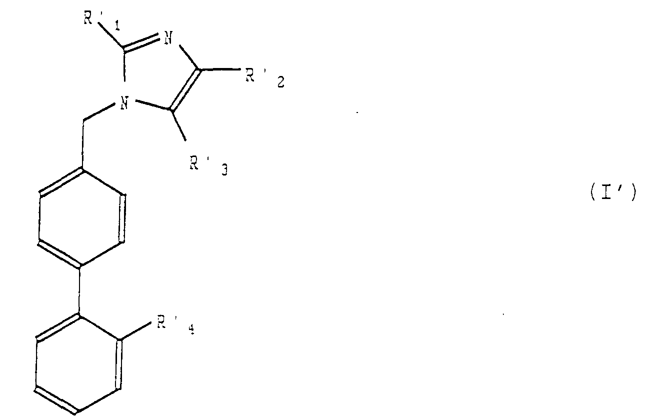 dans laquelle R'₁, R'₂, R'₃ et R'₄ ont les significations indiquées ci-dessus, étant entendu que les produits de formules (D), (E) et (L) au cours du procédé ci-dessus, et les produits de formule (I') peuvent être soumis, si désiré et si nécessaire, à l'une ou plusieurs des réactions suivantes, dans un ordre quelconque :
a) une réaction d'estérification de la fonction acide,
b) une réaction de saponification de la fonction ester,
c) une réaction de transformation de la fonction cyano en fonction acide,
d) une réaction de réduction de la fonction carboxy en fonction alcool,
e) une réaction de transformation de fonction alcoxy en fonction hydroxyle,
f) une réaction d'oxydation de groupement comprenant un atome de soufre en sulfoxyde ou sulfone correspondant,
g) une réaction de transformation de fonction alcool ou sulfone en fonction aldéhyde ou acide correspondante,
h) une réaction de transformation de radical nitrile en tétrazole,
i) une réaction de transformation du radical formyle en radical carbamoyle,
j) une réaction de transformation du radical carbamoyle en radical nitrile,
k) une réaction de transformation du radical 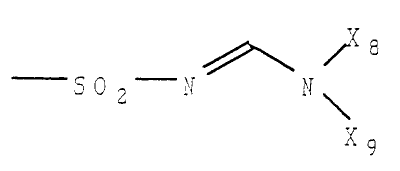 en radical 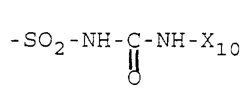 dans lesquels X₈, X₉ et X₁₀, identiques ou différents représentent un atome d'hydrogène ou un radical alkyle ou alkényle renfermant au plus 4 atomes de carbone éventuellement substitué,
produits de formule (I') qui peuvent représenter des produits de formule (I) ou que l'on soumet, si désiré et si nécessaire, pour obtenir les produits de formule (I) à l'une ou plusieurs des réactions suivantes, dans un ordre quelconque :
a) une réaction d'élimination des groupements protecteurs que portent les fonctions réactives protégées,
b) une réaction de salification par un acide minéral ou organique ou par une base pour obtenir le sel correspondant,
c) une réaction de dédoublement des formes racémiques en produits dédoublés
lesdits produits de formule (I) ainsi obtenus étant sous toutes les formes isomères possibles racémiques, énantiomères et diastéréoisomères.

Dans le radical -(CH₂)ₚ-SO₂-X-R₁₀ tel que défini ci-dessus, lorsque p est différent de 0, (CH₂)ₚ représente les valeurs des radicaux alkylène telles que, par exemple, méthylène, éthylène, n-propylène ou n-butylène et notamment lorsque p représente la valeur 0 ou 1, le radical -(CH₂)ₚ- représente respectivement une simple liaison, le radical méthylène ou le radical éthylène.

Parmi les valeurs de (CH₂)ₚ-SO₂-X-R₁₀, on peut citer par exemple et de façon non exhaustive les radicaux : -SO₂-NH₂, -SO₂-NH-CH₃, -SO₂-NH-CF₃, -SO₂-NH-C₆H₅, -SO₂-NH-CH₂-C₆H₅, CH₂-SO₂-NH₂, -CH₂-SO₂-NH-C₆H₅, -SO₂-NH-CO-NH-CH₃, -SO₂-NH-CO-NH-C₆H₅, -SO₂-NH-CO-NH-CF₃, -SO₂-NH-CO-NH-CH2-C₆H₅, -SO₂-NH-CO-NH-D dans lequel D représente un radical phénvle, pyridine ou pyrimidine, 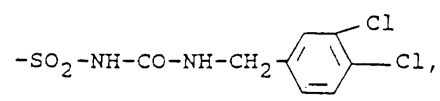 -SO₂-NH-CO-NH-CH₂-CH₂-CH₃, -SO₂-NH-CO-NH-CH=CH-CH₃, 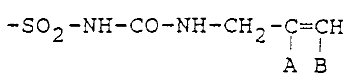 dans lequel A et B, identiques ou différents, sont choisis parmi l'atome d'hydrogène, les radicaux phényle, pyridyle et pyrimidyle, 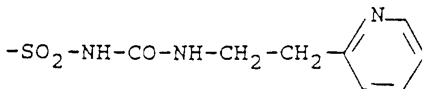 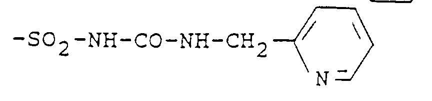 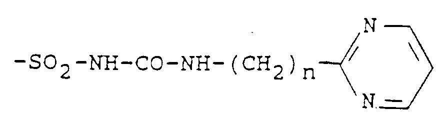 avec n = 1 ou 2.

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle.
- le terme atome d'halogène désigne de préférence l'atome de chlore ou de brome, mais peut aussi représenter un atome de fluor ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle,
- le terme amino substitué par un ou deux radicaux alkyle désigne de préférence des radicaux dans lesquels le ou les radicaux alkyle sont choisis parmi les radicaux alkyle tels que définis ci-dessus comme par exemple pour monoalkylamino, le radical méthylamino ou éthylamino, ou par exemple pour dialkylamino le radical diméthylamino ou encore méthyléthylamino,
- le terme radical alkylthio désigne de préférence les radicaux dans lesquels le radical alkyle est tel que défini ci-dessus, par exemple méthylthio ou éthylthio,
- le radical carbamoyle désigne également les radicaux carbamoyle substitué par un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle; un groupe N-(hydroxyalkyl inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle,
- le terme radical phényle substitué par un radical alkylthio représente par exemple le radical benzylthio.
   Dans les produits de formule (I) et dans ce qui suit, les radicaux alkyle, alkényle et phényle que peuvent représenter ou porter R₁, R₂, R₃ et R₄ peuvent prendre les valeurs définies ci-dessus pour ces radicaux et peuvent ou non être substitués par un ou plusieurs substituants identiques ou différents tels que définis ci-dessus pour ces radicaux.
   R₂ et R₃ peuvent ainsi, par exemple, représenter un radical alkylthio, phénylthio, alkylsulfinyle, phénylsulfinyle, alkylsulfonyle ou phenysulfonyle.
- les termes radical alkylthio, alkylsulfinyle et alkylsulfonyle désignent les radicaux dans lesquels le radical alkyle linéaire ou ramifié peut représenter, par exemple, les valeurs indiquées ci-dessus pour le radical alkyle ; ces radicaux représentent ainsi de préférence les radicaux méthylthio, hydroxyméthylthio, éthylthio, aminoéthylthio, méthylsulfinyle, éthylsulfinyle, méthylsulfonyle, éthylsulfonyle mais peut aussi représenter un radical propylthio, isopropylthio, butylthio, sec-butylthio, tert-butylthio, isopentylthio ou isohexylthio ou ces radicaux dans lesquels le radical thio est oxydé en radical sulfinyle ou sulfonyle.

Selon les valeurs de p et R₁₀ dans le radical -(CH₂)_{P}-SO2-X-R₁₀, R₂ et R₃ peuvent également représenter les radicaux suivants : phénylthio, pyridylthio ou pyrimidylthio, imidazolylthio, N-méthylimidazolylthio ainsi que ceux dans lesquels le radical thio est oxydé en radical sulfinyle ou sulfonyle tel que par exemple dans phénylsulfinyle ou phénylsulfonyle.

Comme exemples de radicaux alkyle substitués, on peut citer ceux substitués par un ou plusieurs radicaux phényle, par exemple, les radicaux benzyle, diphénylméthyle, triphénylméthyle, et ceux substitués par un radical pyridyle, par exemple le radical pyridylméthyle, étant entendu que dans la liste non exhaustive d'exemples de radicaux telle que citée ci-dessus, le radical alkyle peut être représenté tout aussi également par les radicaux éthyle, propyle ou butyle tel que, par exemple, dans le radical phénéthyle.

Comme exemples de radicaux alkényle substitués, on peut citer ceux substitués par un ou plusieurs radicaux phényle ou pyridyle, comme indiqué dans les exemples donnés ci-dessus dans lesquels le radical alkyle est remplacé par un radical alkényle, par exemple les radicaux phénylvinyle ou phénylallyle.

Les radicaux carbamoyle et amino mentionnés plus haut et en particulier : les radicaux 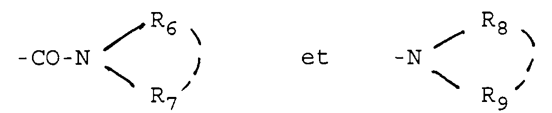 désignent des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, méthoxyméthyle, méthoxyéthyle, éthoxyéthyle ; les radicaux phényle, benzyle, phénéthyle, éventuellement substitués pour donner par exemple le radical phénylamino ou benzylamino.

Parmi les radicaux carbamoyle substitués on peut citer comme radical carbamoyle substitué le groupe N-monoalkyl inférieur carbamoyle, par exemple, N-méthylcarbamoyle, N-éthylcarbamoyle ; le groupe N,N-dialkyl inférieur carbamoyle, par exemple, N,N-diméthylcarbamoyle, N,N-diéthylcarbamoyle ; le groupe N-(hydroxyalkyl inférieur) carbamoyle, par exemple, N-(hydroxyméthyl) carbamoyle, N-(hydroxyéthyl) carbamoyle ; le groupe carbamoylalkyle inférieur, par exemple carbamoylméthyle, carbamoyléthyle ; phénylcarbamoyle ; pyridylcarbamoyle ; benzylcarbamoyle ; N-méthyl N-phénylcarbamoyle ; pyridylméthylcarbamoyle.

L'expression acide aminé désigne de préférence un reste dérivé d'un des acides aminés naturels tels que la glycine, l'alanine, la valine la leucine, l'isoleucine, la phénylalanine et particulièrement la proline ou l'un des autres acides aminés naturels connus de l'homme du métier.

L'hétérocycle que peuvent former R₆ et R₇ ou R₈ et R₉ est de préférence saturé.

Il peut être éventuellement substitué par les substituants déjà mentionnés précédemment et en particulier par un ou plusieurs radicaux choisis parmi les atomes de chlore et de fluor,les radicaux méthyle, éthyle, isopropyle, tert-butyle, méthoxy, éthoxy, propoxy, benzoyle, méthoxycarbonyle, éthoxycarbonyle ; on peut citer par exemple les radicaux méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment par exemple chlorophényle ou trifluorophényle.

Lorsque R₈ ou R₉ représente un radical alcoxycarbonyle, ce radical est de préférence le radical tert-butyloxycarbonyle.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un sel de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alkylmonosulfoniques tels que par exemple l'acide méthanesulfonique, l'acide éthanesulfonique, l'acide propanesulfonique, les acides alkyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Lorsque R₂ et R₃ représentent tous deux un groupement soufré alkylthio ou phénylthio éventuellement oxydé, R₂ et R₃ étant identiques ou différents, les produits préférés de l'invention sont en particulier les produits de formule (I) dans lesquels ces groupements soufrés ont le même degré d'oxydation.

Parmi les produits préférés de l'invention, se trouvent en particulier les produits de formule (I) dans lesquels l'un de R₂ et R₃ représente un groupement soufré éventuellement oxydé tel que défini ci-dessus et l'autre de R₂ et R₃ représente de préférence un radical alkyle, alcoxy, carboxy libre salifié ou estérifié ou phényle éventuellement substitué par un ou plusieurs substituants ainsi qu'il est défini ci-dessus.

Parmi les produits préférés de l'invention, se trouvent tout particulièrement les produits de formule (I) dans lesquels R₂ représente un radical soufré.

R₂ et/ou R₃ peuvent notamment représenter les radicaux alkylthio ou alkénylthio éventuellement substitués par un ou plusieurs radicaux choisis parmi les radicaux formyle ; hydroxyle ; alcoxy ; acyloxy ; carboxy libre, salifié ou estérifié ; amino ; amino substitué ; carbamoyle ; carbamoyle substitué ; alkylthio ; phénylthio ; pyridinyle; pyrimidinyle ; phényle.

Parmi les substituants que peuvent porter les radicaux R₂ et R₃, les radicaux amino et carbamoyle peuvent notamment être substitués par un ou deux radicaux alkyle et acides aminés cités plus haut.

Les radicaux amino et carbamoyle substitués que peuvent porter les radicaux R₂ et R₃ peuvent également former un hétérocycle tel que ceux cités plus haut.

R₂ et R₃ peuvent encore notamment représenter les radicaux alkylthio, substitués par un ou plusieurs atomes d'halogène tels que chlore et fluor. On peut citer par exemple les radicaux :
-S-CF₃ ; -S-CHF₂ ; -S-CH₂F ; -S-CF₂-CHF₂ ;-S-CF₂-CHFCl.

Les radicaux R₂ et R₃ peuvent ainsi représenter les radicaux suivants dans lesquels n, nl et n2, identiques ou différents représentent les valeurs 0 à 2, 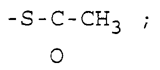 -SO₃H ; -S-CH₃ ;
-S-(CH₂)ₙ₁-S-(CH₂)ₙ₂-X₄ ;
-S-(CH₂)ₙ-X₄ ;
-S-(CH₂)ₙ₁-NH-(CH₂)ₙ₂-X₄ ;
-S-CH=CH-(CH₂)ₙ-X₄ ;
-S-(CH₂)ₙ₁-CH=CH-(CH₂)ₙ₂-X₄ ;
dans lesquels X₄ représente H, OH, cyclohexyle, pyridyle, phényle, CHO, COOH, NH₂ ou

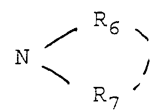

Les radicaux R₂ et R₃ peuvent également représenter, particulièrement, les radicaux suivants :
-COOH ; -CO₂X₅ ; -SX₅ ; -NH₂ ; -C≡N ; -OMe ; -OEt ; -CH=CH-COOH ; tétrazolyle ; 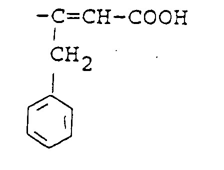 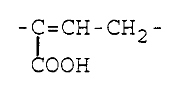 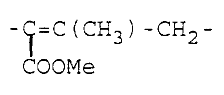 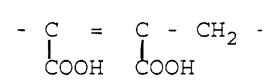 sous toutes leurs formes isomères, isomères cis-trans,

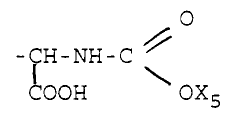

-NH-CH₂-COO-X₅

-NH-COO-X₅

X₅ représentant un radical alkyle ou aryle.

Les radicaux R₂ et R₃ peuvent tout particulièrement représenter le radical :

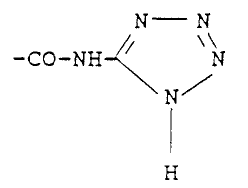

Les produits de formule (I) représentent donc particulièrement les produits dans lesquels R₂ et R₃ ont les significations indiquées ci-dessus et tout particulièrement les produits dans lesquels R₂ représente un radical alkylthio éventuellement substitué tel que défini ci-dessus ou alcoxy tel que par exemple méthoxy et R₃ représente un radical carboxy libre, salifié ou estérifié, ou amidifié tel que notamment -COOH, -COO méthyl, -COO-éthyl, -CONH₂ ou

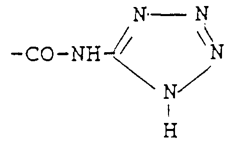

Parmi les valeurs préférées de R₄, on peut citer notamment le radical cyano, le radical -(CH₂)ₘ₁-SO₂-X-R₁₀ tel que défini ci-dessus et plus particulièrement les radicaux indiqués ci-dessous :
-SO₂-NH-CO-NH-CH₂-CH=CH₂, -SO₂-NH-CO-NH-CH₂-CH₂-CH₃,

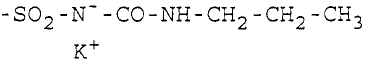

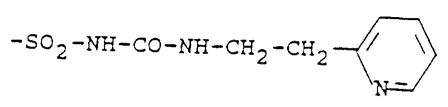

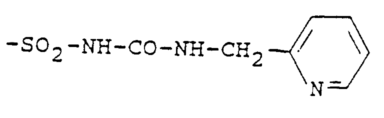

L'invention a particulièrement pour objet le procédé ci-dessus, pour la préparation de produits de formule (I) répondant à la formule (I_{b}) : 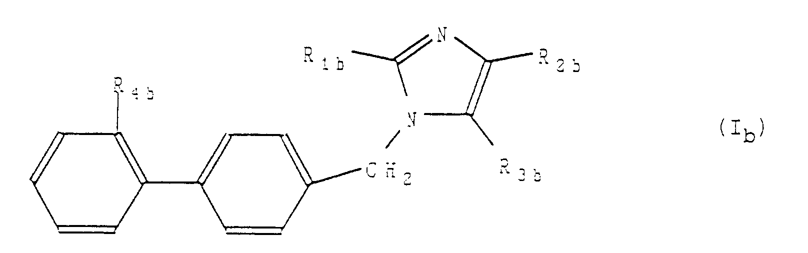 dans laquelle :
R_{1b} représente un radical alkyle renfermant au plus 4 atomes de carbone,
R_{3b} représente un atome d'hydrogène, un radical formyle, acyloxy, alkyle ou alcoxy éventuellement substitué ou un radical carboxy libre, salifié ou estérifié par un radical alkyle,
R_{2b} représente un radical phénylthio, phénylsulfonyle, phénylsulfinyle, alkylthio, alkylsulfonyle ou alkylsulfinyle, éventuellement substitué, tel que dans tous ces radicaux que peuvent représenter R_{2b} et R_{3b}, les radicaux alkyle, alcoxy renfermant au plus 6 atomes de carbone, et les radicaux phényle sont éventuellement substitués par un ou plusieurs radicaux choisis parmi les atomes d'halogène et les radicaux hydroxyle, trifluorométhyle, acyloxy, carboxy libre salifié ou estérifié, phényle, pyridyle, tétrazolyle, alkyle et alcoxy renfermant au plus 4 atomes de carbone et eux-mêmes éventuellement substitués par un radical alcoxy renfermant au plus 4 atomes de carbone,
R_{4b} représente le radical cyano, carboxy libre, salifié ou estérifié, le radical -SO₂-X_{b}-R_{10b} dans lequel X_{b} représente les radicaux -NH-, -NH-CO-, -NH-CO-O-, 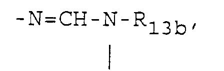 -NH-CO-NH- ou une simple liaison et R_{10b} et R_{13b} identiques ou différents, représentent un atome d'hydrogène, un radical méthyle, éthyle, propyle, vinyle, allyle, pyridyle, phényle, benzyle, nitropyridyle, pyrimidyle, tétrazolyle, diazolyle, pipéridinyle, alkylpipéridinyle, thiazolyle, alkylthiazolyle, tétrahydrofuranyle, méthyltétrahydrofuranyle,
lesdits produits de formule (I_{b}) étant sous toutes les formes isomères possibles racémiques, énantiomères et diastéréoisomères, ainsi que les sels d'addition avec les acides minéraux et organiques ou avec les bases minérales et organiques desdits produits de formule (I_{b}), caractérisé en ce que l'on utilise pour leur préparation telle que définie ci-dessus, des produits de formules (A), (II), (III) et un réactif capable d'introduire le radical R'₂ dans lesquels R'₁, R'₂, R'₃ et R'₄ ont les valeurs indiquées ci-dessus respectivement pour R_{1b}, R_{2b}, R_{3b} et R_{4b} dans lesquelles les fonctions réactives sont éventuellement protégées.

L'invention a plus particulièrement pour objet le procédé ci-dessus pour la préparation de produits de formule (I) répondant à la formule (I_{d}) : 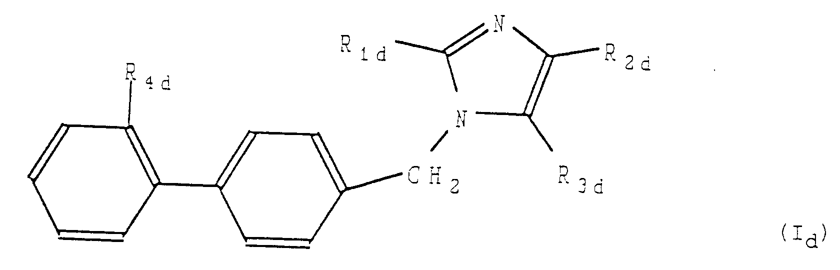 dans laquelle :
R_{1d} représente un radical alkyle renfermant au plus 4 atomes de carbone,
R_{3d} représente un radical :
   carboxy libre, salifié ou estérifié par un radical alkyle linéaire ou ramifié renfermant au plus 4 atomes de carbone, formyle, acyloxy, alkyle renfermant au plus 4 atomes de carbone éventuellement substitués par un radical hydroxyle, R_{2d} représente un radical :
   phénylthio, phénylsulfonyle, phénylsulfinyle,
   alkylthio, alkylsulfonyle ou alkylsulfinyle,
   dans lesquels le radical alkyle renferme au plus 4 atomes de carbone,
et R_{4d} représente le radical -SO₂-NH₂, -SO₂-NH-CO-O-R_{10d}, -SO₂-N=CH-NR_{13d}, ou -SO₂-NH-CO-NH-R_{10d} dans lesquels R_{10d} et R_{13d} identiques ou différents, sont choisis parmi l'atome d'hydrogène, le radical méthyle, éthyle, n-propyle et propényle, lesdits produits de formule (I_{d}) étant sous toutes les formes isomères possibles racémiques, énantiomères et diastéréoisomères, ainsi que les sels d'addition avec les acides minéraux et organiques ou avec les bases minérales et organiques, desdits produits de formule (I_{d}), caractérisé en ce que l'on utilise pour leur préparation telle que définie ci-dessus, des produits de formules (A), (II), (III) et un réactif capable d'introduire le radical R'₂ dans lesquels R'₁, R'₂, R'₃ et R'₄ ont les valeurs indiquées ci-dessus respectivement pour R_{1d}, R_{2d}, R_{3d} et R_{4d} dans lesquelles les fonctions réactives sont éventuellement protégées.

L'invention a particulièrement pour objet le procédé tel que défini ci-dessus, caractérisé en ce que l'on utilise un produit de formule (A) dans laquelle R'₄ représente le radical -SO₂-NH₂, -SO₂-NH-CO-O-R_{10d}, -SO₂-N=CH-NR_{13d}, ou SO₂-NH-CO-NH-R_{10d} dans lesquels R_{10d} et R_{13d} identiques ou différents, sont choisis parmi l'atome d'hydrogène, le radical méthyle, éthyle, n-propyle et propényle, dans lesquels les fonctions réactives sont éventuellement protégées, un produit de formule (II) dans laquelle R'₃ représente le radical : carboxy libre, salifié ou estérifié par un radical alkyle linéaire ou ramifié renfermant au plus 4 atomes de carbone, formyle, acyloxy, ou alkyle renfermant au plus 4 atomes de carbone éventuellement substitués par un radical hydroxyle,
un produit de formule (III) dans laquelle R'₁ représente un radical alkyle renfermant au plus 4 atomes de carbone, et un réactif capable d'introduire le substituant R'₂ dans lequel R'₂ représente le radical : phénylthio, phénylsulfonyle, phénylsulfinyle, alkylthio, alkylsulfonyle ou alkylsulfinyle, dans lesquels le radical alkyle renferme au plus 4 atomes de carbone, et les fonctions réactives sont éventuellement protégées.

L'invention a tout particulièrement pour objet le procédé tel que défini ci-dessus, caractérisé en ce que l'on utilise un produit de formule (A) dans laquelle R'₄ représente le radical 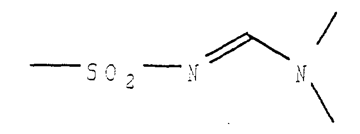 un produit de formule (II) dans laquelle R'₃ représente un radical alcoxy ou carboxy libre, salifié ou estérifié, un produit de formule (III) dans laquelle R'₁ représente un radical alkyle renfermant au plus 4 atomes de carbone et un réactif capable d'introduire le substituant R'₂ dans lequel R'₂ représente un radical alkylthio ou phénylthio éventuellement oxydé sous forme de sulfoxyde ou de sulfone, ces radicaux alcoxy, alkylthio et phénylthio étant éventuellement substitués par un ou plusieurs radicaux choisis parmi les atomes d'halogène, les radicaux alkyl ou alcoxy renfermant au plus 4 atomes de carbone, trifluorométhyle, amino, mono ou dialkylamino, cyano, phényl, hydroxyle, carboxy libre, salifié ou estérifié, acyle et acyloxy.

L'invention a donc aussi pour objet le procédé tel que défini ci-dessus, caractérisé en ce que l'on fait réagir un composé de formule (A₁) : 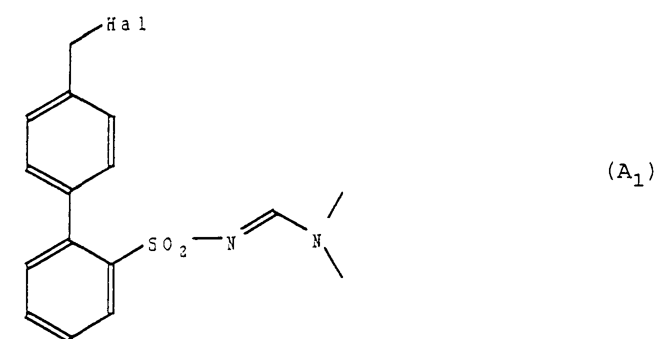 dans laquelle Hal représente un atome d'halogène avec un agent d'oxydation pour obtenir le composé de formule (B₁) : 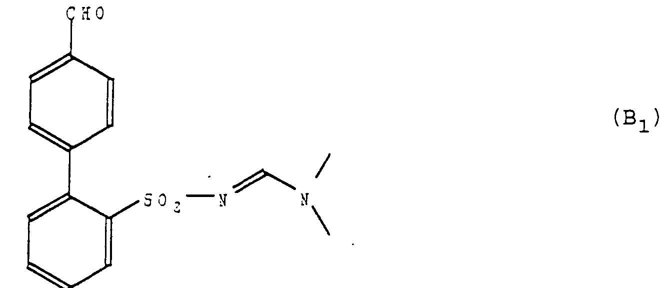 que l'on fait réagir avec un composé de formule (II) : 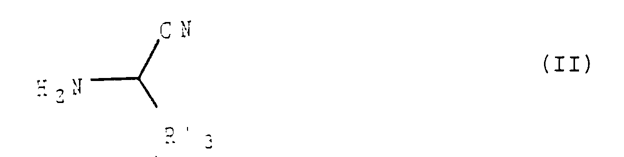 dans laquelle R'₃ a la signification indiquée ci-dessus, pour obtenir un produit de formule (C₁) : 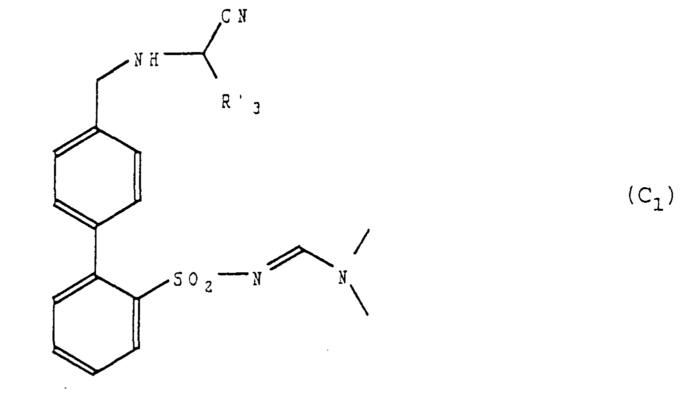 dans laquelle R'₃ a la signification indiquée ci-dessus, que l'on fait réagir avec un composé de formule (III) :

R'₁-CO-Hal (III)

dans laquelle R'₁ et Hal ont la signification indiquée ci-dessus, pour obtenir un produit de formule (D₁) : 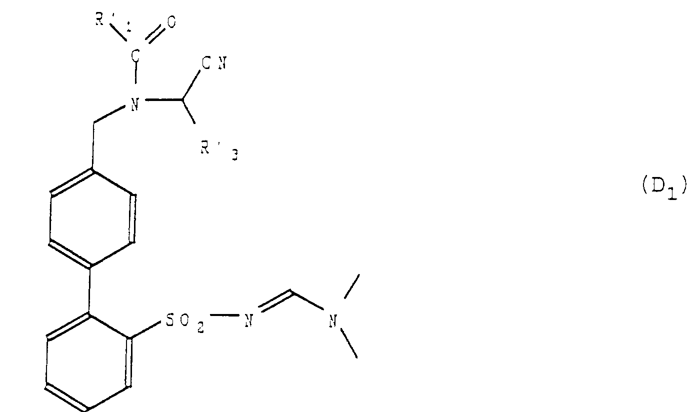 dans laquelle R'₁ et R'₃ ont les significations indiquées ci-dessus, que soit l'on soumet à une réaction d'addition sur le radical CN, à l'aide d'un réactif capable d'introduire le substituant R'₂ tel que défini ci-dessus, pour obtenir le produit de formule (E₁) : 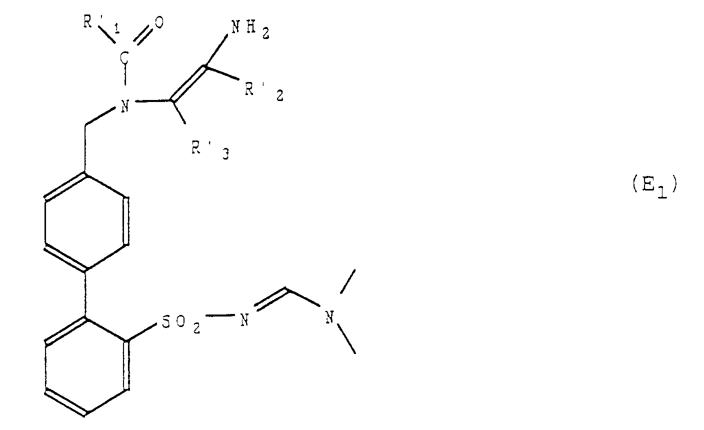 dans laquelle R'₁, R'₂ et R'₃ ont les significations indiquées ci-dessus, que l'on soumet le cas échéant à une réaction de substitution de l'atome d'oxygène par un atome de soufre pour obtenir le produit de formule (L₁) : 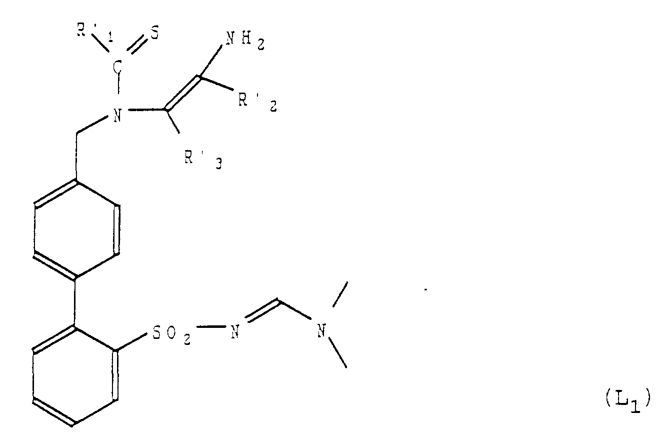 dans laquelle R'₁, R'₂ et R'₃ ont les significations indiquées ci-dessus, que, si nécessaire ou si désiré, l'on transforme en produit de formule (L₂) : 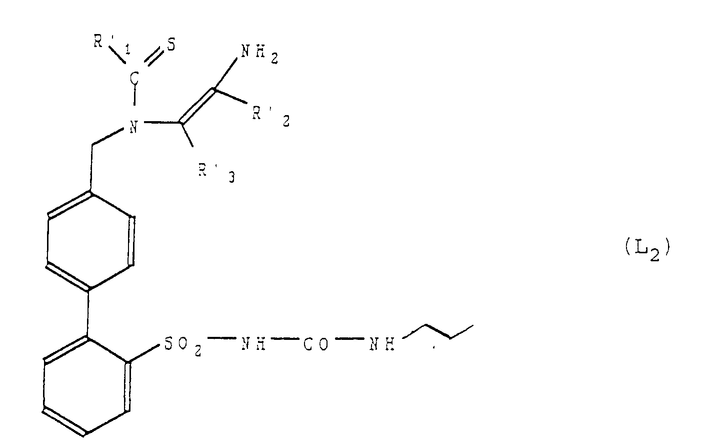 dans laquelle R'₁, R'₂ et R'₃ ont les significations indiquées ci-dessus, produit de formule (E₁), (L₁) ou (L₂) que l'on soumet à une réaction de cyclisation pour obtenir un produit de formule (I'₁) ou (I'₂) : 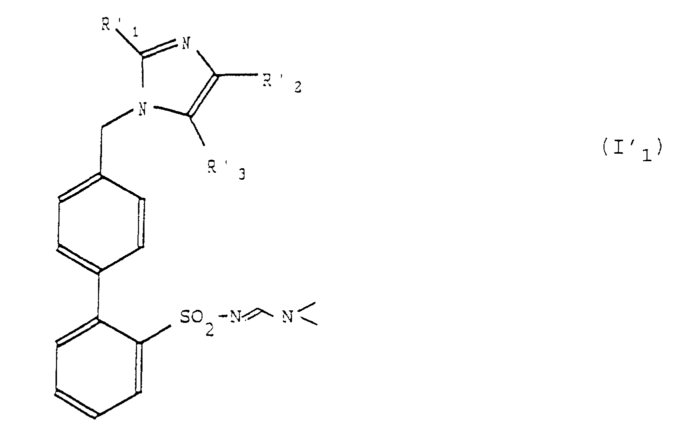 ou 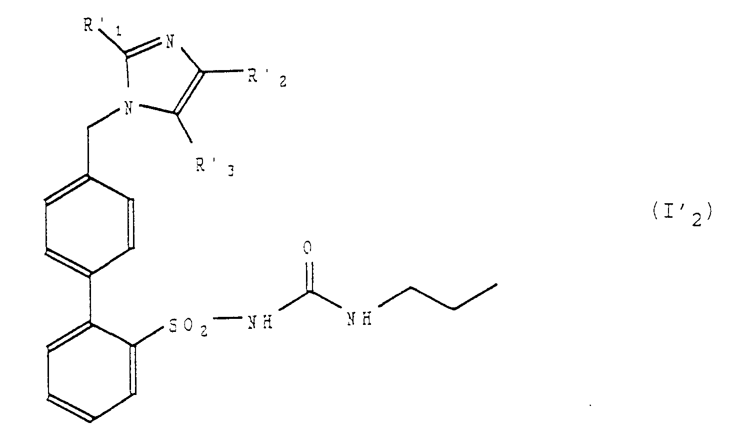 dans laquelle R'₁, R'₂ et R'₃ ont les significations indiquées ci-dessus, et le cas échéant transforme le produit de formule (I'₁) en produit de formule (I'₂) telle que définie ci-dessus,
soit l'on soumet le produit de formule (D₁) à une réaction d'addition sur le radical 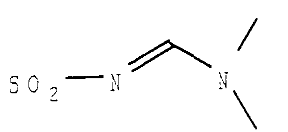 pour obtenir le produit de formule (D₂) : 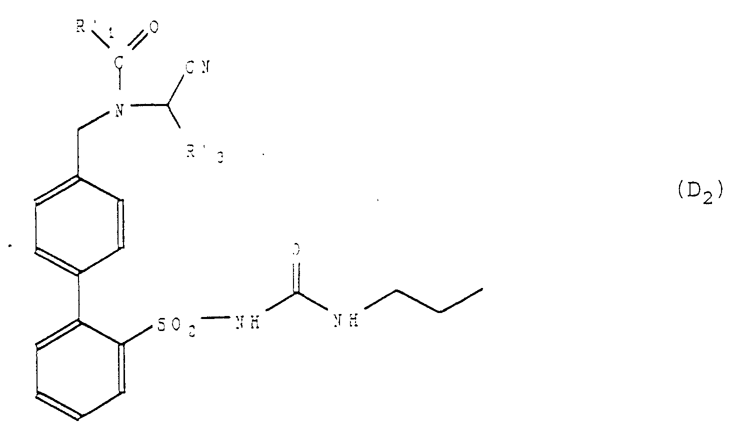 dans laquelle R'₁ et R'₃ ont les significations indiquées ci-dessus,
que l'on soumet à une réaction d'addition sur le radical CN comme indiqué ci-dessus, pour obtenir un produit de formule (E₂) : 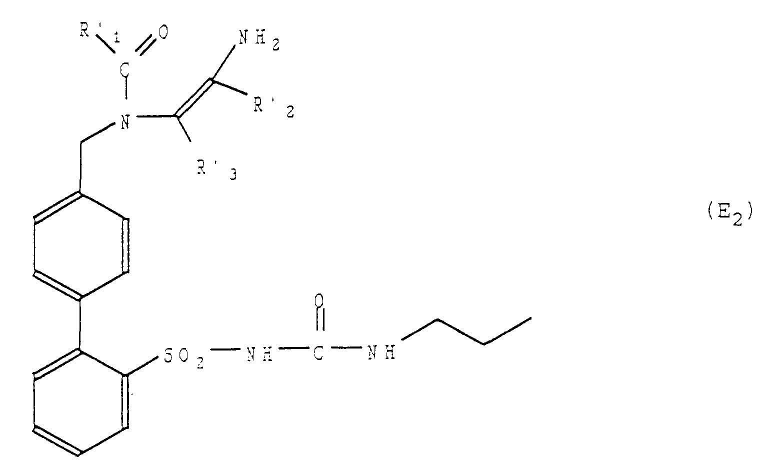 dans laquelle R'₁, R'₂ et R'₃ ont les significations indiquées ci-dessus, que l'on soumet à une réaction de cyclisation pour obtenir un produit de formule (I'₂) telle que définie ci-dessus.

L'invention a plus précisément pour objet le procédé tel que défini ci-dessus, de préparation des produits de formule (I) répondant aux formules suivantes :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl) -2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-butyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) 1H-imidazole-5-carboxylique.

L'invention a plus particulièrement pour objet le procédé tel que défini ci-dessus, de préparation des produits de formule (I) répondant aux formules suivantes :
- le 2-butyl 4-(méthylthio) 1-[[2'-((((propylamino) carbonyl) amino) sulfonyl) (1,1'-biphényl) -4-yl] méthyl]-1H-imidazole 5-carboxylate d'éthyle,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique, di sel de potassium.

Dans des conditions préférentielles de mise en oeuvre de l'invention, le produit de formule (B) peut être obtenu par oxydation de l'halogénure de formule (A), au moyen par exemple d'hexaméthylènetétramine dans de l'acide acétique à 50 % ou de N-oxyde N-méthyl morpholine dans un solvant tel que par exemple le dichlorométhane, le dichloroéthane ou encore le toluène.

Le produit de formule (A) est tel que Hal représente de préférence un atome de brome mais peut également représenter un atome de chlore ou d'iode.

La réaction du composé de formule (B) avec le composé (II) peut être opérée par une amination réductrice, effectuée en deux étapes :

La première étape est la formation de l'imine du produit de formule (B) par addition du produit de formule (II) sur la fonction aldéhyde du produit de formule (B), dans un solvant tel que par exemple le dichlorométhane, dichloroéthane ou tétrahydrofuranne de préférence dans des conditions de catalyse acide, par exemple en présence d'amberlyst H⁺ ou encore d'acide acétique.

La seconde étape est la réduction de l'imine ainsi formée par un agent réducteur tel que par exemple NaBH₃CN ou NaBH(OAc)₃ (préparé au préalable à partir de borohydrure de sodium et d'acide acétique).

Le produit de formule (C) est transformé en produit de formule (D) par un agent d'acylation par exemple un halogénure d'acide tel que notamment le chlorure de butyryle ou de valéryle, en présence de carbonate de sodium ou de potassium, de pyridine ou de triéthylamine, dans un solvant tel que par exemple l'acétone, le tétrahydrofuranne, le dichlorométhane ou le dichloroéthane.

Le produit de formule (D) est transformé en produit de formule (E) par addition du radical R'₂ sur la fonction cyano: cette amidification peut se faire dans un solvant par exemple un alcool tel que le méthanol ou l'éthanol, ou par réaction du composé R'₂-SH sur la fonction cyano dans un solvant tel que par exemple le toluène, le tétrahydrofuranne ou le dichloroéthane.

Dans le cas où R'₂ représente un radical comprenant un atome de soufre, la réaction du produit de formule (D) pour obtenir le produit de formule (E) correspondant est une thioamidification effectuée par action d'un composé de formule R₁₀-SH dans lequel R₁₀ représente le reste du radical R'₂ tel que défini ci-dessus et notamment un radical alkyl linéaire ou ramifié renfermant au plus 4 atomes de carbone ou un radical phényl, ces radicaux étant éventuellement substitués ainsi qu'il est indiqué ci-dessus.

La thioamidification peut être réalisée par exemple en faisant barboter le composé de formule R₁₀-SH tel que défini ci-dessus dans un solvant tel que par exemple un alcool tel que l'éthanol ou le méthanol ou encore le dichloroéthane, le dichlorométhane, le toluène, le tétrahydrofuranne en présence d'une base, par exemple la triéthylamine.

La thioamidification peut également être réalisée avec le réactif R₁₀SNa ou R₁₀SK dans les mêmes conditions de solvant définies pour R₁₀SH.

La réaction de substitution de l'atome d'oxygène par un atome de soufre pour obtenir le produit de formule (L) peut être réalisée par exemple par le réactif de Lawesson.

La réaction de cyclisation du produit de formule (E) en produit de formule (I') peut être réalisée soit par une catalyse acide en utilisant par exemple l'amberlyst H⁺, l'acide tosylique ou l'acide sulfurique, dans un solvant tel que par exemple le toluène, l'acétate d'éthyle, le dichlorométhane, le dichloroéthane, soit avec le pentachlorure dans la pyridine ou la diméthylaminopyridine, soit encore dans la diméthylsulfone.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

On trouvera une liste de différents groupements protecteurs utilisables par exemple dans le brevet BF 2 499 995.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acides des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acides peuvent être protégées par exemple sous forme d'esters formés avec les esters facilement clivables tels que les esters benzyliques ou ter butyliques ou des esters connus dans la chimie des peptides.

Comme pour les produits de formule (I'), les produits de formules (D) et (E) peuvent être soumis, au cours du procédé décrit ci-dessus à diverses réactions indiquées ci-dessus et notamment des réactions concernant le substituant R'₄ tel que défini ci-dessus.

Ainsi le produit de formule (E₁) peut être transformé en produit (E₂) avant de poursuivre la synthèse, comme il est indiqué ci-dessus pour la transformation du produit (D₁) en produit (D₂) .

Selon les valeurs de R'₁, R'₂, R'₃ et R'₄, les produits de formules (I'), (I'₁) ou (I'₂) telles que définies ci-dessus constituent ou non des produits de formule (I).

Les réactions auxquelles les produits de formules (D), (E) et (I') telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions de salification par une base minérale ou organique ou d'estérification : ces réactions d'estérification et de salification peuvent être réalisées selon les méthodes usuelles connues de l'homme du métier.
b) Les éventuelles transformations de fonctions ester en fonction acide peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
d) Les éventuelles fonctions carboxy libre ou estérifié des produits décrits ci-dessus peuvent être, si désiré, réduites en fonction alcool par les méthodes connues de l'homme de métier : pour les fonctions carboxy estérifié, on peut utiliser notamment l'hydrure de lithium et d'aluminium dans un solvant tel que par exemple le tétrahydrofuranne ou encore le dioxane ou l'éther éthylique.
   Pour les fonctions carboxy libre, on peut utiliser notamment par l'hydrure de bore.
e) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou par de l'acide acétique au reflux.
f) Les éventuels groupements comprenant un atome de soufre des produits décrits ci-dessus peuvent être, si désiré, transformés en fonction sulfoxyde ou sulfone correspondantes dans les conditions usuelles connues de l'homme du métier telles que par exemple au moyen de peracides comme par exemple l'acide peracétique ou l'acide métachloroperbenzoïque ou encore par l'ozone, l'oxone, le périodate de sodium dans un solvant tel que par exemple le chlorure de méthylène ou le dioxane à la température ambiante.
   La fonction sulfoxyde peut être obtenue par un mélange équimolaire du produit renfermant un groupement alkylthio ou arylthio et du réactif tel que notamment un peracide.
   La fonction sulfone peut être obtenue par un mélange du produit renfermant un groupement alkylthio ou arylthio avec un excès du réactif tel que notamment un peracide.
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazole dans les conditions usuelles connues de l'homme du métier telles que par exemple par cycloaddition d'un azidure métallique tel que par exemple un azidure de trialkylétain sur la fonction nitrile ainsi qu'il est indiqué dans la méthode décrite dans l'article référencé comme suit : J. Organometallic Chemistry., 33, 337 (1971) KOZIMA S.& coll.
i) Les réactions de transformation de radical formyle en radical carbamoyle et de radical carbamoyle en radical nitrile sont réalisées selon les conditions usuelles connues de l'homme du métier.

Les produits de formule (I') sont en outre soumis le cas échéant aux réactions indiquées ci-dessous.
- l'élimination de groupements protecteurs, dans les conditions usuelles connues de l'homme de métier, notamment par une hydrolyse acide effectuée avec un acide tel que l'acide chlorhydrique, benzène sulfonique ou paratoluène sulfonique, formique ou trifluoroacétique ou encore par une hydrogénation catalytique. Le groupement phtalimido peut être éliminé par l'hydrazine.
- la salification est effectuée par un acide minéral ou organique selon les méthodes usuelles connues de l'homme du métier.
- le dédoublement de racémiques pour obtenir les formes optiquement actives des produits de formule (I) est effectuée selon les méthodes usuelles connues de l'homme du métier.

Les produits de formule (I) sont connus et décrits dans les demandes de brevets européens n° 0 465 368 et 0 503 162.

Les produits de formule (I) préparés selon le procédé tel que défini ci-dessus, ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient l'application en thérapeutique des produits de formule (I) préparés selon le procédé tel que défini ci-dessus à titre de médicaments, lesdits produits de formule (I) étant sous toutes les formes isomères possibles racémiques ou optiquement actives, ainsi que les sels d'addition avec les acides minéraux ou organiques pharmaceutiquement acceptables desdits produits de formule (I).

Les produits de formule (I) préparés selon le procédé défini ci-dessus ainsi que leurs sels d'addition avec les acides minéraux ou organiques pharmaceutiquement acceptables, peuvent être utilisés notamment à titre de médicaments, dans le traitement de l'hypertension artérielle, des insuffisances cardiaques, des insuffisances rénales et dans la prévention des resténoses post-angioplastie.

Ils peuvent également être utilisés dans le traitement de certains désordres gastro-intestinaux, gynécologiques et en particulier pour un effet relaxant au niveau de l'utérus, sous forme de compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Certains produits de départ de formule (A) sont connus et peuvent être préparés par exemple comme indiqué dans le brevet européen EP 0 503 162.

Les produits de formule (II) sont des produits commercialisés ou peuvent être préparés comme indiqué dans la demande de brevet européen n° 0 465 368.

Les produits de formule (III) sont des produits commercialisés ou peuvent être aisément préparés à partir de l'acide ou d'un dérivé approprié.

La présente invention a également pour objet, à titre de produits industriels nouveaux, les composés de formules (B), (C) , (D), (D₁), (D₂), (E) , (E₁), (E₂), (L) , (L₁) et (L₂).

Les exemples décrits ci-après illustrent la présente invention sans toutefois la limiter.

### EXEMPLE 1 : Acide 2-butyl 4-(méthylthio) 1-[[2'-((((propylamino) carbonyl) amino) sulfonyl) 1,1'(biphényl) 4-yl] méthyl] 1H-imidazole 5-carboxylique di sel de potassium

### STADE A : N-[(diméthylamino) méthylène] 4'-formyl (1,1'-biphényl) 2-sulfonamide

On introduit 10 g de N-[(diméthylamino) méthylène] 4'-bromométhyl (1,1'-biphényl) 2-sulfonamide, 8,14 g d'hexaméthylènetétramine, 80 ml d'acide acétique à 50 %. On agite pendant 1 heure à 120°C. Après l'addition de 220 ml d'eau, le mélange réactionnel est neutralisé par une solution saturée de de bicarbonate de sodium, extrait avec de dichlorométhane, séché et évaporé pour donner 7,99 g du produit attendu.
SM MH⁺ 317
RMN (CDCl₃)
2,71 et 2,77 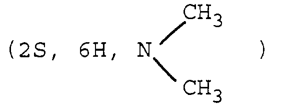 7,17 (s, N=CH-N)
7,15 à 7,91 (système AB, 6H, aromatiques)
10,11 (CH0, 1H)

### STADE B : aminocyano-acétate d'éthyle mono(4-méthylbenzènesulfonate)

On introduit 50 g de (E) cyano(hydroxyimino) acétate d'éthyle dans 440 ml d'eau et 340 ml de solution de sulfate acide de sodium, ajoute 150 g de d'hydrosulfite de sodium et agite à environ 350°C pendant environ 30 minutes.

La solution est saturée par ajout de chlorure de sodium, extraite au chlorure de méthylènee et mise à sec.

Le résidu est repris par de l'éther : on ajoute 44 g de d'acide paratoluène sulfonique dans 110 ml d'éthanol, 740 ml d'éther, élimine les solvants et obtient 35 g de produit attendu. F = 128-130°C.

### STADE C : cyano-[[[2'-[[[(diméthylamino) méthylène] amino] sulfonyl] (1,1'-biphényi) 4-yl] méthyl] amino] acétate d'éthyle

Une solution de 13 g d'aminocyano acétate d'éthyle mono (4-méthylbenzènesulfonate) obtenu au stade B ci-dessus, 1 g d'amberlyst 15⁻(H⁺) et 12,3 g du produit obtenu au stade A dans le dichlorométhane est agité aux reflux pendant environ 7 h. Cette solution est refroidie puis ajoutée à une suspension contenant 3,1 g de borohydrure de sodium, 14,2 ml d'acide acétique glacial et 60 ml de dichlorométhane à 10°C.

La solution ainsi obtenue est agitée pendant environ 18 h. On ajoute 200 ml d'eau, filtre le précipité obtenu. La phase organique est lavée avec de l'eau, séchée et concentrée sous pression réduite. Après purification par chromatographie sur silice dichlorométhane/acétate d'éthyle 85/15) on obtient 9 g du produit attendu sous forme d'huile.
Rf (dichlorométhane - acétate d'éthyle 85/15) = 0,20
IR (CHCl₃)
3340 (-NH-) 1754 (C=O) 1628 (N=CH-N ) 1344, 1148 (SO₂).
RMN (CDCl₃)
1,37 (t, J=7, CO₂CH₂CH₃) ; 2,78 et 2,79 (2S, diméthylamine) ; 2,21 (NH mobile) ; 3,94 et 4,06 (NCH₂-C=, système AB) ; 4,35 (q, J=7, CO₂CH₂CH₃) ; 4,35 (maqué N-CH-C≡N) ; 7,14 (s, N=CH-N) ; 7,22 (dd, 1H, aromatique) ; 7,51 (m, 2H, aromatiques) ; 7,39 (s, aromatiques).

### STADE D : cyano-[[[2'-[[[(diméthylamino) méthylène] amino] sulfonyl] (1,1'-biphényl) 4-yl] méthyl] (1-oxopentyl) amino] acétate d'éthyle

A une solution de 11,1 g de cyano-[[[2'-[[[(diméthylamino) méthylène] amino] sulfonyl] (1,1'-biphényl) 4-yl] méthyl] amino] acétate d'éthyle obtenu au stade C ci-dessus, dans 200 ml d'acétone sec, on ajoute 5,53 g de K₂CO₃ et 3,33 ml de chlorure de valéryle. Après agitation pendant 20 h à température ambiante, la solution est filtrée et mise à sec. On purifie par chromatographie avec de dichlorométhane-acétate d'éthyle 85-15.
On récupère 11,6 g d'un solide blanc.
Rf (dichlorométhane-acétate d'éthyle 85-15) = 0,24

### STADE E : 3-amino 2-[[[2'-[[[(diméthylamino) méthylène] amino] sulfonyl] (1,1'-biphényl) 4-yl] méthyl] (1-oxopentyl) amino] 3-(méthylthio) 2-propénoate d'éthyle

On introduit 250 mg de cyano-[[[2'-[[[(diméthylamino) méthylène] amino] sulfonyl] (1,1'-biphényl) 4-yl] méthyl] (1-oxopentyl) amino] acétate d'éthyle obtenu au stade D ci-dessus, 2,5 ml d'éthanol et 7 *µ*l diéthylamine, refroidit à -5°C et fait barboter du méthane thiol pendant 10 min. On agite à -5°C, pendant 1 h, laisse revenir à température ambiante et on agite pendant 48 h. On fait barboter de l'azote pendant 1 h à température ambiante et élimine les solvants sous pression réduite. Après chromatographie sur silice, (éluant : dichlorométhane-acétate d'éthyle 80-20), on recueille 0,91 g de produit attendu.

### STADE F : 2-butyl 4-(méthylthio) 1-[[2'-[[[(diméthylamino) méthylène] amino] sulfonyl] (1,1'-biphényl) 4-yl] méthyl] 1H-imidazole 5-carboxylate d'éthyle

On introduit 50 mg de 3-amino 2-[[[2'-[[[(diméthylamino) méthylène] amino] sulfonyl] (1,1'-biphényl) 4-yl] méthyl] (1-thiopentyl) amino] 3-(méthylthio) 2-propénoate d'éthyle obtenu au stade E ci-dessus, 500 *µ*l de toluène, et une quantité catalytique d'amberlyst 15. On chauffe au reflux pendant 20 h, filtre l'amberlyst, lave, élimine les solvants sous pression réduite et obtient 0,20 g de produit attendu.

### STADE G : 2-butyl 4-(méthylthio) 1-[[2'-[[[ amino] sulfonyl] i (1,1'-biphényl) 4-yl] méthyl] 1H-imidazole 5-carboxylate d'éthyle

On porte au reflux une solution de 43,5 g de 2-butyl 4-(méthylthio) 1-[[2'-[[[(diméthylamino) méthylène] amino] sulfonyl] (1,1'-biphényl) 4-yl] méthyl] 1H-imidazole 5-carboxylate d'éthyle obtenu comme au stade F ci-dessu dans 800 ml de méthanol, 400 ml d'acide chlorhydrique concentré durant 3 heures. Le méthanol est évaporé, la phase restante est ajustée à pH 5-6 par addition de soude 6N. On extrait 3 fois à l'éther (400 ml) sèche sur Na₂SO₄ puis évapore le solvant. On obtient 37,5 g de produit brut. On empâte dans un mélange de 50 ml d'éthanol + 500 ml d'éther isopropylique, puis filtre. On obtient 31,4 g de produit attendu. F = 127-129°C.

### STADE H : 2-butyl 4-(méthylthio) 1-[[2'-[[[(propylamino) carbonyl] amino] sulfonyl] (1,1'-biphényl) 4-yl] méthyl] 1H-imidazole 5-carboxylate d'éthyle

On porte au reflux, sous atmosphère inerte, une solution de 10 g de 2-butyl 4-(méthylthio) 1-[[2'-[[[amino] sulfonyl] (1,1'-biphényl) 4-yl] méthyl] 1H-imidazole 5-carboxylate d'éthyle obtenu au stade G ci-dessus, 5,6 g de carbonate de potassium (préalablement broyé et séché sous pression réduite à 100°C) durant 2 h, dans 70 ml diméthyl éther de l'éthylène glycol. Après une heure on ajoute 4 ml d'isocyanate de n-propyle et poursuit le chauffage pendant 30 minutes. On laisse revenir à température ambiante, puis on évapore le solvant. On ajoute 100 ml d'eau au résidu puis on ajuste à pH 5-6 par addition lente d'acide chlorhydrique 1N. Le produit cristallise. On le recristallise dans 50 ml d'acétate d'éthyle, on obtient 10,1 g de produit attendu.

### STADE I : Acide 2-butyl 4-(méthylthio) 1-[[2'-[[[(propylamino) carbonyl] amino] sulfonyl] (1,1'-biphényl) 4-yl] méthyl] 1H-imidazole 5-carboxylique di sel de potassium

A une solution de 2 g de 2-butyl 4-(méthylthio) 1-[[2'-[[[(propylamino) carbonyl] amino] sulfonyl] (1,1'-biphényl) 4-yl] méthyl] 1H-imidazole 5-carboxylate d'éthyle obtenu au stade H ci-dessus, dans 40 ml d'éthanol, on ajoute à 0°C, 2,3 ml d'une solution de potasse 6N. On laisse revenir à température ambiante. Après 72 heures, on essore le précipité et lave avec 4 ml d'éthanol puis par 4 ml d'acétate d'éthyle. On obtient, après séchage, 2,04 g de produit recherché F > à 260°C.

| Analyse pour C₂₆H₃₀K₂N₄O₅S₂ | | | | |
|---|---|---|---|---|
| | C | H | N | S |
| % calculés | 50,30 | 4,87 | 9,02 | 10,33 |
| % trouvés | 50,5 | 4,9 | 9,0 | 10,3 |

### EXEMPLE 2 : Acide 2-butyl 4-(méthylthio) 1-[[2'-[[[(propylamino) carbonyl] amino] sulfonyl] (1,1'-biphényl) 4-yl] méthyl] 1H-imidazole 5-carboxylique di sel de potassium

1) cyano-[[[2'-[[[(diméthylamino) méthylène] amino] sulfonyl] (1,1'-biphényl) 4-yl] méthyl] amino] acétate d'éthyle
   On mélange 2 g d'aminocyano acétate d'éthyle mono (4-méthylbenzènesulfonate) obtenu au stade B de l'exemple 1, 1,9 g de N-[(diméthylamino) méthylène] 4'-formyl (1,1'-biphényl) 2-sulfonamide obtenu au stade A de l'exemple 1, et agite dans le dichlorométhane à 35°C pendant 3h puis ajoute 0,396 g de cyano borohydrure de sodium et la solution est laissée sous agitation à température ambiante pendant 18 h. Le mélange réactionnel est filtré, lavé à l'eau et évaporé. On obtient, après chromatographie sur silice (de dichlorométhane-acétate - d'éthyle 85-15) 770 mg du produit attendu, identique à celui obtenu au stade C de l'exemple 1.
2) On procède alors comme à l'exemple 1 en remplaçant le produit obtenu au stade C de l'exemple 1 par le produit obtenu ci-dessus, pour obtenir le produit attendu acide 2-butyl 4-(méthylthio) 1-[[2'-[[[(propylamino) carbonyl] amino] sulfonyl] (1,1'-biphényl) 4-yl] méthyl] 1H-imidazole 5-carboxylique di sel de potassium.

### EXEMPLE 3 : Acide 2-butyl 4-(méthylthio) 1-[[2'-((((propylamino) carbonyl) amino) sulfonyl) 1,1'(biphényl) 4-yl] méthyl] 1H-imidazole 5-carboxylique di sel de potassium

### STADE A : [[[2'-(aminosulfonyl) (1,1'-biphényl) 4-yl] méthyl] (1-oxopentyl) amino] cyanoacétate d'éthyle

1,0 g de cyano-[[[2'-[[[(diméthylamino) méthylène] amino] sulfonyl] (1,1'-biphényl) 4-yl] méthyl] (1-oxopentyl) amino] acétate d'éthyle obtenu au stade D de l'exemple 1, dans 20 ml d'éther diméthylique de l'éthylène glycol et 10 ml d'acide chlorhydrique concentré, sont agités pendant 30 minutes à 100°C. Après refroidissement à température ambiante, on ajuste le pH de la solution à pH ∼ 6 par addition de soude 6N. On extrait la solution à l'acétate d'éthyle. Les phases organiques sont lavées à l'eau saturée de chlorure de sodium et séchées. On purifie par chromatographie (éluant : acétate d'éthyle-heptane 4-1). On récupère 320 mg du produit attendu.

### STADE B : préparation de cyano-[[[2'-[[[(propylamino) carbonyl] amino] sulfonyl] (1,1'-biphényl) 4-yl] méthyl] (1-oxopentyl) amino] acétate d'éthyle

7,2 g de [[[2'-(aminosulfonyl) (1,1'-biphényl) 4-yl] méthyl] (1-oxopentyl) amino] cyanoacétate d'éthyle obtenu au stade A ci-dessus, 6,5 g de carbonate de potassium anhydre et 1,48 ml de n-propyl isocyanate sont agités dans 180 ml d'acétone au reflux pendant 2 h 1/2. Après refroidissement, la solution est traitée par de l'acide chlorhydrique 2N. Le résidu obtenu est dissous dans un mélange aqueux de dichlorométhane. Extraction du dichlorométhane, séchage et élimination des solvants sous pression réduite, on recristallise le produit dans l'acétate d'éthyle et obtient 6,5 g de produit attendu (solide blanc).
Rf (SiO₂, acétate d'éthyle) = 0,48
MP 131-133°C.

### STADE C : 3-amino 2-[[[2'-[[[(propylamino) carbonyl] amino] sulfonyl] (1,1'-biphényl) 4-yl] méthyl] (1-oxopentyl) amino] 3-(méthylthio) 2-propénoate d'éthyle

Une solution de 8,5 g de cyano-[[[2'-[[[(propylamino) carbonyl] amino] sulfonyl] (1,1'-biphényl) 4-yl] méthyl] (1-oxopentyl) amino] acétate d'éthyle obtenu au stade B ci-dessus, et 2,4 ml de triéthylamine dans 100 ml de dichlorométhane est saturée par du méthanethiol et refroidie à -20°C. On laisse remonter la température puis maintient la solution sous une pression d'azote de 10 bar à 40°C pendant 48 h. Après concentration, le résidu est purifié par chromatographie sur silice (éluant : dichlorométhane-MeOH 20-1). On obtient 8,0 g d'une produit attendu.
Rf (SiO₂, dichlorométhane-MeOH 20-1) = 0,39

### STADE D : 2-butyl 4-(méthylthio) 1-[[2'-[[[(propylamino) carbonyl] amino] sulfonyl] (1,1'-biphényl) 4-yl] méthyl] 1H-imidazole 5-carboxylate d'éthyle

180 mg de pentachlorure de phosphore sont mis en suspension dans 16 ml de de dichlorométhane sous atmosphère d'argon. Après refroidissement à -78°C, on ajoute 230 mg de diméthylamino pyridine en solution dans 10 ml de dichlorométhane anhydre et on agite encore 10 minutes. Une solution de 500 mg de 3-amino 2-[[[2'-[[[(propylamino) carbonyl] amino] sulfonyl] (1,1'-biphényl) 4-yl] méthyl] (1-oxopentyl) amino] 3-(méthylthio) 2-propénoate d'éthyle obtenu au stade C ci-dessus, dans 25 ml de dichlorométhane, est ajoutée à -78°C et on agite 18 H à température ambiante. Le précipité est filtré, lavé à l'eau et séché. Après chromatographie sur silice (éluant : dichlorométhane-MeOH 20-1), on obtient 330 mg de produit brut que l'on cristallise dans l'acétate d'éthyle/n-heptane et obtient 270 mg d'un solide blanc.
Rf (SiO₂, acétate d'éthyle/n-heptane 4-1) 0,42
F = 127-129°C

### STADE E : Acide 2-butyl 4-(méthylthio) 1-[[2'-[[[(propylamino) carbonyl] amino] sulfonyl] (1,1'-biphényl) 4-yl] méthyl] 1H-imidazole 5-carboxylique

Une solution de 130 mg de 2-butyl 4-(méthylthio) 1-[[2'-[[[(propylamino) carbonyl] amino] sulfonyl] (1,1'-biphényl) 4-yl] méthyl] 1H-imidazole 5-carboxylate d'éthyle dans 10 ml de méthanol et 3,1 ml de soude 2N est agitée pendant 18 h à température ambiante. Le méthanol est évaporé et la solution aqueuse restante est acidifiée à pH ∼ 6 par addition d'acide chlorhydrique 2N. Le précipité est filtré, lavé à l'eau et séché sous pression réduite. On obtient 115 mg de produit attendu.
F = 109-111°C

### STADE F : Acide 2-butyl 4-(méthylthio) 1-[[2'-[[[(propylamino) carbonyl] amino] sulfonyl] (1,1'-biphényl) 4-yl] méthyl] 1H-imidazole 5-carboxylique di sel de potassium

Par salification de l'acide 2-butyl 4-(méthylthio) 1-[[2'-[[[(propylamino) carbonyl] amino] sulfonyl] (1,1'-biphényl) 4-yl] méthyl] 1H-imidazole 5-carboxylique obtenu au stade C ci-dessus, à l'aide d'une solution aqueuse de potasse comme indiqué à l'exemple 1, on obtient le produit attendu.

### EXEMPLE 4 : Acide 2-butyl 4-(méthylthio) 1-[[2'-([[(propylamino) carbonyl] amino] sulfonyl] (1,1'-biphényl) 4-yl] méthyl] 1H-imidazole 5-carboxylique di sel de potassium

1) cyano-[[[2'-[[[(diméthylamino) méthylène] amino] sulfonyl] (1,1'-biphényl) 4-yl] méthyl] (1-oxopentyl) amino] acétate d'éthyle
   200 mg de 3-amino 2-[[[2'-[[[(propylamino) carbonyl] amino] sulfonyl] (1,1'-biphényl) 4-yl] méthyl] (1-oxopentyl) amino] 3-(méthylthio) 2-propénoate d'éthyle obtenu au stade C de l'exemple 3, est dissous dans 5 ml de diméthyl éther de l'éthylène glycol et 1 ml d'acide sulfurique concentré est ajouté à température ambiante. Après 1 h d'agitation on ajoute de l'eau en refroidissant avec un bain de glace. On extrait avec du dichlorométhane, lave les phases organiques, séche et évapore de solvant sous pression réduite. On obtient 180 mg de produit brut que l'on cristallise dans l'acétate d'éthyle et obtient 140 mg de produit attendu.
   F = 130-132°C.
2) On poursuit la synthèse comme indiqué à l'exemple 3 en remplaçant le produit obtenu au stade D de l'exemple 3 par le produit obtenu ci-dessus pour obtenir le produit attendu.

### EXEMPLE 5 : Acide 2-butyl 4-(méthylthio) 1-[[2'-[[[(propylamino) carbonyl] amino] sulfonyl] (1,1'-biphényl) 4-yl] méthyl] 1H-imidazole 5-carboxylique di sel de potassium

### STADE A : cyano-[[[2'-[[[(propylamino) carbonyl] amino] sulfonyl] (1,1'-biphényl) 4-yl] méthyl] amino] acétate d'éthyle

Une solution de 2 g de formyl (1,1'-biphényl) 2-[2'-[[(propylamino) carbonyl] amino] sulfonyl] préparé comme indiqué à l'exemple 1 stade A à partir du dérivé sulfonamide approprié et de 1,83 g de cyanoamino acétate d'éthyle préparé comme indiqué dans la demande de brevet européen n° 0 465 368 dans 40 ml de dichlorométhane est agitée à 35°C pendant 6 h. Cette solution est ajoutée à la suspension contenant 11,55 mmoles de borohydrure de sodium, 2 ml d'acide acétique glacial et 30 ml de dichlorométhane, à +10°C. La solution ainsi obtenue est agitée pendant 16 heures. On ajoute de l'eau, extrait la phase organique, lave et sèche puis évaporate les solvants sous pression réduite et chromatographie sur silice (éluant : dichlorométhane-MeOH 30-1). On obtient 1,8 g de produit attendu.
Rf (SiO₂ ; dichlorométhane-MeOH 20-1) = 0,39

### STADE B : cyano-[[[2'-[[[(propylamino) carbonyl] amino] sulfonyl] (1,1'-biphényl) 4-yl] méthyl] (1-oxopentyl) amino] acétate d'éthyle

1,7 g de cyano-[[[2'-[[[(propylamino) carbonyl] amino] sulfonyl] (1,1'-biphényl) 4-yl] méthyl] amino] acétate d'éthyle obtenu au stade A ci-dessus, est dissous dans 50 ml d'acétone, 0,79 g de carbonate de potassium et 0,45 ml de chlorure de valéryle sont ajoutés et le mélange ainsi obtenu est agité 6 h à température ambiante. On filtre l'insoluble et on amène à sec le filtrat, qui après purification par chromatographie (éluant : dichlorométhane-acétate d'éthyle 4-1, donne 295 mg de produit attendu.
Rd (SiO₂, dichlorométhane-acétate d'éthyle 4-1) = 0,30

On poursuit la synthèse comme indiqué aux stades C, D, E et F de l'exemple 3 pour obtenir le produit attendu.

### EXEMPLE 6 : Acide 2-butyl 4-(méthylthio) 1-[[2'-((((propylamino) carbonyl) amino) sulfonyl) 1,1'(biphényl) 4-yl] méthyl] 1H-imidazole 5-carboxylique di sel de potassium

### STADE A : 3-amino 2-[[[2'-[[[(propylamino) carbonyl] amino] sulfonyl] (1,1'-biphényl) 4-yl] méthyl] (1-thiopentyl) amino] 3-(méthylthio) 2-propénoate d'éthyle

Une solution de 1 g de 3-amino 2-[[[2'-[[[(propylamino) carbonyl] amino] sulfonyl] (1,1'-biphényl) 4-yl] méthyl] (1-oxopentyl) amino] 3-(méthylthio) 2-propénoate d'éthyle obtenu au stade C de l'exemple 3 et 342 mg du réactif de Lawesson dans 10 ml de diméthyl éther de l'éthylène glycol anhydre est agitée à température ambiante, pendant 2 jours. La solution est concentrée sous pression réduite et le résidu repris par un mélange de dichlorométhane-eau (1-1). Après extraction et lavage à l'eau, séchage et élimination des solvants, on purifie l'huile restante et obtient 506 mg du produit attendu.
Rf (SiO₂ acétate d'éthyle/n-heptane 4-1) 0,45

### STADE B : 2-butyl 4-(méthylthio) 1-[[2'-[[[(propylamino) carbonyl] amino] sulfonyl] (1,1'-biphényl) 4-yl] méthyl] 1H-imidazole 5-carboxylate d'éthyle

Une solution de 150 mg de 3-amino 2-[[[2'-[[[(propylamino) carbonyl] amino] sulfonyl] (1,1'-biphényl) 4-yl] méthyl] (1-thiopentyl) amino] 3-(méthylthio) 2-propénoate d'éthyle obtenu au stade A ci-dessus, 58 *µ*l de diméthyl sulfone dans 5 ml de dichlorométhane est agitée au reflux durant 15 h. Après refroidissement à température ambiante, la solution est lavée avec une solution saturée de de chlorure de sodium, et évaporée à sec sous pression réduite. Après chromatographie sur silice (éluant : acétate d'éthyle/n-heptane 2/1), on obtient 35 mg de produit attendu.

On poursuit la synthèse ensuite comme indiqué aux stades E et F de l'exemple 3 pour obtenir le produit attendu.

## Revendications

1. Procédé de préparation de produits de formule (I) : 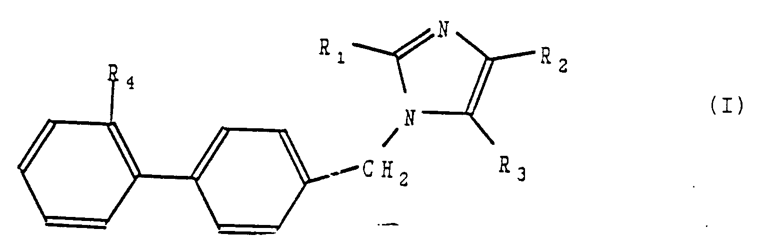 dans laquelle :
R₁ représente un radical alkyle ou alkényle linéaire ou ramifié renfermant au plus 4 atomes de carbone,
R₂ et R₃, identiques ou différents, sont choisis parmi :
a) l'atome d'hydrogène ; le radical mercapto ; les radicaux formyle ; carboxy libre, salifié ou estérifié ; les atomes d'halogène ; le radical hydroxyle ; cyano ; nitro ; acyle ;
b) les radicaux alkyle, alkényle, alcoxy, alkylthio dans lequel l'atome de soufre est éventuellement mono ou dioxydé, ces radicaux étant linéaires ou ramifiés et renfermant au plus 6 atomes de carbone, les radicaux phényle, benzoyle, phénylthio dans lequel l'atome de soufre est éventuellement mono ou dioxydé, tous ces radicaux étant éventuellement substitués par un ou plusieurs radicaux identiques ou différents choisis parmi :
les atomes d'halogène,
les radicaux hydroxyle, trifluorométhyle, cyano, nitro, formyle, alkyle et alcoxy renfermant au plus 4 atomes de carbone, phényle et carboxy libre, salifié ou estérifié,
c) les radicaux 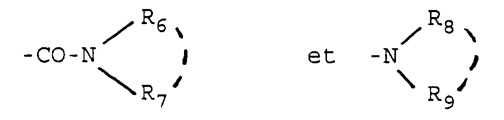 dans lesquels :
ou bien R₆, R₇, R₈ et R₉, identiques ou différents, sont choisis parmi l'atome d'hydrogène, les acides aminés, les radicaux alkyle, ces radicaux renfermant au plus 6 atomes de carbone, phényle, benzyle, phénéthyle,
ou bien d'une part R₆ et R₇ et d'autre part R₈ et R₉ forment respectivement avec l'atome d'azote auxquels ils sont liés un radical hétérocyclique, ces radicaux identiques ou différents étant choisis parmi les radicaux imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, nitro, alkyle et alcoxy, ces radicaux renfermant au plus 6 atomes de carbone et phényle,
R₄ représente le radical cyano, carboxy libre, salifié ou estérifié, le radical -(CH₂)ₚ-SO₂-X-R₁₀ dans lequel p représente les valeurs 0 et 1, X représente les radicaux NH-, -NH-CO-, -NH-CO-O-, -N=CH-N-R₁₃, -NH-CO-NH- ou une simple liaison et R₁₀ et R₁₃ identiques ou différents, représentent un atome d'hydrogène, un radical alkyle ou alkényle linéaire ou ramifié, renfermant au plus 6 atomes de carbone et éventuellement substitués, pyridyle, phényle, benzyle, nitropyridyle, pyrimidyle, tétrazolyle, diazolyle, pipéridinyle, alkylpipéridinyle, thiazolyle, alkylthiazolyle, tétrahydrofuranyle, méthyltétrahydrofuranyle ;
les radicaux alkyl et alkényl étant éventuellement substitués par un ou plusieurs radicaux choisis parmi les atomes d'halogène, le radical hydroxyle, nitro, les radicaux alkyle, alkényle et alcoxy renfermant au plus 4 atomes de carbone, le radical trifluorométhyle, cyano, amino, mono et dialkylamino, carboxy libre, salifié ou estérifié, phényl, tétrazolyle ; lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I), caractérisé en ce que l'on fait réagir un composé de formule (A) : 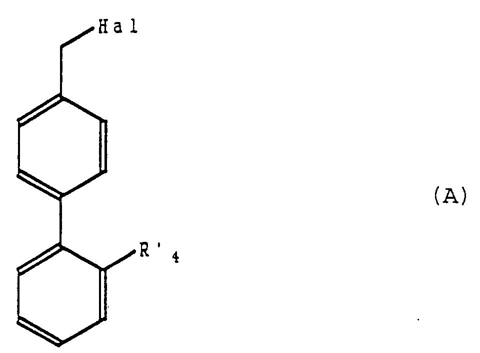 dans laquelle Hal représente un atome d'halogène et R'₄ a la signification indiquée ci-dessus pour R₄ dans laquelle les éventuelles fonctions réactives sont éventuellement protégées avec un agent d'oxydation, pour obtenir le composé de formule (B) : 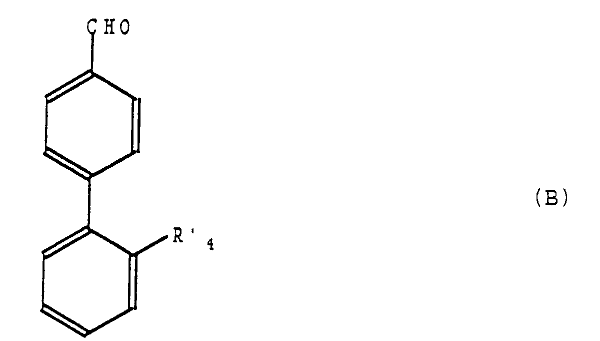 dans laquelle R'₄ a la signification indiquée ci-dessus, que l'on fait réagir avec un composé de formule (II) : 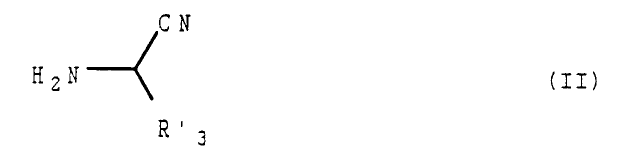 dans laquelle R'₃ a la signification indiquée ci-dessus pour R₃ dans laquelle les éventuelles fonctions réactives sont éventuellement protégées, pour obtenir un produit de formule (C) : 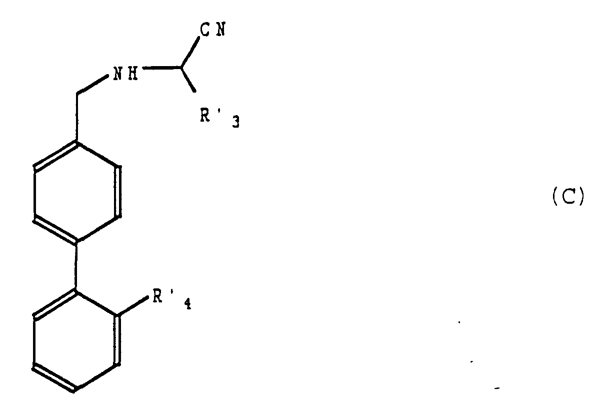 dans laquelle R'₃ et R'₄ ont les significations indiquées ci-dessus, que l'on fait réagir avec un composé de formule (III) :
R'₁-CO-Hal (III)
dans laquelle R'₁ a la signification indiquée ci-dessus pour R₁ dans laquelle les éventuelles fonctions réactives sont éventuellement protégées et Hal représente un atome d'halogène, pour obtenir un produit de formule (D) : 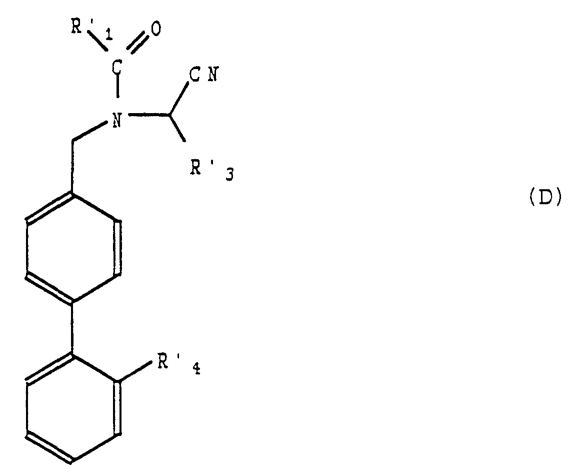 dans laquelle R'₁, R'₃ et R'₄ ont les significations indiquées ci-dessus, que l'on soumet à une réaction d'addition sur le radical CN, à l'aide d'un réactif capable d'introduire le substituant R'₂, R'₂ ayant la signification indiquée ci-dessus pour R₂ dans lequel les éventuelles fonctions réactives sont éventuellement protégées, le produit de formule (D) étant transformé en produit de formule (E) soit par amidification dans un solvant tel que par exemple un alcool tel que le méthanol ou l'éthanol, ou par réaction du composé R'₂-SH sur la fonction cyano dans un solvant tel que par exemple le toluène, le tétrahydrofuranne ou le dichloroéthane,
soit dans le cas où R'₂ représente un radical comprenant un atome de soufre, par thioamidification effectuée par action d'un composé de formule R₁₀-SH, R₁₀Sna ou R₁₀SK dans lequel R₁₀ représente le reste du radical R'₂ tel que défini ci-dessus,
une telle thioamidification pouvant être réalisée par exemple en faisant barboter le composé de formule R₁₀-SH, R₁₀Sna ou R₁₀SK tel que défini ci-dessus dans un solvant tel que par exemple un alcool tel que l'éthanol ou le méthanol ou encore le dichloroéthane, le dichlorométhane, le toluène, le tétrahydrofuranne en présence d'une base, par exemple la triéthylamine, pour obtenir un produit de formule (E) : 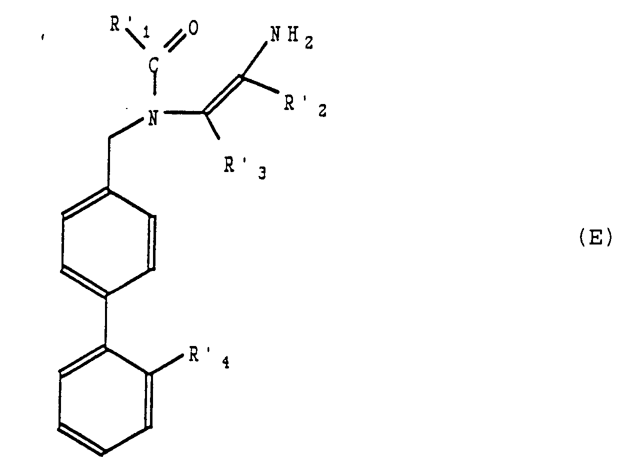
dans laquelle R'₁, R'₂, R'₃ et R'₄ ont les significations indiquées ci-dessus, que l'on soumet le cas échéant à une réaction de substitution de l'atome d'oxygène par un atome de soufre pour obtenir le produit de formule (L) : 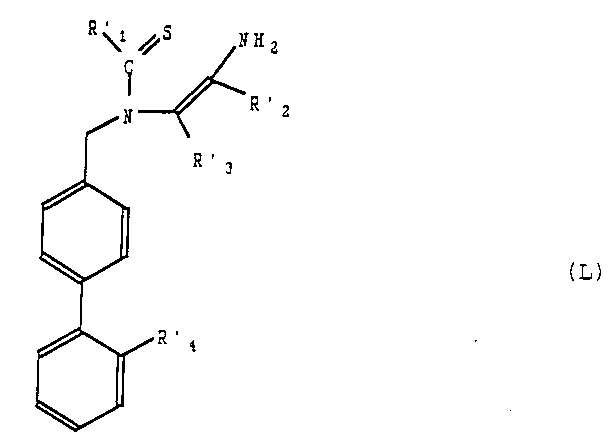 dans laquelle R'₁, R'₂, R'₃ et R'₄ ont les significations indiquées ci-dessus, produit de formule (E) ou produit de formule (L) que l'on soumet à une réaction de cyclisation réalisée soit par une catalyse acide en utilisant par exemple l'amberlyst H⁺, l'acide tosylique ou l'acide sulfurique, dans un solvant tel que par exemple le toluène, l'acétate d'éthyle, le dichlorométhane, le dichloroéthane, soit avec le pentachlorure dans la pyridine ou la diméthylaminopyridine, soit encore dans la diméthylsulfone, pour obtenir un produit de formule (I') : 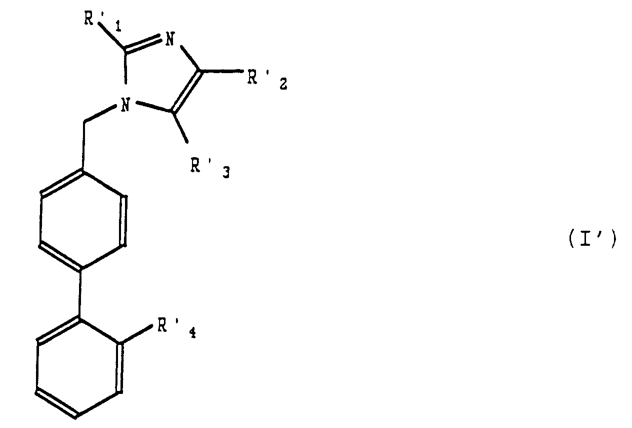 dans laquelle R'₁, R'₂, R'₃ et R'₄ ont les significations indiquées ci-dessus, étant entendu que les produits de formules (D), (E) et (L) au cours du procédé ci-dessus, et les produits de formule (I') peuvent être soumis, si désiré et si nécessaire, à l'une ou plusieurs des réactions suivantes, dans un ordre quelconque :
a) une réaction d'estérification de la fonction acide,
b) une réaction de saponification de la fonction ester,
c) une réaction de transformation de la fonction cyano en fonction acide,
d) une réaction de réduction de la fonction carboxy en fonction alcool,
e) une réaction de transformation de fonction alcoxy en fonction hydroxyle,
f) une réaction d'oxydation de groupement comprenant un atome de soufre en sulfoxyde ou sulfone correspondant,
g) une réaction de transformation de fonction alcool ou sulfone en fonction aldéhyde ou acide correspondante,
h) une réaction de transformation de radical nitrile en tétrazole,
i) une réaction de transformation du radical formyle en radical carbamoyle,
j) une réaction de transformation du radical carbamoyle en radical nitrile,
k) une réaction de transformation du radical 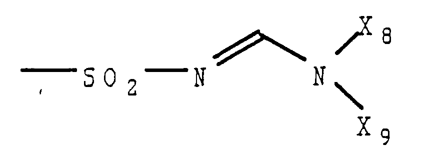 en radical 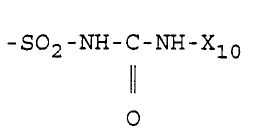 dans lesquels X₈, X₉ et X₁₀, identiques ou différents représentent un atome d'hydrogène ou un radical alkyle ou alkényle renfermant au plus 4 atomes de carbone éventuellement substitué,
produits de formule (I') qui peuvent représenter des produits de formule (I) ou que l'on soumet, si désiré et si nécessaire, pour obtenir les produits de formule (I) à l'une ou plusieurs des réactions suivantes, dans un ordre quelconque :
a) une réaction d'élimination des groupements protecteurs que portent les fonctions réactives protégées,
b) une réaction de salification par un acide minéral ou organique ou par une base pour obtenir le sel correspondant,
c) une réaction de dédoublement des formes racémiques en produits dédoublés
lesdits produits de formule (I) ainsi obtenus étant sous toutes les formes isomères possibles racémiques, énantiomères et diastéréoisomères.

2. Procédé selon la revendication 1 pour la préparation de produits de formule (I) répondant à la formule (I_{b}) : 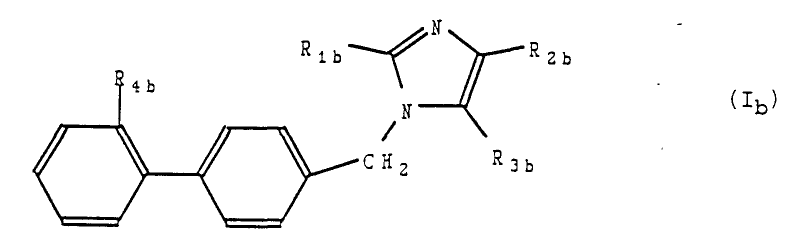 dans laquelle :
R_{1b} représente un radical alkyle renfermant au plus 4 atomes de carbone,
R_{3b} représente un atome d'hydrogène, un radical formyle, acyloxy, alkyle ou alcoxy éventuellement substitué, ou un radical carboxy libre, salifié ou estérifié par un radical alkyle,
R_{2b} représente un radical phénylthio, phénylsulfonyle, phénylsulfinyle, alkylthio, alkylsulfonyle ou alkylsulfinyle, éventuellement substitué, tel que dans tous ces radicaux que peuvent représenter R_{2b} et R_{3b}, les radicaux alkyle, alcoxy renfermant au plus 6 atomes de carbone, et les radicaux phényle sont éventuellement substitués par un ou plusieurs radicaux choisis parmi les atomes d'halogène et les radicaux hydroxyle, trifluorométhyle, acyloxy, carboxy libre salifié ou estérifié, phényle, pyridyle, tétrazolyle, alkyle et alcoxy renfermant au plus 4 atomes de carbone et eux-mêmes éventuellement substitués par un radical alcoxy renfermant au plus 4 atomes de carbone,
R_{4b} représente le radical cyano, carboxy libre, salifié ou estérifié, le radical -SO₂-X_{b}-R_{10b} dans lequel X_{b} représente les radicaux -NH-, -NH-CO-, -NH-CO-O-, -N=CH-N-R_{13b}, -NH-CO-NH- ou une simple liaison et R_{10b} et R_{13b} identiques ou différents, représentent un atome d'hydrogène, un radical méthyle, éthyle, propyle, vinyle, allyle, pyridyle, phényle, benzyle, nitropyridyle, pyrimidyle, tétrazolyle, diazolyle, pipéridinyle, alkylpipéridinyle, thiazolyle, alkylthiazolyle, tétrahydrofuranyle, méthyltétrahydrofuranyle,
lesdits produits de formule (I_{b}) étant sous toutes les formes isomères possibles racémiques, énantiomères et diastéréoisomères, ainsi que les sels d'addition avec les acides minéraux et organiques ou avec les bases minérales et organiques desdits produits de formule (I_{b}), caractérisé en ce que l'on utilise pour leur préparation telle que définie à la revendication 1, des produits de formules (A) , (II), (III) et un réactif capable d'introduire le radical R'₂ tel que défini à la revendication 1, dans lesquels R'₁, R'₂, R'₃ et R'₄ ont les valeurs indiquées ci-dessus respectivement pour R_{1b}, R_{2b}, R_{3b} et R_{4b} dans lesquelles les fonctions réactives sont éventuellement protégées.

3. Procédé selon la revendication 1 pour la préparation de produits de formule (I) répondant à la formule (I_{d}) : 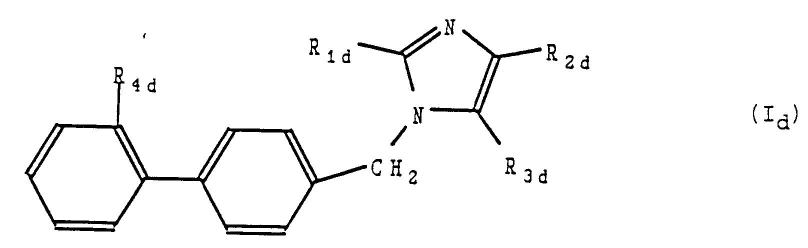 dans laquelle :
R_{1d} représente un radical alkyle renfermant au plus 4 atomes de carbone,
R_{3d} représente un radical :
- carboxy libre, salifié ou estérifié par un radical alkyle linéaire ou ramifié renfermant au plus 4 atomes de carbone, formyle, acyloxy, ou alkyle renfermant au plus 4 atomes de carbone éventuellement substitués par un radical hydroxyle, R_{2d} représente un radical :
phénylthio, phénylsulfonyle, phénylsulfinyle, alkylthio, alkylsulfonyle ou alkylsulfinyle,
dans lesquels le radical alkyle renferme au plus 4 atomes de carbone,
et R_{4d} représente le radical -SO₂-NH₂, -SO₂-NH-CO-O-R_{10d}, -SO₂-N=CH-NR_{13d}, ou -SO₂-NH-CO-NH-R_{10d} dans lesquels R_{10d} et R_{13d} identiques ou différents, sont choisis parmi l'atome d'hydrogène, le radical méthyle, éthyle, n-propyle et propényle, lesdits produits de formule (I_{d}) étant sous toutes les formes isomères possibles racémiques, énantiomères et diastéréoisomères, ainsi que les sels d'addition avec les acides minéraux et organiques ou avec les bases minérales et organiques, desdits produits de formule (I_{d}), caractérisé en ce que l'on utilise pour leur préparation telle que définie à la revendication 1, des produits de formules (A), (II), (III) et un réactif capable d'introduire le radical R'₂ tel que défini à la revendication 1, dans lesquels R'₁, R'₂, R'₃ et R'₄ ont les valeurs indiquées ci-dessus respectivement pour R_{1d}, R_{2d}, R_{3d} et R_{4d} dans lesquelles les fonctions réactives sont éventuellement protégées.

4. Procédé selon la revendication 1, caractérisé en ce que l'on utilise un produit de formule (A) dans laquelle R'₄ représente le radical -SO₂-NH₂, -SO₂-NH-CO-O-R_{10d}, -SO₂-N=CH-NR_{13d}, ou -SO₂-NH-CO-NH-R_{10d} dans lesquels R_{10d} et R_{13d} identiques ou différents, sont choisis parmi l'atome d'hydrogène, le radical méthyle, éthyle, n-propyle et propényle, dans lesquels les fonctions réactives sont éventuellement protégées, un produit de formule (II) dans laquelle R'₃ représente le radical : carboxy libre, salifié ou estérifié par un radical alkyle linéaire ou ramifié renfermant au plus 4 atomes de carbone, formyle, acyloxy, ou alkyle renfermant au plus 4 atomes de carbone éventuellement substitués par un radical hydroxyle,
un produit de formule (III) dans laquelle R'₁ représente un radical alkyle renfermant au plus 4 atomes de carbone, et un réactif capable d'introduire le substituant R'₂ tel que défini à la revendication 1, dans lequel R'₂ représente le radical : phénylthio, phénylsulfonyle, phénylsulfinyle, alkylthio, alkylsulfonyle ou alkylsulfinyle,
dans lesquels le radical alkyle renferme au plus 4 atomes de carbone, et les fonctions réactives sont éventuellement protégées.

5. Procédé selon la revendication 1, caractérisé en ce que l'on utilise un produit de formule (A) dans laquelle R'₄ représente le radical 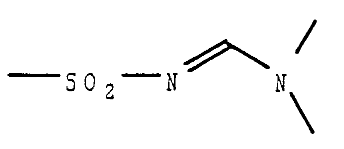 un produit de formule (II) dans laquelle R'₃ représente un radical alcoxy ou carboxy libre, salifié ou estérifié, un produit de formule (III) dans laquelle R'₁ représente un radical alkyle renfermant au plus 4 atomes de carbone et un réactif capable d'introduire le substituant R'₂ tel que défini à la revendication 1, dans lequel R'₂ représente un radical alkylthio ou phénylthio éventuellement oxydé sous forme de sulfoxyde ou de sulfone, ces radicaux alcoxy, alkylthio et phénylthio étant éventuellement substitués par un ou plusieurs radicaux choisis parmi les atomes d'halogène, les radicaux alkyl ou alcoxy renfermant au plus 4 atomes de carbone, trifluorométhyle, amino, mono ou dialkylamino, cyano, phényl, hydroxyle, carboxy libre, salifié ou estérifié, acyle et acyloxy.

6. Procédé selon la revendication 1, caractérisé en ce que l'on fait réagir un composé de formule (A₁) : 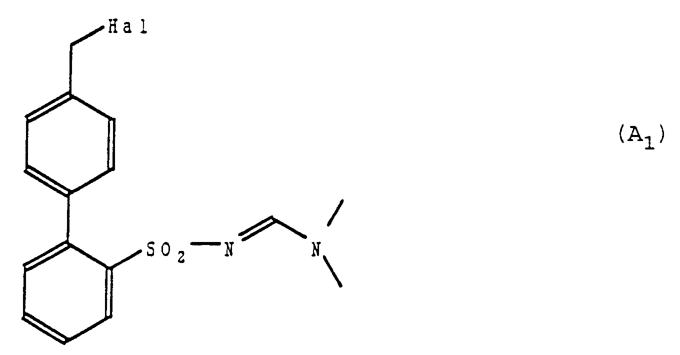 dans laquelle Hal représente un atome d'halogène avec un agent d'oxydation pour obtenir le composé de formule (B₁) : 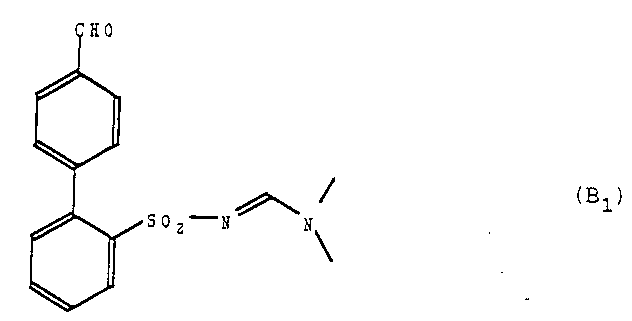 que l'on fait réagir avec un composé de formule (II) : 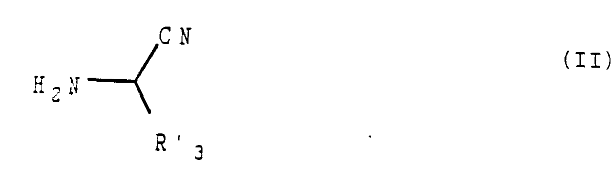 dans laquelle R'₃ a la signification indiquée ci-dessus, pour obtenir un produit de formule (C₁) : 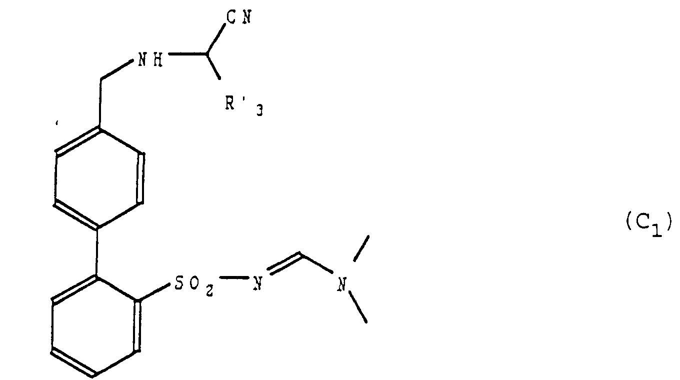 dans laquelle R'₃ a la signification indiquée ci-dessus, que l'on fait réagir avec un composé de formule (III) :
R'₁-CO-Hal (III)
dans laquelle R'₁ et Hal ont la signification indiquée ci-dessus, pour obtenir un produit de formule (D₁) : 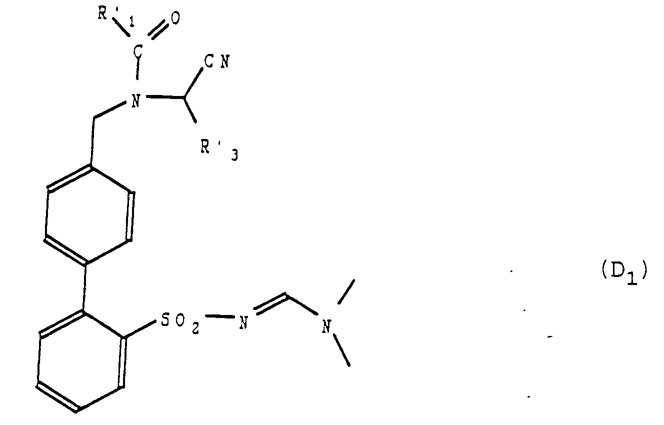 dans laquelle R'₁ et R'₃ ont les significations indiquées ci-dessus, que soit l'on soumet à une réaction d'addition sur le radical CN, à l'aide d'un réactif, tel que défini à la revendication 1, capable d'introduire le substituant R'₂ tel que défini ci-dessus, pour obtenir le produit de formule (E₁) : 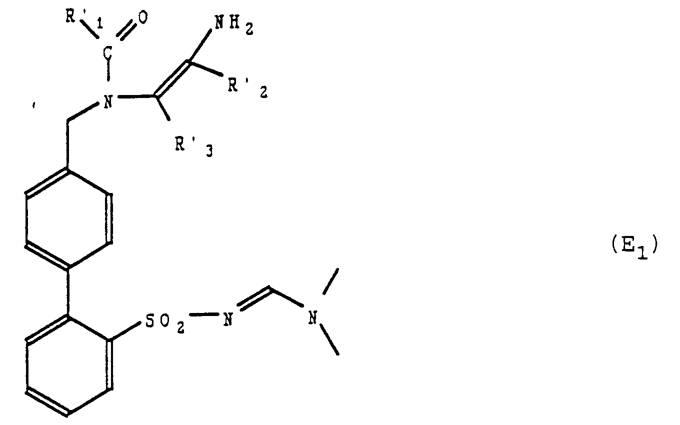 dans laquelle R'₁, R'₂ et R'₃ ont les significations indiquées ci-dessus, que l'on soumet le cas échéant à une réaction de substitution de l'atome d'oxygène par un atome de soufre pour obtenir le produit de formule (L₁) : 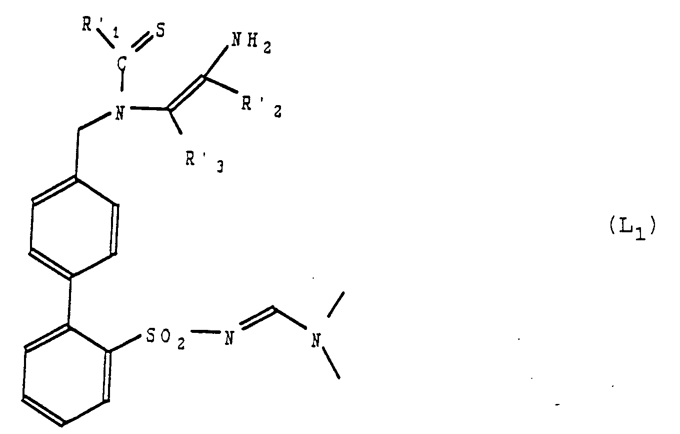 dans laquelle R'₁, R'₂ et R'₃ ont les significations indiquées ci-dessus, que, si nécessaire ou si désiré, l'on transforme en produit de formule (L₂) : 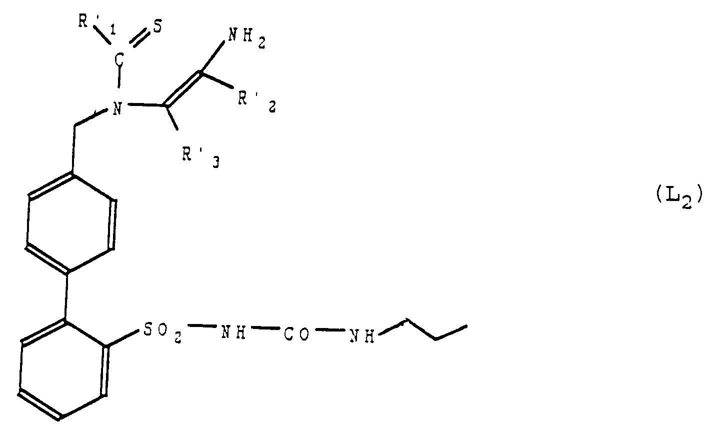 dans laquelle R'₁, R'₂ et R'₃ ont les significations indiquées ci-dessus, produit de formule (E₁), (L₁) ou (L₂) que l'on soumet à une réaction de cyclisation pour obtenir un produit de formule (I'₁) ou (I'₂) : 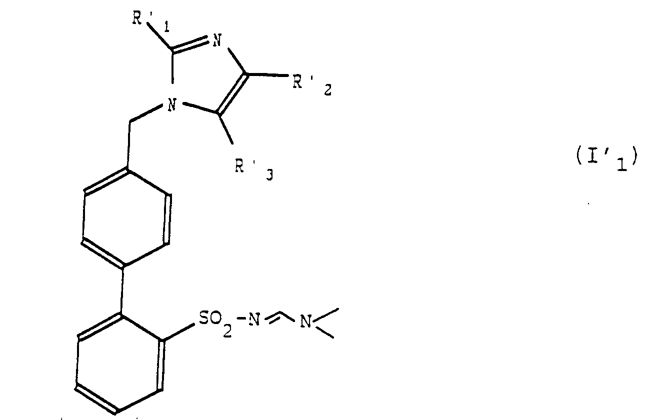 ou 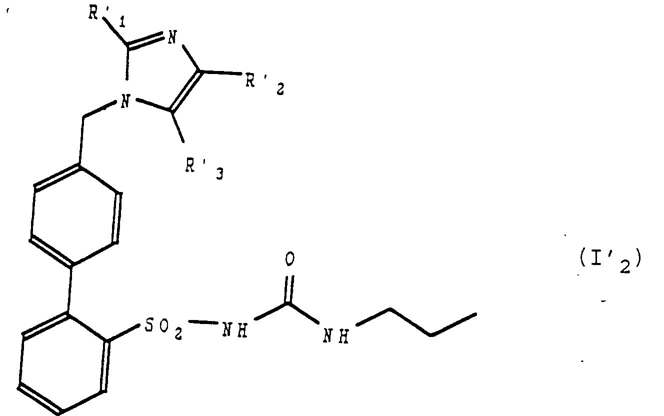 dans laquelle R'₁, R'₂ et R'₃ ont les significations indiquées ci-dessus, et le cas échéant transforme le produit de formule (I'₁) en produit de formule (I'₂) telle que définie ci-dessus,
soit l'on soumet le produit de formule (D₁) à une réaction d'addition sur le radical 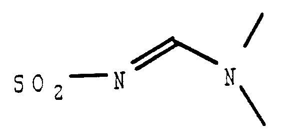 pour obtenir le produit de formule (D₂) : 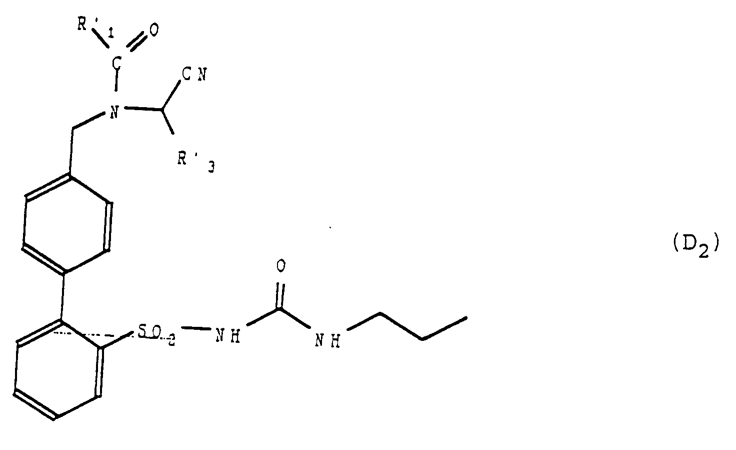 dans laquelle R'₁ et R'₃ ont les significations indiquées ci-dessus,
que l'on soumet à une réaction d'addition sur le radical CN comme indiqué ci-dessus, pour obtenir un produit de formule (E₂) : 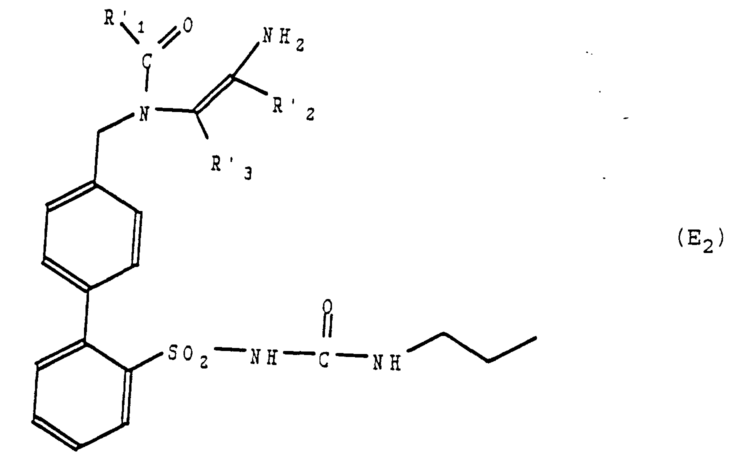 dans laquelle R'₁, R'₂ et R'₃ ont les significations indiquées ci-dessus, que l'on soumet à une réaction de cyclisation pour obtenir un produit de formule (I'₂) telle que définie ci-dessus.

7. Procédé tel que défini à la revendication 1, de préparation des produits de formule (I) répondant aux formules suivantes :
- l'acide 2-butyl 1-[[2'-carboxy (1,1'-biphényl)-4-yl] méthyl] 4-(phénylthio) lH-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy(1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-butyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) 1H-imidazole-5-carboxylique.

8. Procédé tel que défini à la revendication 1, de préparation des produits de formule (I) répondant aux formules suivantes :
- le 2-butyl 4-(méthylthio) 1-[[2'-((((propylamino) carbonyl) amino) sulfonyl) (1,1'-biphényl)-4-yl] méthyl]-1H-imidazole 5-carboxylate d'éthyle,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique,
- l'acide 2-butyl 4-(méthylthio) 1- [[2'-((((propylamino) carbonyl) amino) sulfonyl) (1,1'-biphényl)-4-yl] méthyl]-1H-imidazole 5-carboxylique, di sel de potassium.

9. Les composés de formule 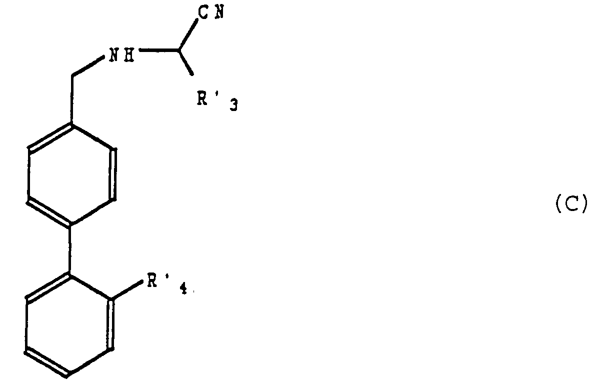 dans laquelle R'₃ et R'₄ ont les significations indiquées à la revendication 1.

10. Les composés de formule (D), (D₁) et (D₂) tels que définis ci-après : 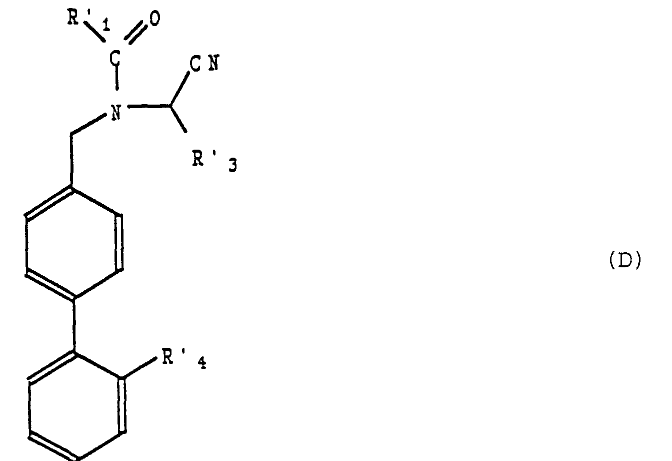 dans laquelle R'₁, R'₃ et R'₄ ont les significations indiquées à la revendication 1, 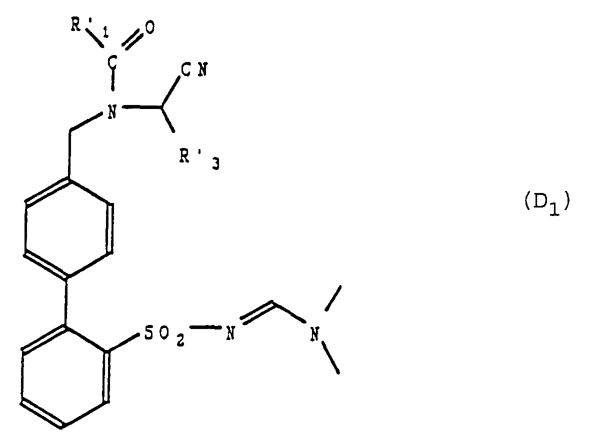 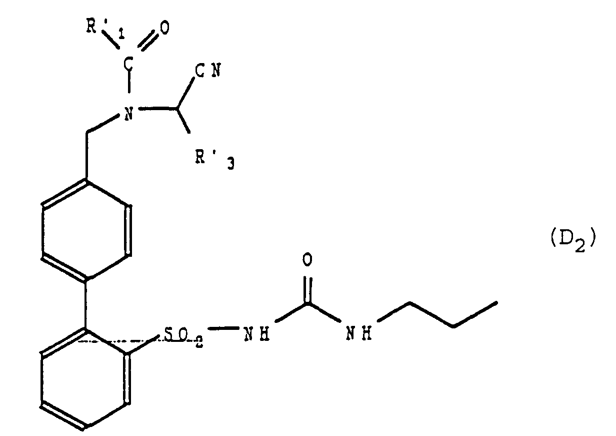 dans lesquelles R'₁ et R'₃ ont les significations indiquées à la revendication 1.

11. Les composés de formules (E), (E₁) et (E₂), tels que définis ci-après 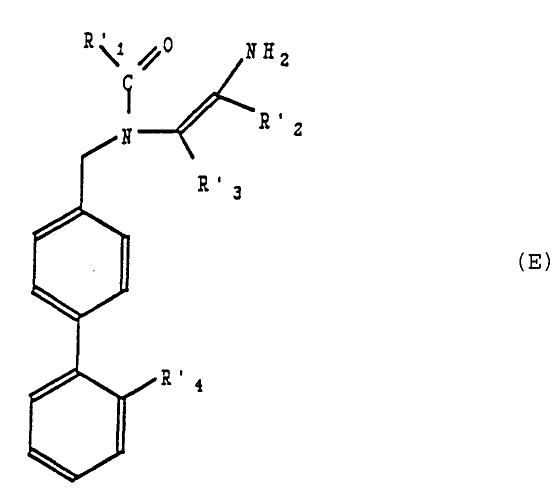 dans laquelle R'₁, R'₂, R'₃ et R'₄ ont les significations indiquées à la revendication 1, 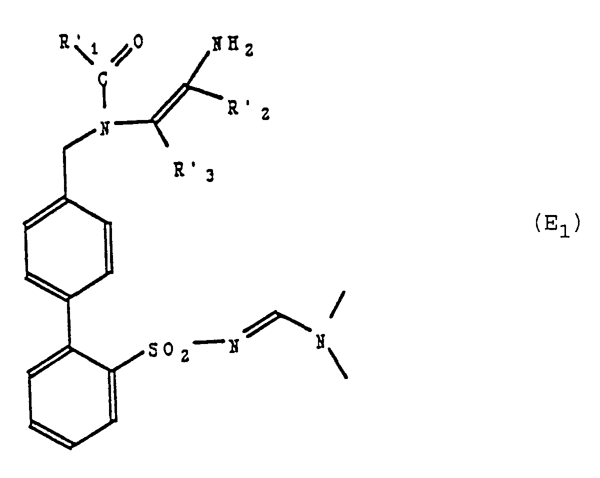 et 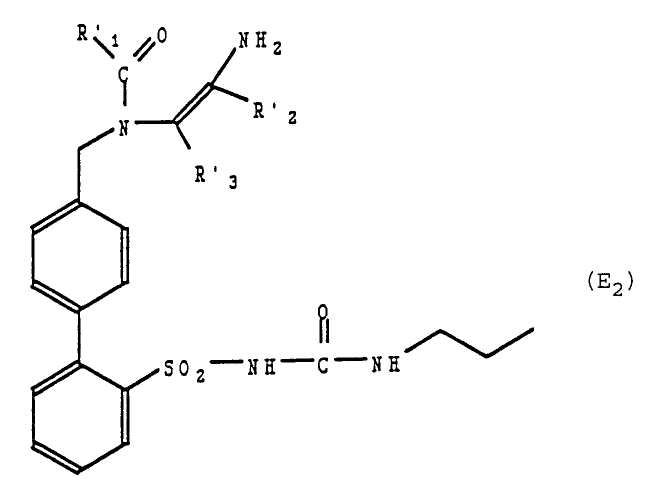 dans lesquelles R'₁, R'₂ et R'₃ ont les significations indiquées à la revendication 1.

12. Les composés de formules (L, (L₁) et (L₂), tels que définis ci-après : 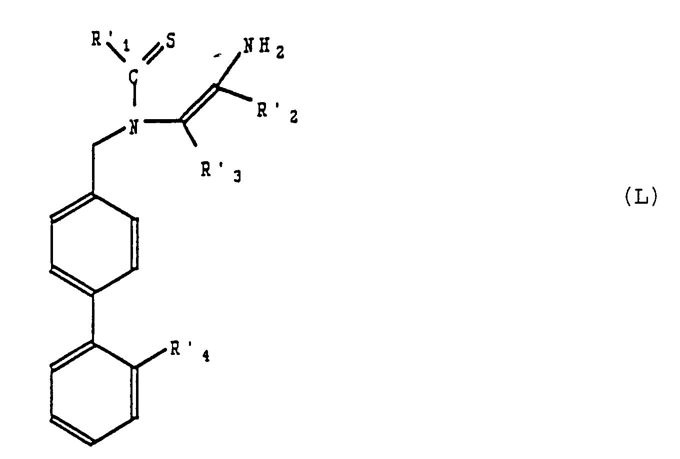 dans laquelle R'₁, R'₂, R'₃ et R'₄ ont les significations indiquées à la revendication 1, 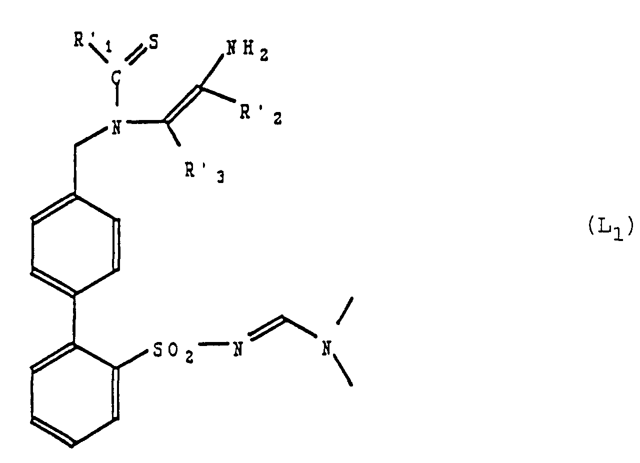 et 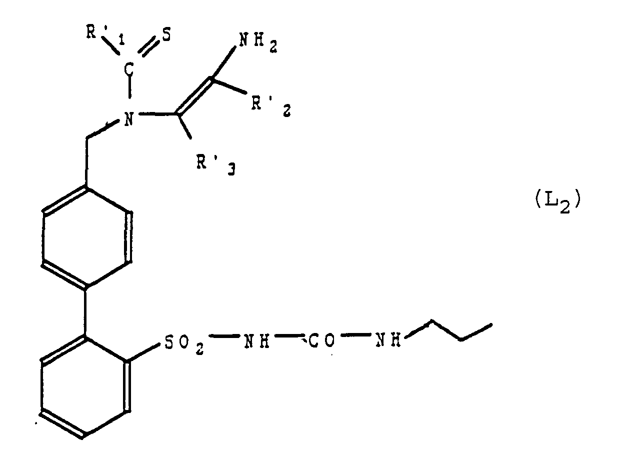 dans laquelle R'₁, R'₂ et R'₃ ont les significations indiquées à la revendication 1.

## Claims

1. Process for the preparation of products of formula (I) : 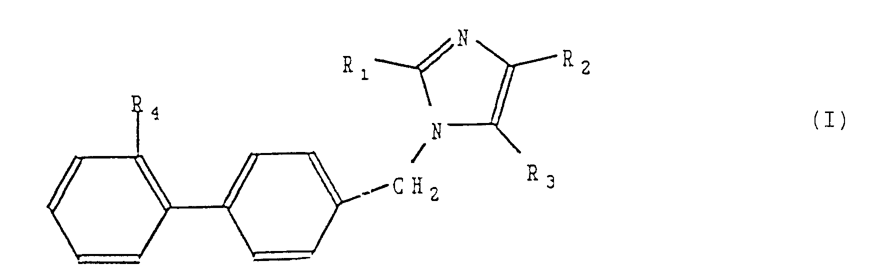 in which:
R₁ represents a linear or branched alkyl or alkenyl radical containing at most 4 carbon atoms,
R₂ and R₃, identical or different, are chosen from:
a) the hydrogen atom; the mercapto radical; the formyl; free, salified or esterified carboxy radicals; halogen atoms; the hydroxyl; cyano; nitro; acyl radical;
b) the alkyl, alkenyl, alkoxy, alkylthio radicals in which the sulphur atom is optionally mono- or dioxidized, these radicals being linear or branched and containing at most 6 carbon atoms, the phenyl, benzoyl, phenylthio radicals in which the sulphur atom is optionally mono- or dioxidized, all these radicals being optionally substituted by one or more radicals identical or different chosen from: halogen atoms,
hydroxyl, trifluoromethyl, cyano, nitro, formyl, alkyl and alkoxy radicals containing at most 4 carbon atoms, phenyl and free salified or esterified carboxy, ,
c) the 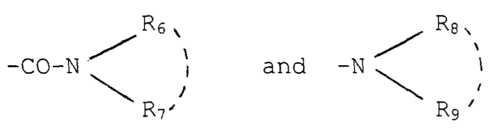 radicals in which:
either R₆, R₇, R₈ and R₉, identical or different, are chosen from the hydrogen atom, the amino acids, the alkyl radicals, these radicals containing at most 6 carbon atoms, phenyl, benzyl, phenethyl,
or on the one hand R₆ and R₇ and on the other hand R₈ and R₉ form respectively with the nitrogen atom to which they are linked a heterocyclic radical, these identical or different radicals being chosen from the imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl radicals, these radicals being optionally substituted by one or more identical or different radicals chosen from the halogen atoms, the hydroxyl, nitro, alkyl and alkoxy radicals, these radicals containing at most 6 carbon atoms and phenyl,
R₄ₐ represents the cyano, free, salified or esterified carboxy radical, the -(CH₂)ₚ-SO₂-X-R₁₀ radical in which p represents the values 0 and 1, X represents the -NH-, -NH-CO-, -NH-CO-O-, -N=CH-N-R₁₃, -NH-CO-NH- radicals or a single bond and R₁₀ and R₁₃ identical or different, represent a hydrogen atom, a linear or branched alkyl or alkenyl radical, containing at most 6 carbon atoms and optionally substituted, pyridyl, phenyl, benzyl, nitropyridyl, pyrimidyl, tetrazolyl, diazolyl, piperidinyl, alkylpiperidinyl, thiazolyl, alkylthiazolyl, tetrahydrofuranyl, methyltetrahydrofuranyl; the alkyl and alkenyl radicals being optionally substituted by one or more radicals chosen from the halogen atoms, the hydroxyl, nitro radical, the alkyl, alkenyl and alkoxy radicals containing at most 4 carbon atoms, the trifluoromethyl, cyano, amino, mono- and dialkylamino, free, salified or esterified carboxy, phenyl, tetrazolyl radical; said products of formula (I) being in all possible racemic, enantiomeric and diastereoisomeric isomer forms, as well as the addition salts with mineral and organic acids or with mineral and organic bases of said products of formula (I), characterized in that a compound of formula (A): 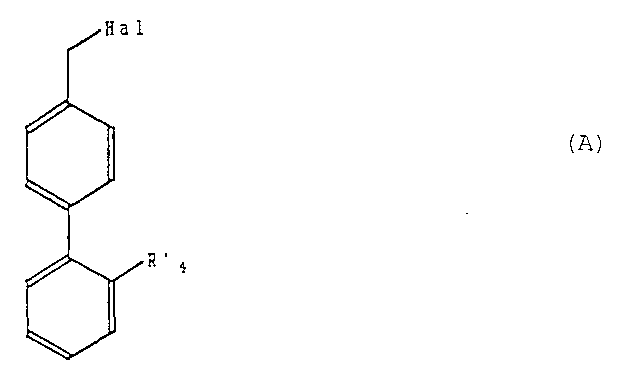 in which Hal represents a halogen atom and R'₄ has the meaning indicated above for R₄ in which the optional reactive functions are optionally protected, is reacted with an oxidizing agent, in order to obtain the compound of formula (B): 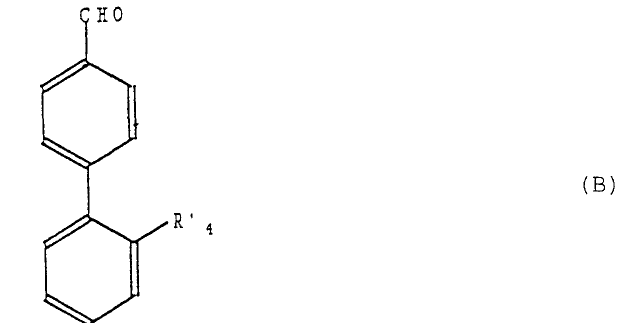 in which R'₄ has the meaning indicated above, which is reacted with a compound of formula (II) : 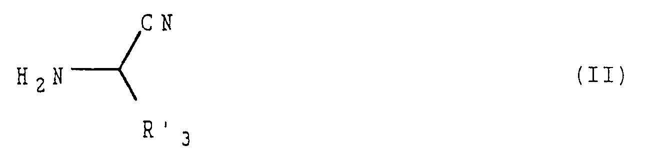 in which R'₃ has the meaning indicated above for R₃ in which the optional reactive functions are optionally protected, in order to obtain a product of formula (C) : 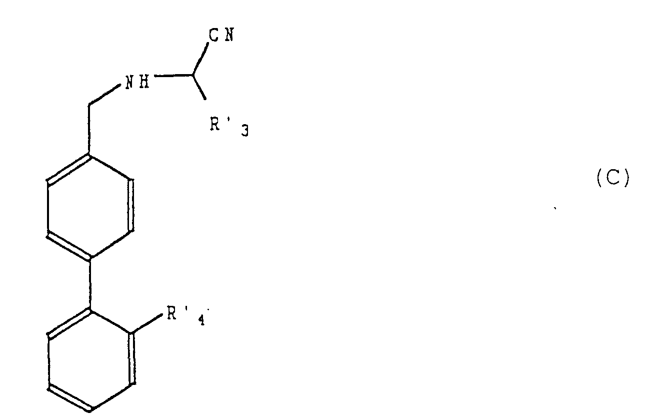 in which R'₃ and R'₄ have the meanings indicated above, which is reacted with a compound of formula (III):
R'₁-CO-Hal (III)
in which R'₁ has the meaning indicated above for R₁ in which the optional reactive functions are optionally protected and Hal represents a halogen atom, in order to obtain a product of formula (D): 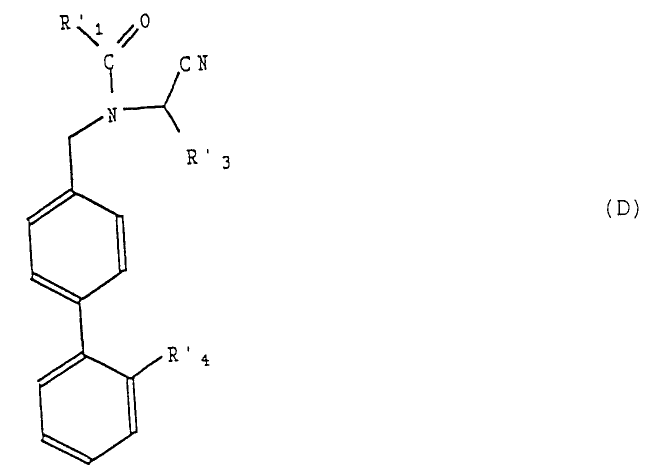 in which R'₁, R'₃ and R'₄ have the meanings indicated above, which is subjected to an addition reaction on the CN radical, using a reagent capable of introducing the substituent R'₂, R'₂ having the meaning indicated above for R₂ in which the optional reactive functions are optionally protected, the product of formula (D) being converted to a product of formula (E) either by amidification in a solvent such as for example an alcohol such as methanol or ethanol, or by reaction of compound R'₂-SH on the cyano function in a solvent such as for example toluene, tetrahydrofuran or dichloroethane,
or in the case where R'₂ represents a radical comprising a sulphur atom, by thioamidification carried out by the action of a compound of formula R₁₀-SH, R₁₀Sna or R₁₀SK in which R₁₀ represents the remainder of the R'₂ radical as defined above,
such a thioamidification being able to be carried out for example by bubbling the compound of formula R₁₀-SH, R₁₀Sna or R₁₀SK as defined above through a solvent such as for example an alcohol such as ethanol or methanol or also dichloroethane, dichloromethane, toluene, tetrahydrofuran in the presence of a base, for example triethylamine, in order to obtain a product of formula (E): 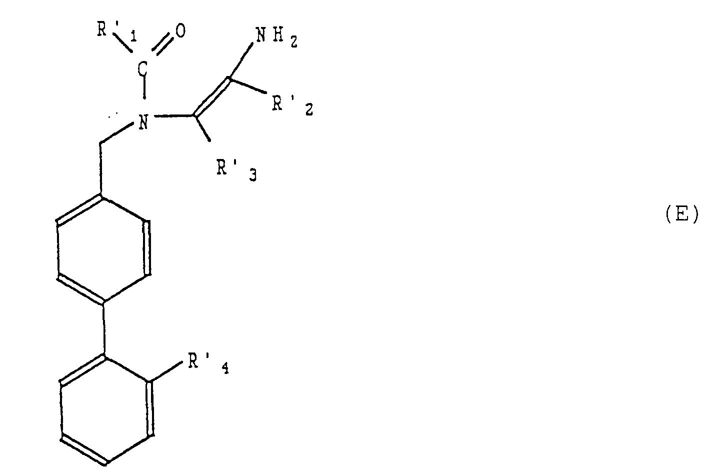 in which R'₁, R'₂, R'₃ and R'₄ have the meanings indicated above, which, if appropriate, is subjected to a substitution reaction of the oxygen atom by a sulphur atom in order to obtain the product of formula (L): 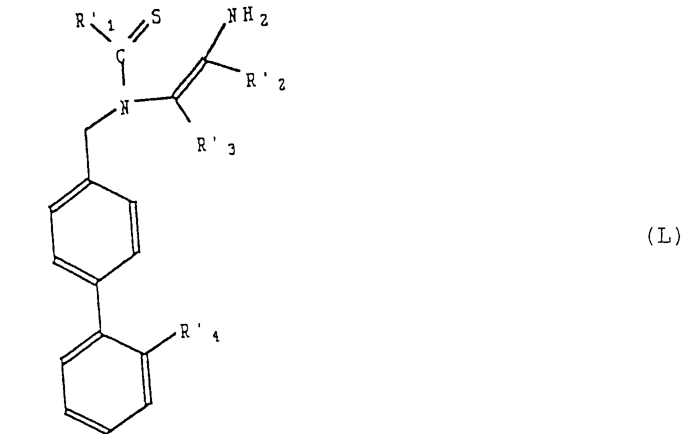 in which R'₁, R'₂, R'₃ and R'₄ have the meanings indicated above, which product of formula (E) or product of formula (L) is subjected to a cyclization reaction carried out either by an acid catalysis using for example amberlyst H⁺, tosylic acid or sulphuric acid, in a solvent such as for example toluene, ethyl acetate, dichloromethane, dichloroethane, or with the pentachloride in pyridine or dimethylaminopyridine, or also in dimethylsulphone, in order to obtain a product of formula (I') : 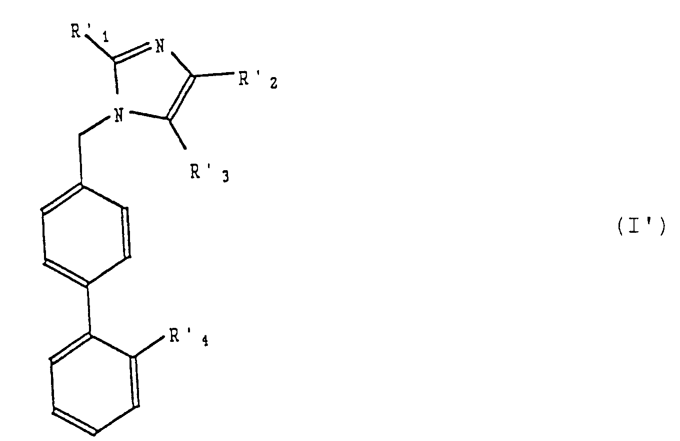 in which R'₁, R'₂, R'₃ and R'₄ have the meanings indicated above, it being understood that the products of formulae (D), (E) and (L) during the above process, and the products of formula (I') can be subjected, if desired and if necessary, to one or more of the following reactions, in any order:
a) an esterification reaction of the acid function,
b) a saponification reaction of the ester function,
c) a conversion reaction of the cyano function to an acid function,
d) a reduction reaction of the carboxy function to an alcohol function,
e) a conversion reaction of the alkoxy function to a hydroxyl function,
f) an oxidation reaction of the group comprising a sulphur atom to a corresponding sulphoxide or sulphone,
g) a conversion reaction of the alcohol or sulphone function to a corresponding aldehyde or acid function,
h) a conversion reaction of the nitrile radical to a tetrazole,
i) a conversion reaction of the formyl radical to a carbamoyl radical,
j) a conversion reaction of the carbamoyl radical to a nitrile radical,
k) a conversion reaction of the 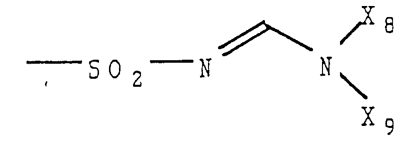 radical, to an 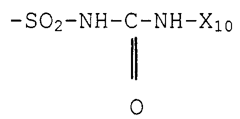 radical in which X₈, X₉ and X₁₀, identical or different, represent a hydrogen atom or an alkyl or alkenyl radical containing at most 4 optionally substituted carbon atoms,
which products of formula (I') can represent products of formula (I) or which can be subjected, if desired and if necessary, in order to obtain the products of formula (I), to one or more of the following reactions, in any order:
a) an elimination reaction of the protective groups which are carried by the protected reactive functions,
b) a salification reaction by a mineral or organic acid or by a base in order to obtain the corresponding salt,
c) a resolution reaction of the racemic forms to resolved products
said products of formula (I) thus obtained being in all possible racemic, enantiomeric and diastereoisomeric isomer forms.

2. Process according to claim 1 for the preparation of products of formula (I) corresponding to formula (I_{b}) : 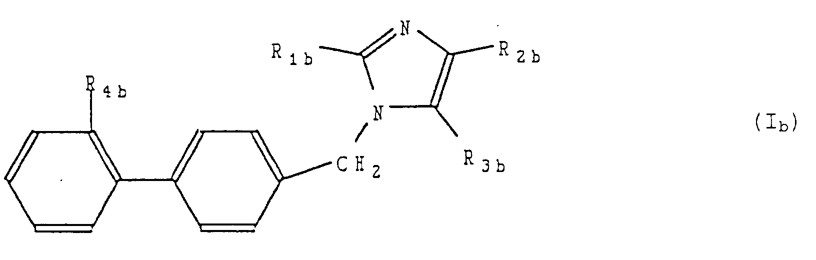 in which:
R_{1b} represents an alkyl radical containing at most 4 carbon atoms,
R_{3b} represents a hydrogen atom, a formyl radical, an optionally substituted acyloxy, alkyl or alkoxy radical, or a carboxy radical free, salified or esterified by an alkyl radical,
R_{2b} represents a phenylthio, phenylsulphonyl, phenylsulphinyl, alkylthio, alkylsulphonyl or alkylsulphinyl radical optionally substituted, such that in all these radicals which can be represented by R_{2b} and R_{3b}, the alkyl, alkoxy radicals containing at most 6 carbon atoms, and the phenyl radicals are optionally substituted by one or more radicals chosen from halogen atoms and the hydroxyl, trifluoromethyl, acyloxy, free, salified or esterified carboxy, phenyl, pyridyl, tetrazolyl radicals, alkyl and alkoxy radicals containing at most 4 carbon atoms and themselves optionally substituted by an alkoxy radical containing at most 4 carbon atoms,
R_{4b} represents the cyano, free, salified or esterified carboxy radical, the -SO₂-X_{b}-R_{10b} radical in which X_{b} represents the-NH-, -NH-CO-, -NH-CO-O-, -N=CH-N-R_{13b}, -NH-CO-NH- radicals or a single bond and R_{10b} and R_{13b}, identical or different, represent a hydrogen atom, a methyl, ethyl, propyl, vinyl, allyl, pyridyl, phenyl, benzyl, nitropyridyl, pyrimidyl, tetrazolyl, diazolyl, piperidinyl, alkylpiperidinyl, thiazolyl, alkylthiazolyl, tetrahydrofuranyl, methyltetrahydrofuranyl radical,
said products of formula (I_{b}) being in all possible racemic, enantiomeric and diastereoisomeric isomer forms, as well as the addition salts with mineral and organic acids or with mineral and organic bases of said products of formula (I_{b}), characterized in that the products of formulae (A), (II), (III) and a reagent capable of introducing the R'₂ radical as defined in claim 1 are used for their preparation as defined in claim 1,, in which R'₁, R'₂, R'₃ and R'₄ have the values indicated above respectively for R_{1b}, R_{2b}, R_{3b} and R_{4b} in which the reactive functions are optionally protected.

3. Process according to claim 1 for the preparation of products of formula (I) corresponding to formula (I_{d}) : 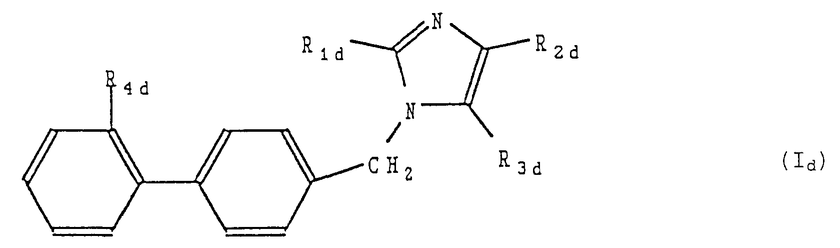 in which:
R_{1d} represents an alkyl radical containing at most 4 carbon atoms,
R_{3d} represents a
- carboxy radical free, salified or esterified by a linear or branched alkyl radical containing at most 4 carbon atoms, formyl, acyloxy, or alkyl radical containing at most 4 carbon atoms optionally substituted by a hydroxyl radical,
R_{2d} represents a phenylthio, phenylsulphonyl, phenylsulphinyl, alkylthio, alkylsulphonyl or alkylsulphinyl radical in which the alkyl radical contains at most 4 carbon atoms, and R_{4d} represents the -SO₂-NH₂, -SO₂-NH-CO-O-R_{10d}, -SO₂-N=CH-NR_{13d}, or -SO₂-NH-CO-NH-R_{10d} radical in which R_{10d} and R_{13d}, identical or different, are chosen from the hydrogen atom, the methyl, ethyl, n-propyl and propenyl radical, said products of formula (I_{d}) being in all possible racemic, enantiomeric and diastereoisomeric isomer forms, as well as the addition salts with mineral and organic acids or with mineral and organic bases, of said products of formula (I_{d}), characterized in that the products of formulae (A), (II), (III) and a reagent capable of introducing the R'₂ radical such as defined in claim 1 are used for their preparation as defined in claim 1,, in which R'₁, R'₂, R'₃ and R'₄ have the values indicated above respectively for R_{1d}, R_{2d}, R_{3d} and R_{4d} in which the reactive functions are optionally protected.

4. Process according to claim 1, characterized in that a product of formula (A) is used in which R'₄ represents the -SO₂-NH₂, -SO₂-NH-CO-O-R_{10d}, -SO₂-N=CH-NR_{13d}, or -SO₂-NH-CO-NH-R_{10d} radical in which R_{10d} and R_{13d}, identical or different, are chosen from the hydrogen atom, the methyl, ethyl, n-propyl and propenyl radical, in which the reactive functions are optionally protected, a product of formula (II) in which R'₃ represents the carboxy radical free, salified or esterified by a linear or branched alkyl radical containing at most 4 carbon atoms, formyl, acyloxy, or alkyl containing at most 4 carbon atoms optionally substituted by a hydroxyl radical, a product of formula (III) in which R'₁ represents an alkyl radical containing at most 4 carbon atoms, and a reagent capable of introducing the substituent R'₂ as defined in claim 1, in which R'₂ represents the phenylthio, phenylsulphonyl, phenylsulphinyl, alkylthio, alkylsulphonyl or alkylsulphinyl radical,
in which the alkyl radical contains at most 4 carbon atoms, and the reactive functions are optionally protected.

5. Process according to claim 1, characterized in that a product of formula (A) is used in which R'₄ represents the 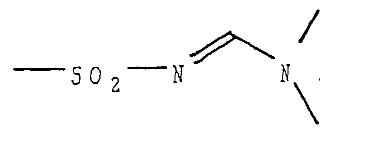 radical, a product of formula (II) in which R'₃ represents an alkoxy or a free, salified or esterified carboxy radical, a product of formula (III) in which R'₁ represents an alkyl radical containing at most 4 carbon atoms and a reagent capable of introducing the substituent R'₂ as defined in claim 1, in which R'₂ represents an alkylthio or phenylthio radical optionally oxidized in the form of sulphoxide or sulphone, these alkoxy, alkylthio and phenylthio radicals being optionally substituted by one or more radicals chosen from the halogen atoms, the alkyl or alkoxy radicals containing at most 4 carbon atoms, trifluoromethyl, amino, mono or dialkylamino, cyano, phenyl, hydroxyl, free, salified or esterified carboxy, acyl and acyloxy radicals.

6. Process according to claim 1, characterized in that a compound of formula (A₁) : 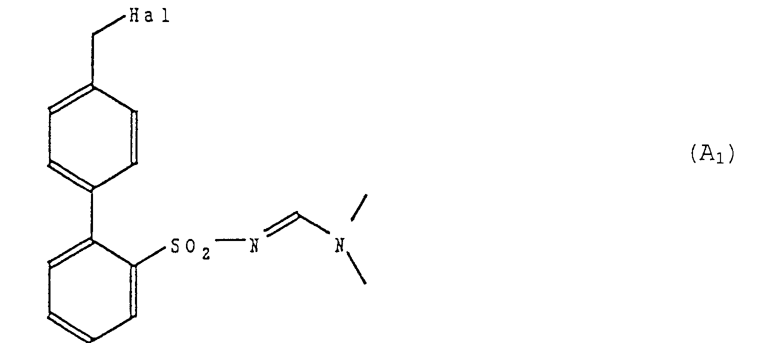 in which Hal represents a halogen atom is reacted with an oxidizing agent in order to obtain the compound of formula (B₁) : 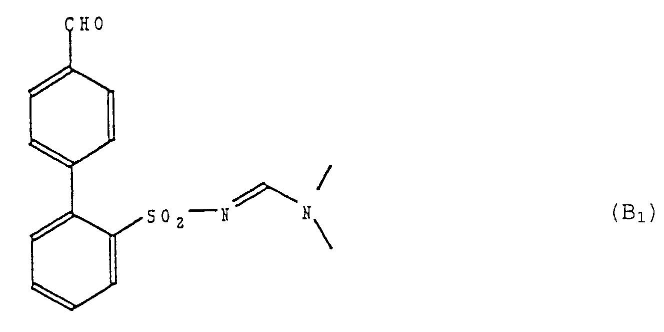 which is reacted with a compound of formula (II): 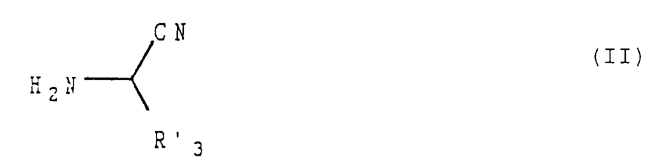 in which R'₃ has the meaning indicated above, in order to obtain a product of formula (C₁) : 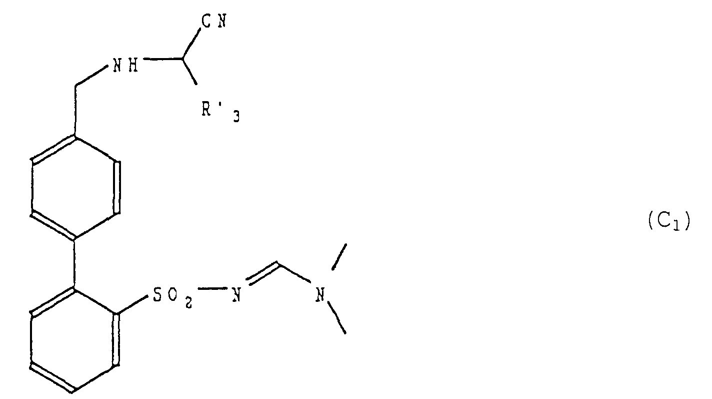 in which R'₃ has the meaning indicated above, which is reacted with a compound of formula (III):
R'₁-CO-Hal (III)
in which R'₁ and Hal have the meaning indicated above, in order to obtain a product of formula (D₁) : 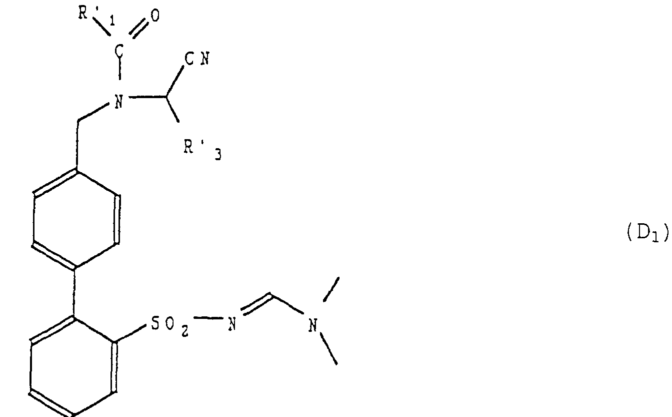 in which R'₁ and R'₃ have the meanings indicated above, which either is subjected to an addition reaction on the CN radical, using a reagent, as defined in claim 1, capable of introducing the substituent R'₂ as defined above, in order to obtain the product of formula (E₁) : 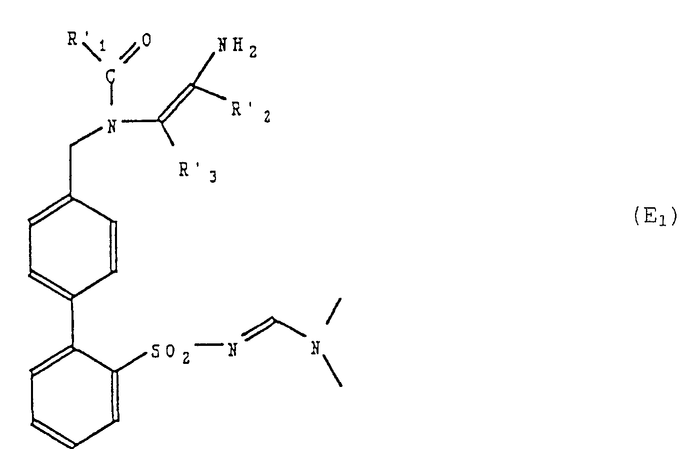 in which R'₁, R'₂ and R'₃ have the meanings indicated above, which is subjected, if appropriate, to a substitution reaction of the oxygen atom by a sulphur atom in order to obtain the product of formula (L₁) : 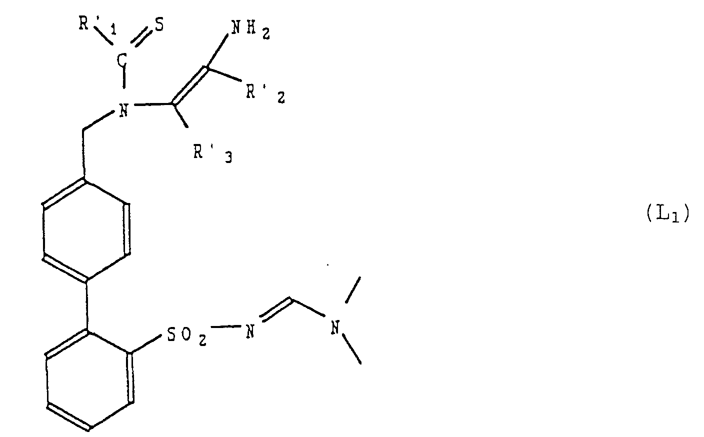 in which R'₁, R'₂ and R'₃ have the meanings indicated above, which, if necessary or if desired, is converted to a product of formula (L₂) : 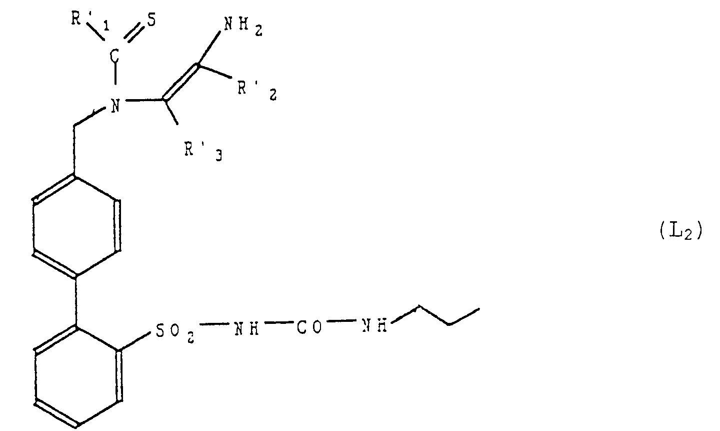 in which R'₁, R'₂ and R'₃ have the meanings indicated above, which product of formula (E₁), (L₁) or (L₂) is subjected to a cyclization reaction in order to obtain a product of formula (I'₁) or (I'₂) : 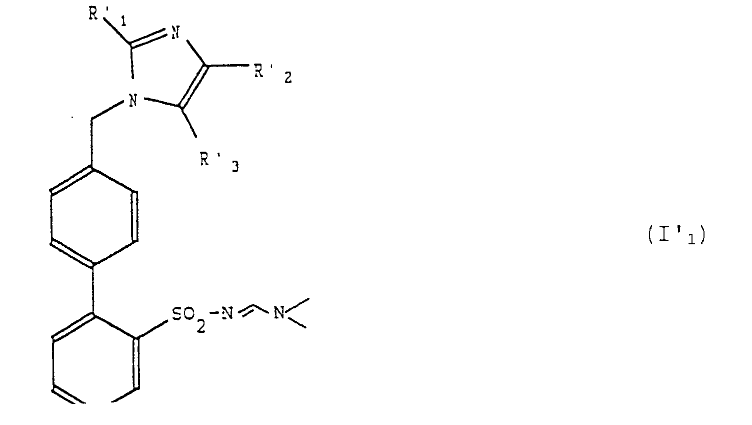 or 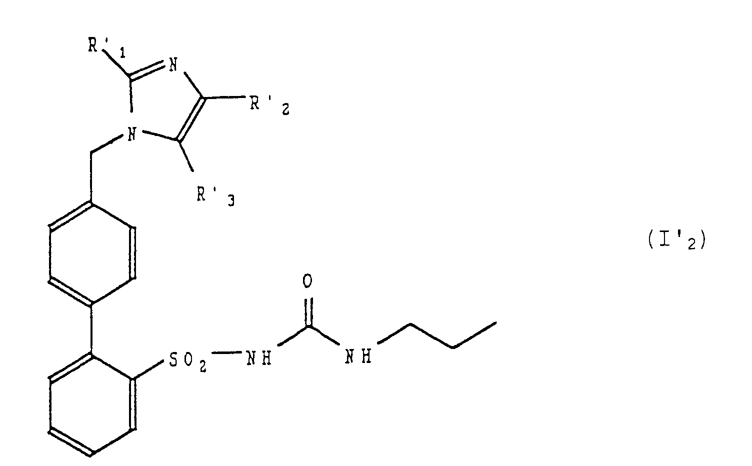 in which R'₁, R'₂ and R'₃ have the meanings indicated above, and, if appropriate, the product of formula (I'₁) is converted to a product of formula (I'₂) as defined above,
or the product of formula (D₁) is subjected to an addition reaction on the 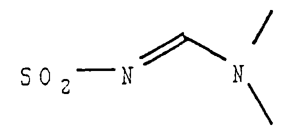 radical in order to obtain the product of formula (D₂) : 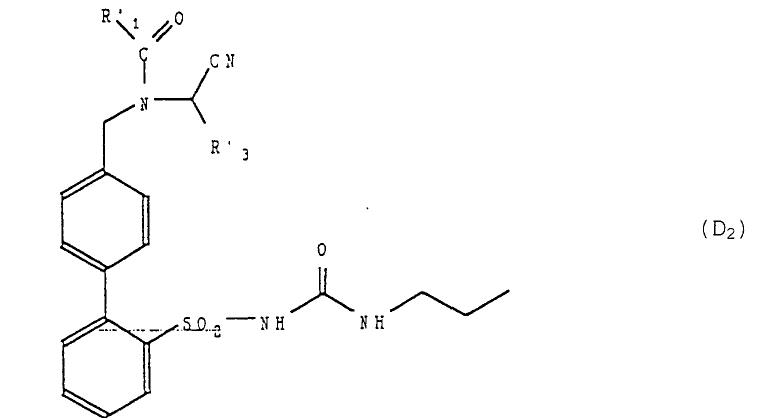 in which R'₁ and R'₃ have the meanings indicated above, which is subjected to an addition reaction on the CN radical as indicated above, in order to obtain a product of formula (E₂) 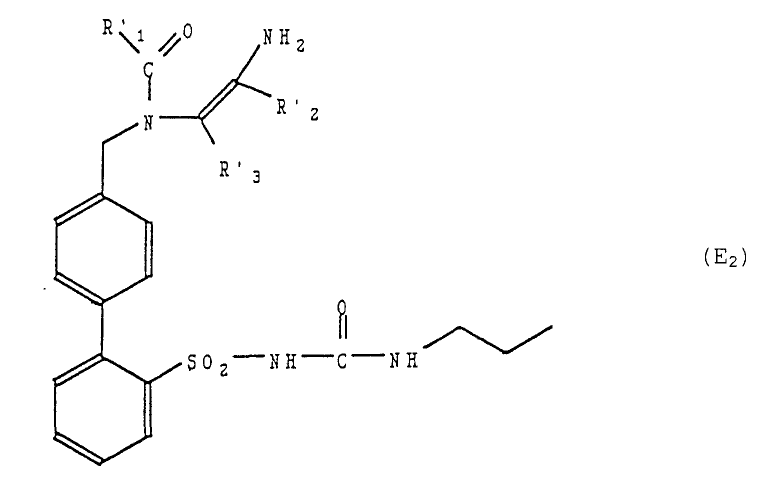 in which R'₁, R'₂ and R'₃ have the meanings indicated above, which is subjected to a cyclization reaction in order to obtain a product of formula (I'₂) as defined above.

7. Process such as defined in claim 1, for the preparation of the products of formula (I) corresponding to the following formulae:
- 2-butyl 1-[[2'-carboxy (1,1'-biphenyl)-4-yl] methyl] 4-(phenylthio) lH-imidazole-5-carboxylic acid
- 2-butyl 1-[[2'-carboxy (1,1'-biphenyl)-4-yl] methyl] 4-(methylthio) lH-imidazole-5-carboxylic acid
- 4'-[[2-butyl 4-(ethylthio) 5-(hydroxymethyl) 1H-imidazol-1-yl] methyl] (1,1'-biphenyl)-2-carboxylic acid
- 2-butyl 1-[[2'-carboxy (1,1'-biphenyl)-4-yl] methyl] 4-(ethylsulphonyl) 1H-imidazole-5-carboxylic acid
- 2-butyl 1-[[2'-carboxy (1,1'-biphenyl)-4-yl] methyl] 4-(ethylsulphinyl) 1H-imidazole-5-carboxylic acid
- 2-butyl 1-[[2'-carboxy (1,1'-biphenyl)-4-yl] methyl] 4-(ethylthio) lH-imidazole-5-carboxylic acid
- 2-butyl 1-[[2'-carboxy (1,1'-biphenyl)-4-yl] methyl] 4-(phenylsulphonyl) lH-imidazole-5-carboxylic acid
- 2-butyl 1-[[2'-carboxy (1,1'-biphenyl)-4-yl] methyl] 4-(phenylsulphinyl) lH-imidazole-5-carboxylic acid
- 2-butyl 1-[[2'-tetrazolyl (1,1'-biphenyl)-4-yl] methyl] 4-(methylthio) lH-imidazole-5-carboxylic acid.

8. Process as defined in claim 1, for the preparation of the products of formula (I) corresponding to the following formulae:
- ethyl 2-butyl 4-(methylthio) 1-[[2'-((((propylamino) carbonyl) amino) sulphonyl) (1,1'-biphenyl)-4-yl] methyl]-1H-imidazole 5-carboxylate,
- 2-butyl 4-(methylthio) 1-[[2'-((((propylamino) carbonyl) amino) sulphonyl) (1,1'-biphenyl)-4-yl] methyl]-1H-imidazole 5-carboxylic acid,
- 2-butyl 4-(methylthio) 1-[[2'-((((propylamino) carbonyl) amino) sulphonyl) (1,1'-biphenyl)-4-yl] methyl]-1H-imidazole 5-carboxylic acid, dipotassium salt.

9. The compounds of formula 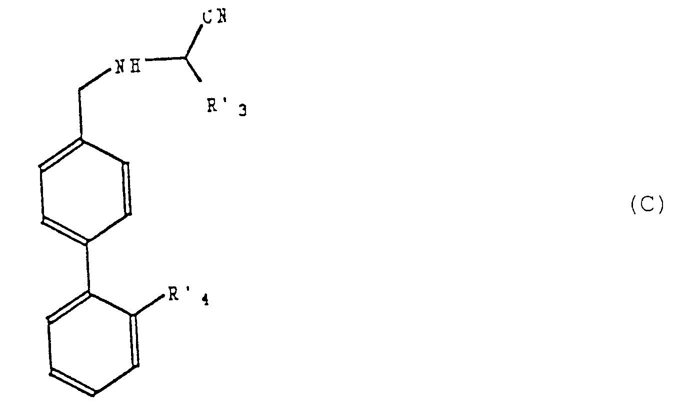 in which R'₃ and R'₄ have the meanings indicated in claim 1.

10. The compounds of formula (D), (D₁) and (D₂) as defined hereafter: 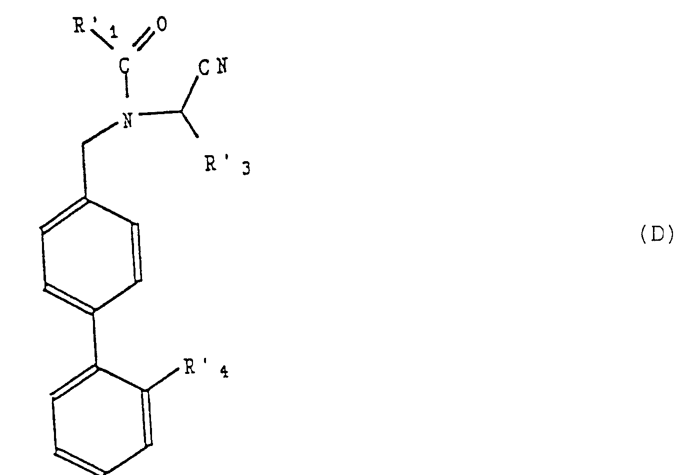 in which R'₁, R'₃ and R'₄ have the meanings indicated in claim 1, 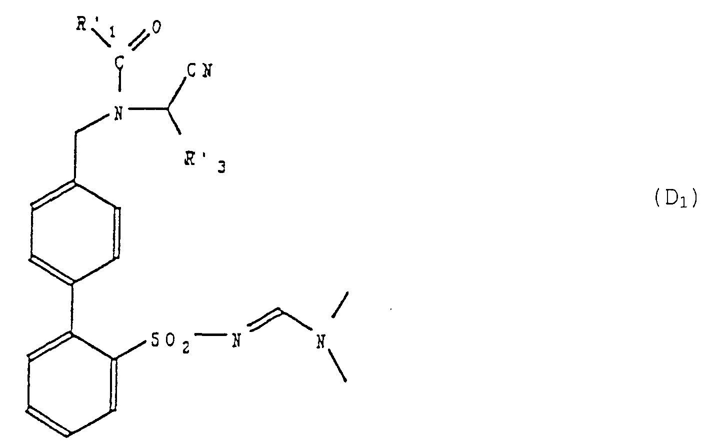 and 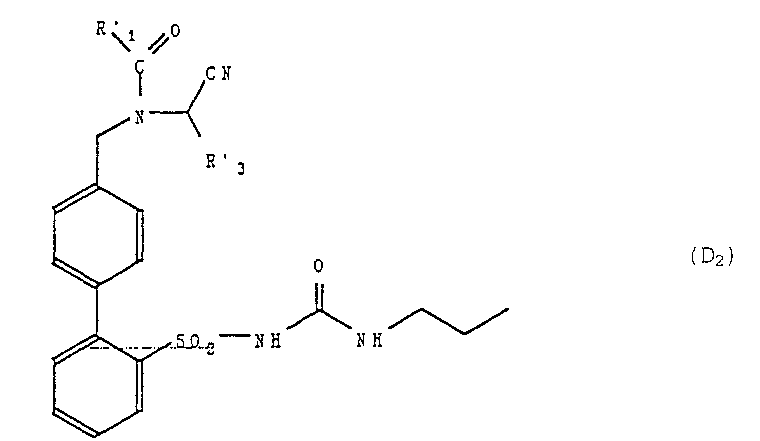 in which R'₁ and R'₃ have the meanings indicated in claim 1.

11. The compounds of formulae (E), (E₁) and (E₂), as defined hereafter 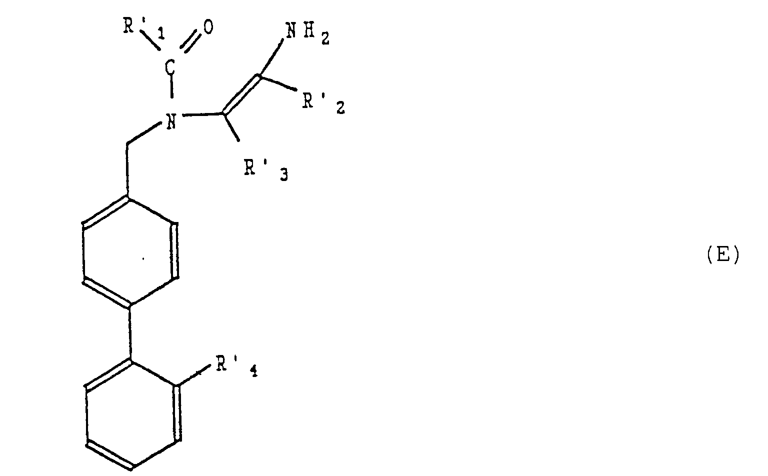 in which R'₁, R'₂, R'₃ and R'₄ have the meanings indicated in claim 1, 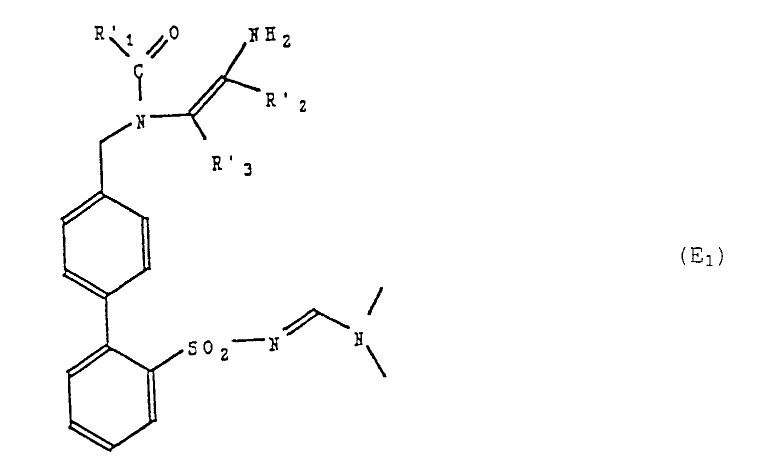 and 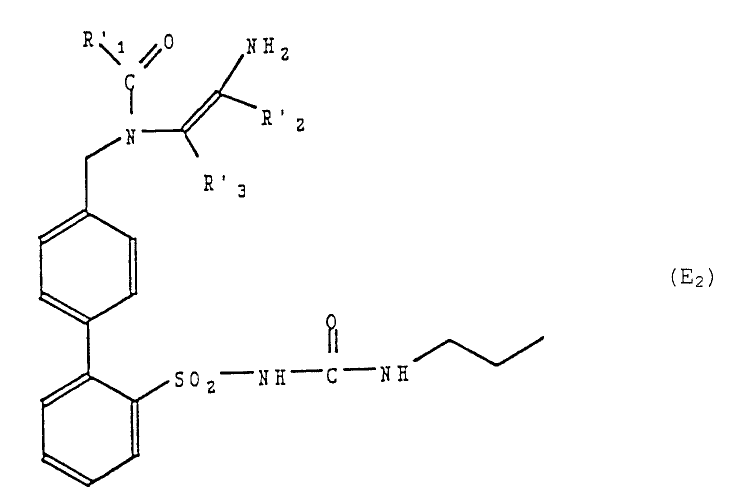 in which R'₁, R'₂ and R'₃ have the meanings indicated in claim 1.

12. The compounds of formulae (L, (L₁) and (L₂), as defined hereafter: 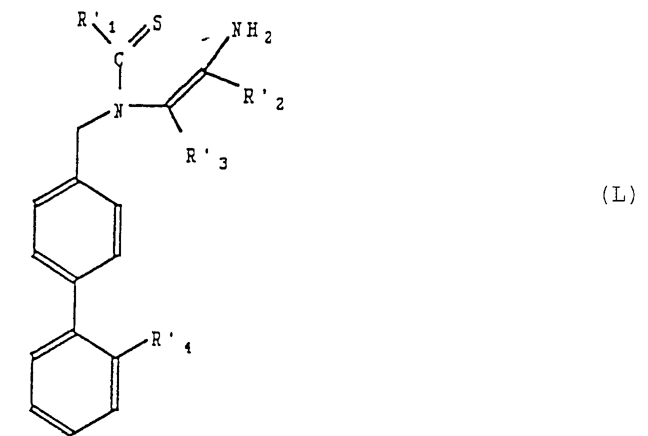 in which R'₁, R'₂, R'₃ and R'₄ have the meanings indicated in claim 1, 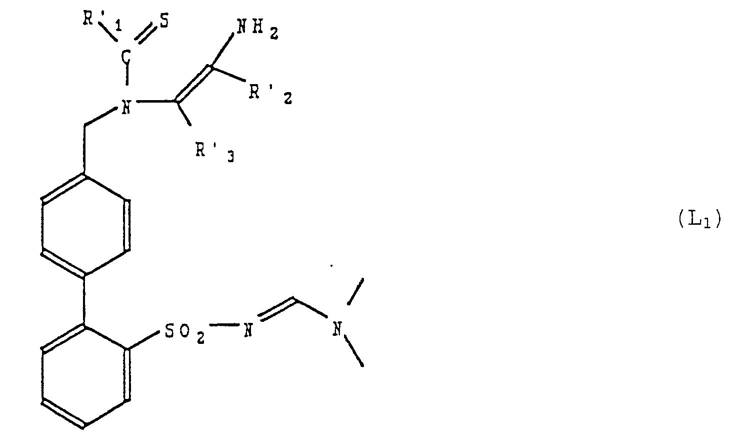 and 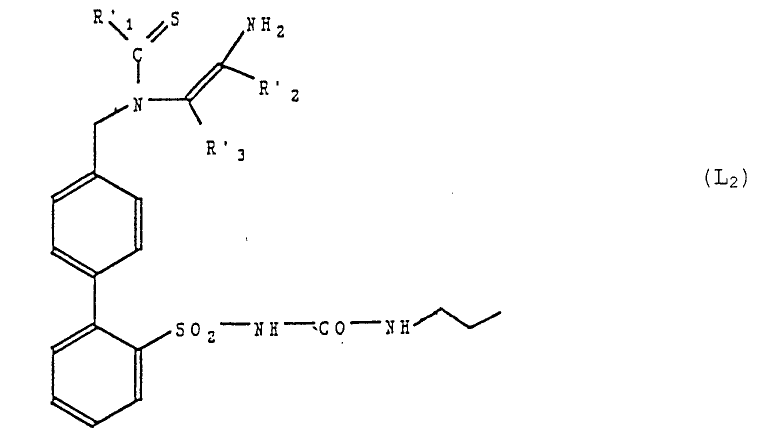 in which R'₁, R'₂ and R'₃ have the meanings indicated in claim 1.

## Patentansprüche

1. Verfahren zur Herstellung von Produkten der Formel (I) 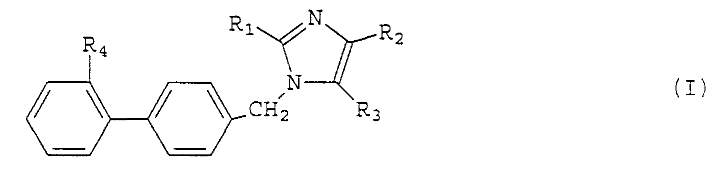 in der
R₁ einen geraden oder verzweigten Rest Alkyl oder Alkenyl mit höchstens 4 Kohlenstoffatomen darstellt,
R₂ und R₃, gleich oder verschieden, ausgewählt werden unter:
a) dem Wasserstoffatom; dem Rest Mercapto; den Resten Formyl; Carboxy, frei, in Salz überführt oder verestert; den Halogenatomen; dem Rest Hydroxyl; Cyano; Nitro; Acyl;
b) den Resten Alkyl, Alkenyl, Alkoxy, Alkylthio, worin das Schwefelatom gegebenenfalls mono- oder dioxidiert ist, wobei diese Reste gerade oder verzweigt sind und höchstens 6 Kohlenstoffatome umfassen, den Resten Phenyl, Benzoyl, Phenylthio, worin das Schwefelatom gegebenenfalls mono- oder dioxidiert ist, wobei alle diese Reste gegebenenfalls durch einen oder mehrere, gleiche oder verschiedene Reste substituiert sind, ausgewählt unter:
den Halogenatomen, den Resten Hydroxyl, Trifluormethyl, Cyano, Nitro, Formyl, Alkyl und Alkoxy mit höchstens 4 Kohlenstoffatomen, Phenyl und Carboxy, frei, in Salz überführt oder verestert;
c) den Resten 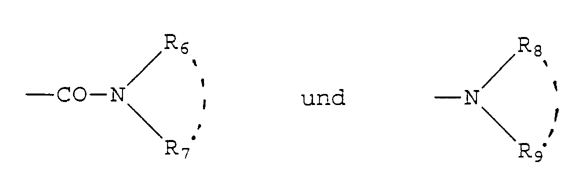 worin
entweder R₆, R₇, R₈ und R₉, gleich oder verschieden, ausgewählt werden unter: dem Wasserstoffatom, den Aminosäuren, den Resten Alkyl, wobei diese Reste höchstens 6 Kohlenstoffatome umfassen, Phenyl, Benzyl, Phenethyl,
oder einerseits R₆ und R₇ und andererseits R₈ und R₉ jeweils mit dem Stickstoffatom, an das sie gebunden sind, einen heterocyclischen Rest bilden, wobei diese gleichen oder verschiedenen Reste ausgewählt werden unter-den Resten Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin, Indolyl, wobei diese Reste gegebenenfalls substituiert sind durch einen oder mehrere, gleiche oder verschiedene Reste, ausgewählt unter den Halogenatomen, den Resten Hydroxyl, Nitro, Alkyl und Alkoxy, wobei diese Reste höchstens 6 Kohlenstoffatome umfassen, und Phenyl,
R₄ darstellt: den Rest Cyano, Carboxy, frei, in Salz überführt oder verestert, den Rest-(CH₂)ₚ-SO₂-X-R₁₀, worin p die Werte 0 und 1 besitzt, X die Reste -NH-, -NH-CO-, -NH-CO-O-, -N=CH-N-R₁₃, -NH-CO-NH- oder eine einfache Bindung bedeutet und R₁₀ und R₁₃, gleich oder verschieden, ein Wasserstoffatom, einen geraden oder verzweigten Rest Alkyl oder Alkenyl mit höchstens 6 Kohlenstoffatomen und gegebenenfalls substituiert, Pyridyl, Phenyl, Benzyl, Nitropyridyl, Pyrimidyl, Tetrazolyl, Diazolyl, Piperidinyl, Alkylpiperidinyl, Thiazolyl, Alkylthiazolyl, Tetrahydrofuranyl, Methyltetrahydrofuranyl bedeuten,
wobei die Reste Alkyl und Alkenyl gegebenenfalls substituiert sind durch einen oder mehrere Reste, ausgewählt unter den Halogenatomen, dem Rest Hydroxyl, Nitro, den Resten Alkyl, Alkenyl oder Alkoxy mit höchstens 4 Kohlenstoffatomen, dem Rest Trifluormethyl, Cyano, Amino, Mono- und Dialkylamino, Carboxy, frei, in Salz überführt oder verestert, Phenyl, Tetrazolyl;
wobei die genannten Produkte der Formel (I) in allen möglichen isomeren racemischen, enantiomeren und diastereoisomeren Formen vorliegen können, sowie die Additionssalze der genannten Produkte der Formel (I) mit Mineralsäuren und organischen Säuren oder mit Mineralbasen und organischen Basen, dadurch gekennzeichnet, daß man eine Verbindung der Formel (A) 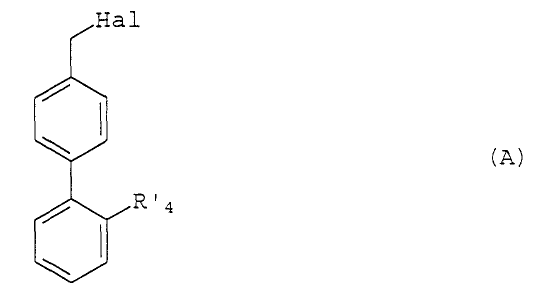 in der Hal ein Halogenatom ist und R'₄ die oben bei R₄ angegebene Bedeutung besitzt, worin die eventuellen reaktiven Funktionen gegebenenfalls geschützt sind, mit einem Oxidationsmittel zur Reaktion bringt, um die Verbindung der Formel (B) 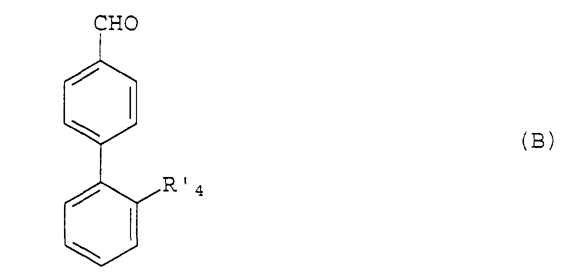 zu erhalten, in der R'₄ die oben angegebene Bedeutung besitzt, die man dann mit einer Verbindung der Formel (II) 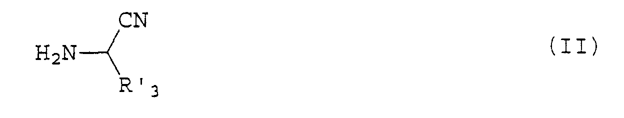 zur Reaktion bringt, in der R'₃ die oben bei R₃ angegebene Bedeutung besitzt, worin die eventuellen reaktiven Funktionen gegebenenfalls geschützt sind, um ein Produkt der Formel (C) 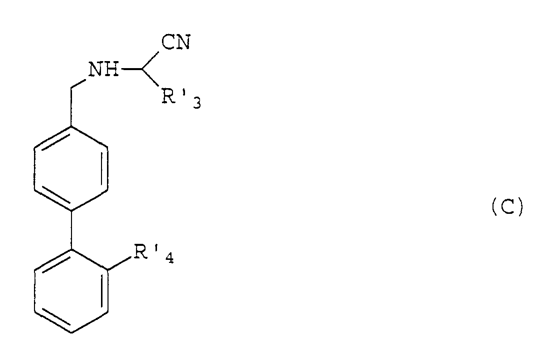 zu erhalten, in der R'₃ und R'₄ die oben angegebenen Bedeutungen besitzen, das man dann mit einer Verbindung der Formel (III)
R'₁-CO-Hal (III)
zur Reaktion bringt, in der R'₁ die oben bei R₁ angegebene Bedeutung besitzt, worin die eventuellen reaktiven Funktionen gegebenenfalls geschützt sind und Hal ein Halogenatom ist, um ein Produkt der Formel (D) 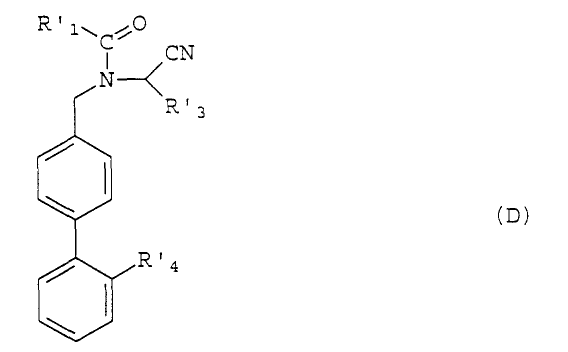 zu erhalten, in der R'₁, R'₃ und R'₄ die oben angegebenen Bedeutungen besitzen, das man dann mit Hilfe eines Reaktanden, der geeignet ist, den Substituenten R'₂ einzubringen, einer Additionsreaktion an dem Rest CN unterzieht, wobei R'₂ die oben bei R₂ angegebene Bedeutung besitzt, worin die eventuellen reaktiven Funktionen gegebenenfalls geschützt sind,
wobei das Produkt der Formel (D) in das Produkt der Formel (E) überführt wird, entweder durch Amidierung in einem Lösungsmittel, wie beispielsweise einem Alkohol wie Methanol oder Ethanol, oder durch Reaktion der Verbindung R'₂-SH mit der Funktion Cyano in einem Lösungsmittel wie beispielsweise Toluol, Tetrahydrofuran oder Dichlorethan,
oder in dem Fall, wo R'₂ einen Rest darstellt, der ein Schwefelatom umfaßt, durch Thioamidierung, durchgeführt mittels Einwirkung einer Verbindung der Formel R₁₀-SH, R₁₀Sna oder R₁₀SK, worin R₁₀ den Rest des wie oben definierten Restes R'₂ darstellt,
wobei eine derartige Thioamidierung beispielsweise durch Einleiten der wie oben definierten Verbindung der Formel R₁₀-SH, R₁₀Sna oder R₁₀SK in ein Lösungsmittel realisiert werden kann, wie beispielsweise in einen Alkohol wie Methanol oder Ethanol oder auch Dichlorethan, Dichlormethan, Toluol, Tetrahydrofuran sowie in Anwesenheit einer Base, beispielsweise Triethylamin, um ein Produkt der Formel (E) 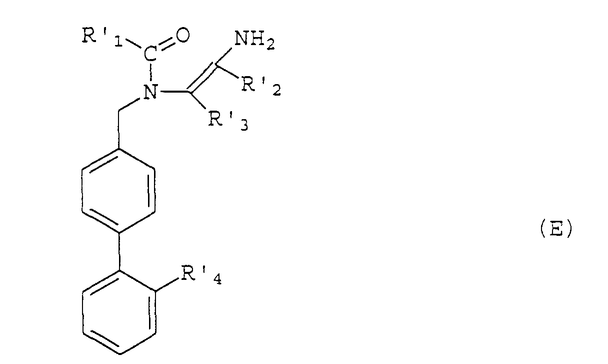 zu erhalten, in der R'₁, R'₂, R'₃ und R'₄ die oben angegebenen Bedeutungen besitzen, das man gegebenenfalls einer Reaktion zur Substitution des Sauerstoffatoms durch ein Schwefelatom unterzieht, um das Produkt der Formel (L) 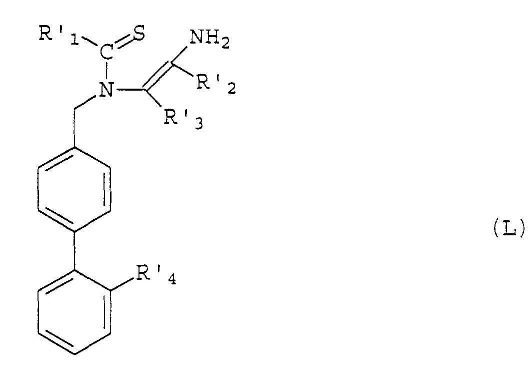 zu erhalten, in der R'₁, R'₂, R'₃ und R'₄ die oben angegebenen Bedeutungen besitzen, und man dann das Produkt der Formel (E) oder das Produkt der Formel (L) einer Reaktion zur Cyclisierung unterzieht, entweder realisiert durch eine saure Katalyse unter Verwendung von beispielsweise Amberlyst H⁺, p-Toluolsulfonsäure (acide tosylique) oder Schwefelsäure in einem Lösungsmittel wie beispielsweise Toluol, Ethylacetat, Dichlormethan, Dichlorethan, oder mit Pentachlorid in Pyridin oder Dimethylaminopyridin, oder auch in Dimethylsulfon, um ein Produkt der Formel (I') 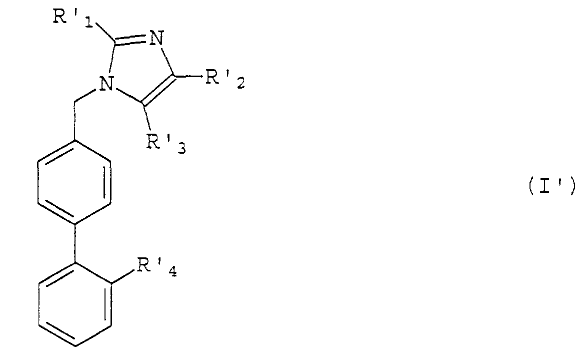 zu erhalten, in der R'₁, R'₂, R'₃ und R'₄ die oben angegebenen Bedeutungen besitzen, mit der Maßgabe, daß die Produkte der Formeln (D), (E) und (L) im Verlaufe des obigen Verfahrens und die Produkte der Formel (I'), wenn erwünscht und wenn erforderlich, einer oder mehreren der nachfolgenden Reaktionen in irgendeiner Reihenfolge unterzogen werden können:
a) einer Reaktion zur Veresterung der Säurefunktion,
b) einer Reaktion zur Verseifung der Esterfunktion,
c) einer Reaktion zur Umwandlung der Cyanofunktion in die Säurefunktion,
d) einer Reaktion zur Reduktion der Carboxyfunktion in die Alkoholfunktion,
e) einer Reaktion zur Umwandlung der Alkoxyfunktion in die Hydroxylfunktion,
f) einer Reaktion zur Oxidation der Gruppe, die ein Schwefelatom umfaßt, in das entsprechende Sulfoxid oder Sulfon,
g) einer Reaktion zur Umwandlung der Alkoholfunktion oder der Sulfonfunktion in die entsprechende Aldehydfunktion oder Säurefunktion,
h) einer Reaktion zur Umwandlung des Restes Nitril in Tetrazol,
i) einer Reaktion zur Umwandlung des Restes Formyl in den Rest Carbamoyl,
j) einer Reaktion zur Umwandlung des Restes Carbamoyl in den Rest Nitril,
k) einer Reaktion zur Umwandlung des Restes 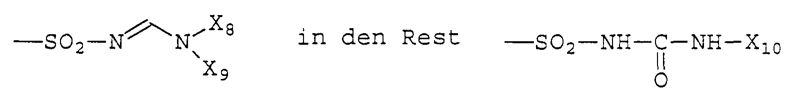 worin X₈, X₉ und X₁₀, gleich oder verschieden, ein Wasserstoffatom oder einen Rest Alkyl oder Alkenyl mit höchstens 4 Kohlenstoffatomen, gegebenenfalls substituiert, darstellen,
wobei die Produkte der Formel (I') Produkte der Formel (I) darstellen können, oder man unterzieht sie, wenn erwünscht und wenn notwendig, zum Erhalten der Produkte der Formel (I) einer oder mehreren der nachfolgenden Reaktionen in irgendeiner Reihenfolge:
a) einer Reaktion zur Entfernung der Schutzgruppen, die von den geschützten reaktiven Funktionen getragen werden,
b) einer Reaktion zur Salzbildung durch eine Mineralsäure oder organische Säure oder durch eine Base, um das entsprechende Salz zu erhalten,
c) einer Reaktion zur Aufspaltung der racemischen Formen in die Spaltprodukte,
wobei die auf diese Weise erhaltenen Produkte der Formel (I) in allen möglichen isomeren racemischen, enantiomeren und diastereoisomeren Formen vorliegen können.

2. Verfahren nach Anspruch 1 zur Herstellung der Produkte der Formel (I), die der Formel (I_{b}) entsprechen: 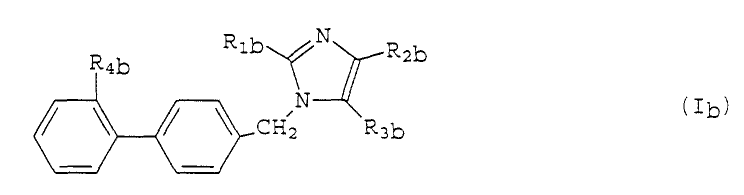 in der
R_{1b} einen Rest Alkyl mit höchstens 4 Kohlenstoffatomen darstellt, R_{3b} ein Wasserstoffatom, einen Rest Formyl, Acyloxy, Alkyl oder Alkoxy, gegebenenfalls substituiert, oder einen Rest Carboxy, frei, in Salz überführt oder verestert durch einen Rest Alkyl darstellt,
R_{2b} einen Rest Phenylthio, Phenylsulfonyl, Phenylsulfinyl, Alkylthio, Alkylsulfonyl oder Alkylsulfinyl, gegebenenfalls substituiert, bedeutet, wie in allen diesen Resten, die R_{2b} und R_{3b} darstellen können, die Reste Alkyl, Alkoxy mit höchstens 6 Kohlenstoffatomen und die Reste Phenyl sind gegebenenfalls substituiert durch einen oder mehrere Reste, ausgewählt unter den Halogenatomen und den Resten Hydroxyl, Trifluormethyl, Acyloxy, Carboxy, frei, in Salz überführt oder verestert, Phenyl, Pyridyl, Tetrazolyl, Alkyl und Alkoxy mit höchstens 4 Kohlenstoffatomen und gegebenenfalls selbst substituiert durch einen Rest Alkoxy mit höchstens 4 Kohlenstoffatomen,
R_{4b} darstellt: den Rest Cyano, Carboxy, frei, in Salz überführt oder verestert, den Rest -SO₂-X_{b}-R_{10b}, worin X_{b} die Reste-NH-, -NH-CO-, -NH-CO-O-, -N=CH-N-R_{13b}, -NH-CO-NH- oder eine einfache Bindung bedeutet und R_{10b} und R_{13b}, gleich oder verschieden, ein Wasserstoffatom, einen Rest Methyl, Ethyl, Propyl, Vinyl, Allyl, Pyridyl, Phenyl, Benzyl, Nitropyridyl, Pyrimidyl, Tetrazolyl, Diazolyl, Piperidinyl, Alkylpiperidinyl, Thiazolyl, Alkylthiazolyl, Tetrahydrofuranyl, Methyltetrahydrofuranyl bedeuten,
wobei die genannten Produkte der Formel (I_{b}) in allen möglichen isomeren racemischen, enantiomeren und diastereoisomeren Formen vorliegen können, sowie die Additionssalze der genannten Produkte der Formel (I_{b}) mit Mineralsäuren und organischen Säuren oder mit Mineralbasen und organischen Basen, dadurch gekennzeichnet, daß man für ihre wie in Anspruch 1 definierte Herstellung die Produkte der Formeln (A), (II), (III) und einen Reaktanden verwendet, der geeignet ist, den wie in Anspruch 1 definierten Rest R'₂ einzubringen, wobei in den obigen Formeln R'₁, R'₂, R'₃ und R'₄ die oben jeweils für R_{1b}, R_{2b}, R_{3b} und R_{4b} angegebenen Werte besitzen und in denen die reaktiven Funktionen gegebenenfalls geschützt sind.

3. Verfahren nach Anspruch 1 zur Herstellung der Produkte der Formel (I) , die der Formel (I_{d}) entsprechen: 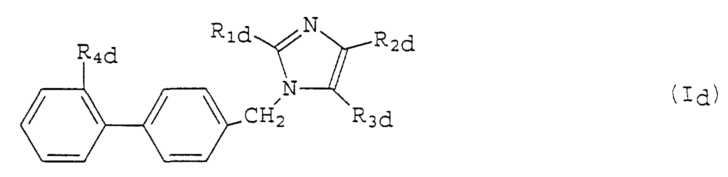 in der
R_{1d} einen Rest Alkyl mit höchstens 4 Kohlenstoffatomen darstellt, R_{3d} einen Rest Carboxy, frei, in Salz überführt oder verestert durch einen geraden oder verzweigten Rest Alkyl mit höchstens 4 Kohlenstoffatomen, Formyl, Acyloxy oder Alkyl mit höchstens 4 Kohlenstoffatomen, gegebenenfalls substituiert durch einen Rest Hydroxyl, darstellt,
R_{2d} einen Rest Phenylthio, Phenylsulfonyl, Phenylsulfinyl, Alkylthio, Alkylsulfonyl oder Alkylsulfinyl bedeutet, in denen der Rest Alkyl höchstens 4 Kohlenstoffatome umfaßt, und
R_{4d} darstellt: den Rest -SO₂-NH₂, -SO₂-NH-CO-O-R_{10d}, -SO₂-N=CH-NR_{13d} oder -SO₂-NH-CO-NH-R_{10d}, worin R_{10d} und R_{13d}, gleich oder verschieden, ausgewählt werden unter dem Wasserstoffatom, dem Rest Methyl, Ethyl, n-Propyl und Propenyl,
wobei die genannten Produkte der Formel (I_{d}) in allen möglichen isomeren racemischen, enantiomeren und diastereoisomeren Formen vorliegen können, sowie die Additionssalze der genannten Produkte der Formel (I_{d}) mit Mineralsäuren und organischen Säuren oder mit Mineralbasen und organischen Basen, dadurch gekennzeichnet, daß man für ihre wie in Anspruch 1 definierte Herstellung die Produkte der Formeln (A), (II), (III) und einen Reaktanden verwendet, der geeignet ist, den wie in Anspruch 1 definierten Rest R'₂ einzubringen, wobei in den obigen Formeln R'₁, R'₂, R'₃ und R'₄ die oben jeweils für R_{1d}, R_{2d}, R_{3d} und R_{4d} angegebenen Werte besitzen und in denen die reaktiven Funktionen gegebenenfalls geschützt sind.

4. Verfahren nach Anspruch 1, dadurch gekennzeichnet, daß man ein Produkt der Formel (A), in der R'₄ den Rest -SO₂-NH₂, -SO₂-NH-CO-O-R_{10d}, -SO₂-N=CH-NR_{13d} oder -SO₂-NH-CO-NH-R_{10d} darstellt, worin R_{10d} und R_{13d}, gleich oder verschieden, ausgewählt werden unter dem Wasserstoffatom, dem Rest Methyl, Ethyl, n-Propyl und Propenyl, worin die reaktiven Funktionen gegebenenfalls geschützt sind, ein Produkt der Formel (II), in der R'₃ den Rest Carboxy, frei, in Salz überführt oder verestert durch einen geraden oder verzweigten Rest Alkyl mit höchstens 4 Kohlenstoffatomen, Formyl, Acyloxy oder Alkyl mit höchstens 4 Kohlenstoffatomen, gegebenenfalls substituiert durch einen Rest Hydroxyl, darstellt, ein Produkt der Formel (III), in der R'₁ einen Rest Alkyl mit höchstens 4 Kohlenstoffatomen darstellt, und einen Reaktanden verwendet, der geeignet ist, den wie in Anspruch 1 definierten Rest R'₂ einzubringen, worin R'₂ einen Rest Phenylthio, Phenylsulfonyl, Phenylsulfinyl, Alkylthio, Alkylsulfonyl oder Alkylsulfinyl bedeutet, in denen der Rest höchstens 4 Kohlenstoffatome umfaßt, und worin die reaktiven Funktionen gegebenenfalls geschützt sind.

5. Verfahren nach Anspruch 1, dadurch gekennzeichnet, daß man ein Produkt der Formel (A), worin R'₄ den Rest 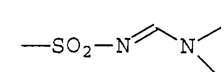 darstellt, ein Produkt der Formel (II), in der R'₃ ein Rest Alkoxy oder Carboxy, frei, in Salz überführt oder verestert ist, ein Produkt der Formel (III), in der R'₁ einen Rest Alkyl mit höchstens 4 Kohlenstoffatomen bedeutet, und einen Reaktanden verwendet, der geeignet ist, den wie in Anspruch 1 definierten Rest R'₂ einzubringen, worin R'₂ einen Rest Alkylthio oder Phenylthio, gegebenenfalls oxidiert in Form von Sulfoxid oder Sulfon, darstellt, wobei diese Reste Alkoxy, Alkylthio und Phenylthio gegebenenfalls substituiert sind durch einen oder mehrere Reste, ausgewählt unter den Halogenatomen, den Resten Alkyl oder Alkoxy mit höchstens 4 Kohlenstoffatomen, Trifluormethyl, Amino, Mono- oder Dialkylamino, Cyano, Phenyl, Hydroxyl, Carboxy, frei, in Salz überführt oder verestert, Acyl und Acyloxy.

6. Verfahren nach Anspruch 1, dadurch gekennzeichnet, daß man eine Verbindung der Formel (A₁) 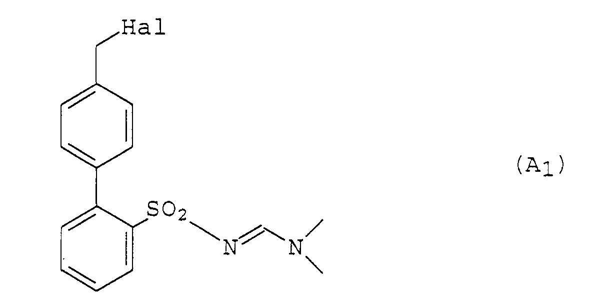 in der Hal ein Halogenatom darstellt, mit einem Oxidationsmittel zur Reaktion bringt, um die Verbindung der Formel (B₁) 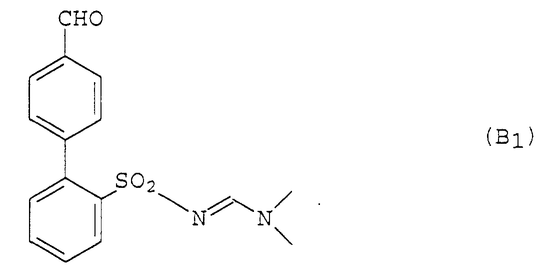 zu erhalten, die man dann mit einer Verbindung der Formel (II) 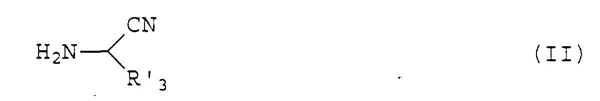 in der R'₃ die oben angegebene Bedeutung besitzt, zur Reaktion bringt, um ein Produkt der Formel (C₁) 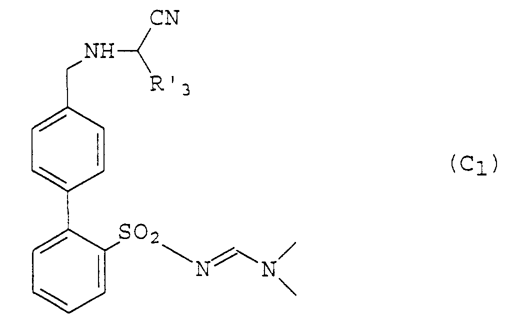 zu erhalten, in der R'₃ die oben angegebene Bedeutung besitzt, das man anschließend mit einer Verbindung der Formel (III)
R'₁-CO-Hal (III)
in der R'₁ und Hal die oben angegebenen Bedeutungen besitzen, zur Reaktion bringt, um ein Produkt der Formel (D₁) 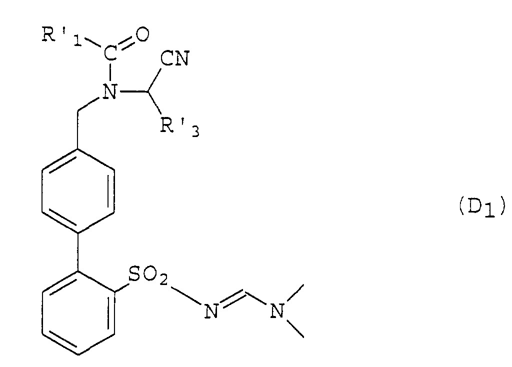 zu erhalten, in der R'₁ und R'₃ die oben angegebenen Bedeutungen besitzen, das man dann
entweder einer Reaktion zur Addition des Restes CN mit Hilfe eines wie in Anspruch 1 definierten Reaktanden, der geeignet ist, den wie oben definierten Substituenten R'₂ einzubringen, unterzieht, um das Produkt der Formel (E₁) 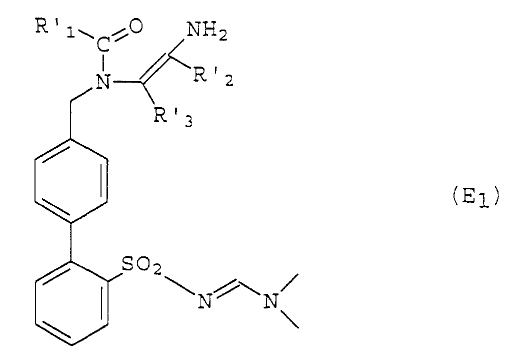 zu erhalten, in der R'₁, R'₂ und R'₃ die oben angegebenen Bedeutungen besitzen, das man danach gegebenenfalls einer Reaktion zur Substitution des Sauerstoffatoms durch ein Schwefelatom unterzieht, um das Produkt der Formel (L₁) 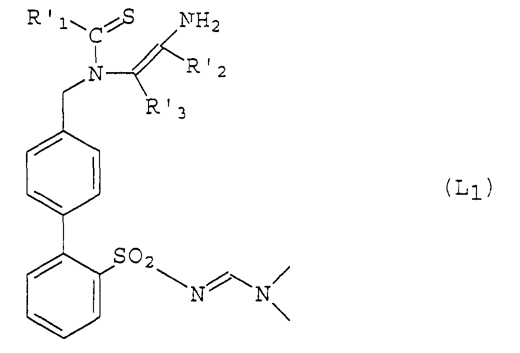 zu erhalten, in der R'₁, R'₂ und R'₃ die oben angegebenen Bedeutungen besitzen, das man, wenn erforderlich oder wenn gewünscht, in ein Produkt der Formel (L₂) 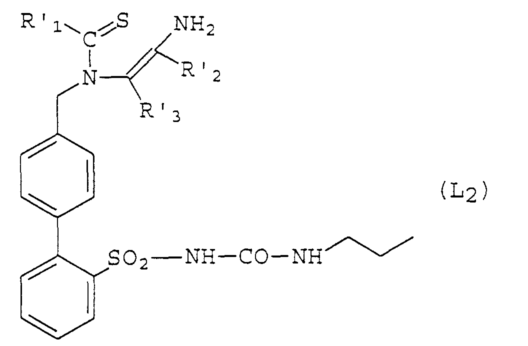 umwandelt, in der R'₁, R'₂ und R'₃ die oben angegebenen Bedeutungen besitzen,
wobei man dann die Produkte der Formeln (E₁), (L₁) oder (L₂) einer Reaktion zur Cyclisierung unterzieht, um ein Produkt der Formel (I'₁) oder (I'₂) 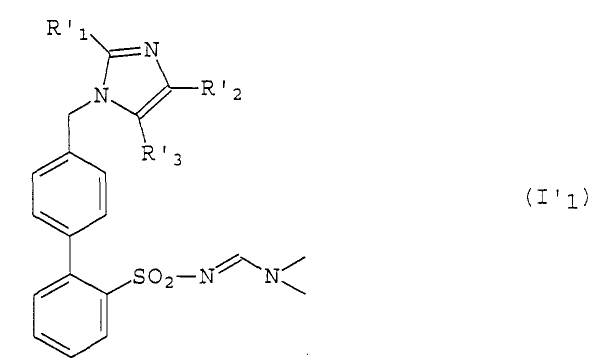 oder 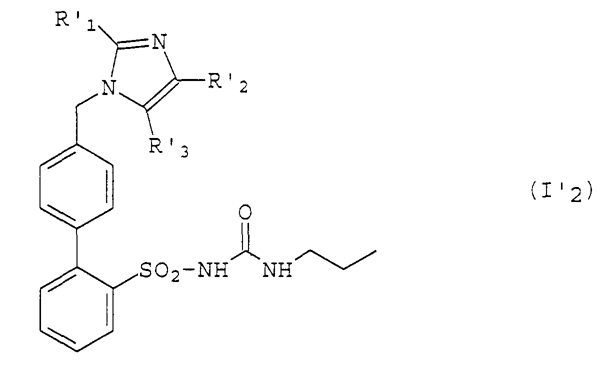 zu erhalten, in der R'₁, R'₂ und R'₃ die oben angegebenen Bedeutungen besitzen, und man gegebenenfalls das Produkt der Formel (I'₁) in das wie oben definierte Produkt der Formel (I'₂) umwandelt, oder das Produkt der Formel (D₁) einer Reaktion zur Addition des Restes 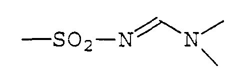 unterzieht, um das Produkt der Formel (D₂) 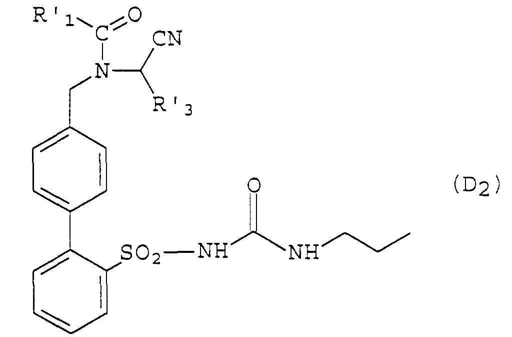 zu erhalten, in der R'₁ und R'₃ die oben angegebenen Bedeutungen besitzen, das man dann einer Reaktion zur Addition des Restes CN wie oben angegeben unterzieht, um ein Produkt der Formel (E₂) 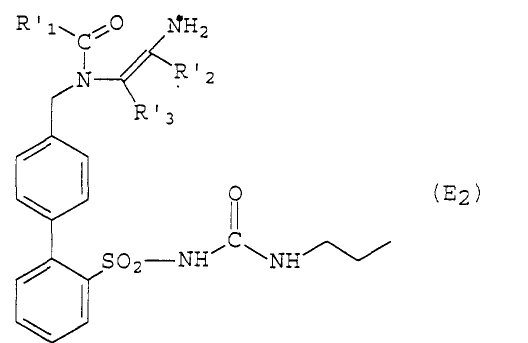 zu erhalten, in der R'₁, R'₂ und R'₃ die oben angegebenen Bedeutungen besitzen, und man dieses schließlich einer Reaktion zur Cyclisierung unterzieht, um ein wie oben definiertes Produkt der Formel (I'₂) zu erhalten.

7. Verfahren wie in Anspruch 1 definiert zur Herstellung von Produkten der Formel (I), die den folgenden Formeln entsprechen:
- 2-Butyl-1-{[2'-carboxy-(1,1'-biphenyl)-4-yl]-methyl}-4-(phenylthio)-1H-imidazol-5-carbonsäure,
- 2-Butyl-1-{[2'-carboxy-(1,1'-biphenyl)-4-yl]-methyl}-4-(methylthio)-1H-imidazol-5-carbonsäure,
- 4'-{[2-Butyl-4-(ethylthio)-5-(hydroxymethyl)-1H-imidazol-1-yl]methyl}-(1,1'-biphenyl)-2-carbonsäure,
- 2-Butyl-1-([2'-carboxy-(1,1'-biphenyl)-4-yl]-methyl}-4-(ethylsulfonyl)-1H-imidazol-5-carbonsäure,
- 2-Butyl-1-{[2'-carboxy-(1,1'-biphenyl)-4-yl]-methyl}-4-(ethylsulfinyl)-1H-imidazol-5-carbonsäure,
- 2-Butyl-1-{[2'-carboxy-(1,1'-biphenyl)-4-yl]-methyl}-4-(ethylthio) -lH-imidazol-5-carbonsäure,
- 2-Butyl-1-{[2'-carboxy-(1,1'-biphenyl)-4-yl]-methyl}-4-(phenylsulfonyl)-1H-imidazol-5-carbonsäure,
- 2-Butyl-1-{[2'-carboxy-(1,1'-biphenyl)-4-yl]-methyl}-4-(phenylsulfinyl)-1H-imidazol-5-carbonsäure,
- 2-Butyl-1-{[2'-tetrazolyl-(1,1'-biphenyl)-4-yl]-methyl}-4-(methylthio)-1H-imidazol-5-carbonsäure.

8. Verfahren wie in Anspruch 1 definiert zur Herstellung von Produkten der Formel (I), die den folgenden Formeln entsprechen:
- 2-Butyl-4-(methylthio)-1-{[2'-((((propylamino)-carbonyl)amino)-sulfonyl)-(1,1'-biphenyl)-4-yl]-methyl}-1H-imidazol-5-carbonsäure-ethylester,
- 2-Butyl-4-(methylthio)-1-{[2'-((((propylamino)-carbonyl)amino)-sulfonyl)-(1,1'-biphenyl)-4-yl]-methyl)-1H-imidazol-5-carbonsäure,
- 2-Butyl-4-(methylthio)-1-{[2'-((((propylamino)-carbonyl)amino)-sulfonyl)-(1,1'-biphenyl)-4-yl]-methyl}-1H-imidazol-5-carbonsäure-Dikaliumsalz.

9. Die Verbindungen der Formel (C) 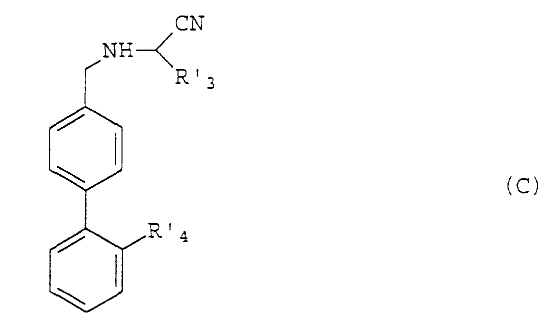 in der R'₃ und R'₄ die in Anspruch 1 angegebenen Bedeutungen besitzen.

10. Die Verbindungen der Formeln (D), (D₁) und (D₂) wie nachstehend definiert: 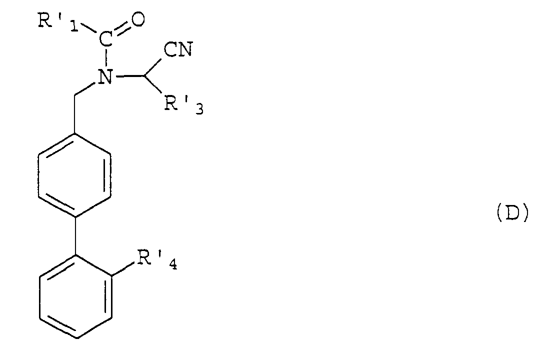 in der R'₁, R'₃ und R'₄ die in Anspruch 1 angegebenen Bedeuungen besitzen, 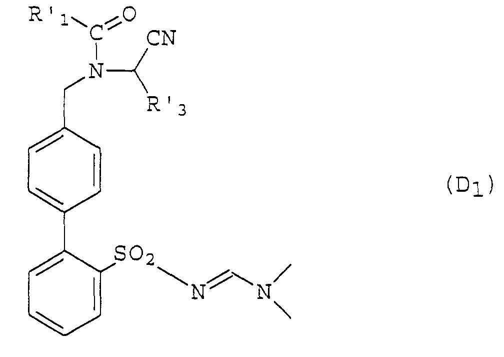 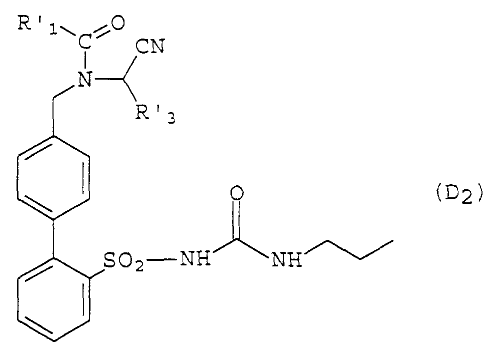 worin R'₁ und R'₃ die in Anspruch 1 angegebenen Bedeutungen besitzen.

11. Die Verbindungen der Formeln (E), (E₁) und (E₂) wie nachstehend definiert: 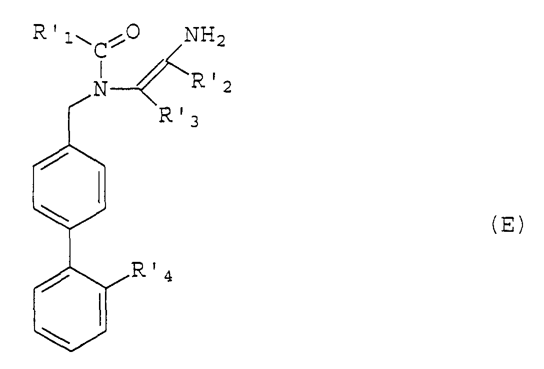 in der R'₁, R'₂, R'₃ und R'₄ die in Anspruch 1 angegebenen Bedeutungen besitzen, 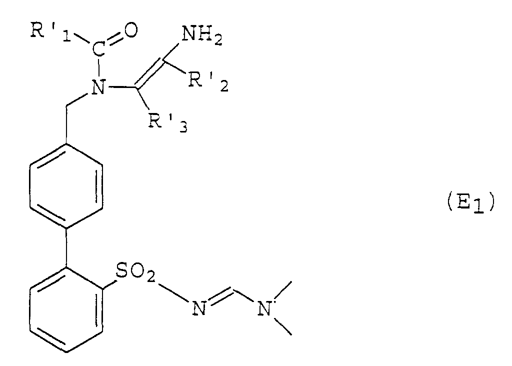 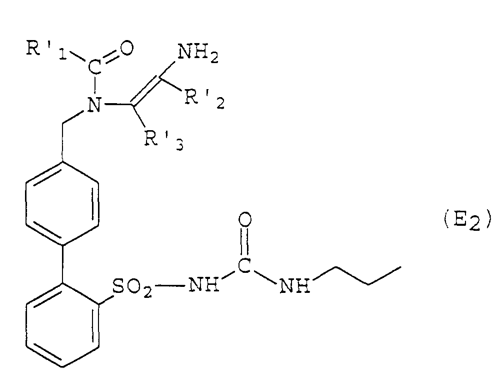 worin R'₁, R'₂, und R'₃ die in Anspruch 1 angegebenen Bedeutungen besitzen.

12. Die Verbindungen der Formeln (L), (L₁) und (L₂) wie nachstehend definiert: 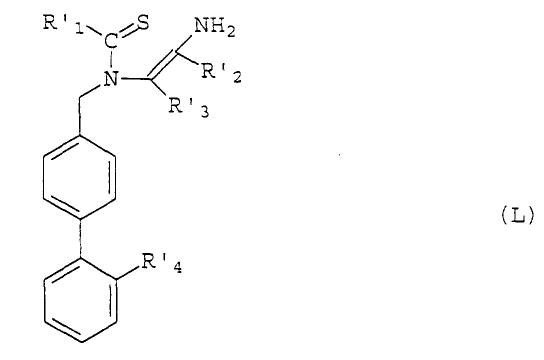 in der R'₁, R'₂, R'₃ und R'₄ die in Anspruch 1 angegebenen Bedeutungen besitzen, 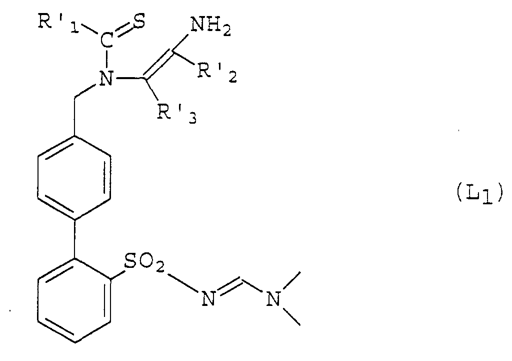 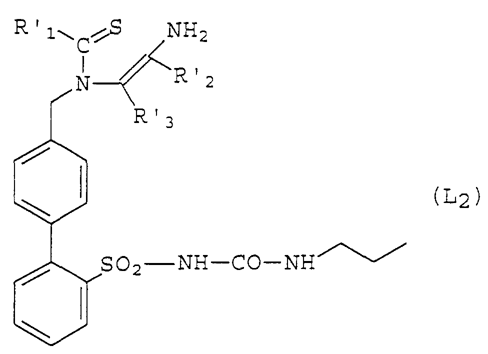 worin R'₁, R'₂, und R'₃ die in Anspruch 1 angegebenen Bedeutungen besitzen.
